# EUROPEAN PATENT APPLICATION

(11) **EP 3 431 469 A1**
(43) Date of publication of application: **23.01.2019**
(21) Application number: 18191137.1
(22) Date of filing: 27.02.2015
(51) Int. Cl.: C07D 233/64, A61K 31/4172, C07K 5/037, A61P 31/12, A61P 31/14, A61P 31/20, A61P 31/04, A61P 27/02, A61P 11/00, A61P 11/06, A61P 1/04, A61P 1/16

(54) **AMIDE COMPOUND AND USE THEREOF AS AGENT FOR THE TREATMENT AND PREVENTION OF DISEASES CAUSED BY RNA- AND/OR DNA-CONTAINING VIRUSES, AND CONCOMITANT DISEASES**

(30) Priority: 12.03.2014 RU 2014109441
(62) Divisional of application: 15761359.7
(71) Applicant: Obschestvo S Ogranichennoi Otvetstvennostiyu "Pharmenterprises", Moscow 117571 (RU)
(72) Inventor: Nebolsin, Vladimir Evgenievich, 143581 Moskovskaya obl. (RU); Kromova, Tatyana Alexandrovna, 248010 Kaluga (RU)
(74) Representative: Hoffmann Eitle

(57) **Abstract**

The present invention relates to medicine and is concerned with a compound of formula (a) below or a pharmaceutically acceptable salt thereof; pharmaceutical compositions comprising an effective amount of the compound of formula (a) or a pharmaceutically acceptable salt thereof; and kits comprising the pharmaceutical composition comprising an effective amount of the compound of formula (a) or a pharmaceutically acceptable salt thereof, pharmaceutically acceptable carriers and excipients, and instructions for use thereof; for use in preventing or treating diseases caused by RNA- and DNA-containing viruses, and concomitant diseases.

The invention addresses the object of providing a compound for use in the prevention or treatment of diseases or complications of infectious diseases, whereby the diseases or infectious diseases are caused by an RNA-containing virus belonging to the Enterovirus, Metapneumovirus, Pneumovirus, Respirovirus, or Alfa-coronavirus genus, and/or by a DNA-containing virus belonging to the Adenoviridae and/or Herpesviridae family; for use in the prevention or treatment of asthma exacerbation, chronic obstructive pulmonary disease, mucoviscidosis, conjunctivitis, gastroenteritis, hepatitis, myocarditis; and for use in the prevention or treatment of rhinorrhea, acute and infectious rhinitis, pharyngitis, nasopharyngitis, tonsillitis, laryngitis, laryngotracheitis, laryngotracheobronchitis, bronchitis, bronchiolitis, pneumonia, or airway obstructive syndrome.

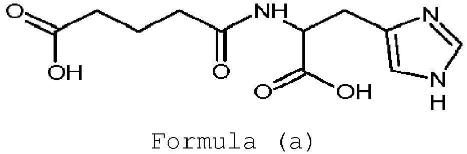

## Description

### FIELD OF THE INVENTION

The present invention relates to medicine, in particular, to the use of a compound of formula (a) or pharmaceutically acceptable salts thereof for the prevention and/or treatment of diseases caused by RNA- and/or DNA-containing viruses, and concomitant diseases.

### BACKGROUND

Viral infections are a serious health problem. Antiviral drugs against most hazardous and dangerous viral infections have not been developed, and the existing medicaments are often toxic to humans or insufficiently effective. Most existing or under-development drugs act through a specific interaction with certain viral proteins. Such drugs have a limited spectrum of activity and promote a rapid emergence of resistant virus variants. According to the Baltimore virus classification system, class I includes viruses whose genome is composed of double-stranded DNA, and classes IV and V include viruses containing single-stranded (+) or (-) RNA. One of the families belonging to class I is the Adenoviridae family that comprises the Mastadenovirus genus that is known to comprise 7 groups of A to G. Human adenoviruses cause a variety of diseases including conjunctivitis, gastroenteritis, hepatitis, myocarditis, and pneumonia. Children under the age of 5 years are most susceptible to adenovirus infection. From 5 to 7% of all respiratory infections in children in the world are caused by adenoviruses. Some serotypes (for example, 14) cause severe, potentially lethal pneumonias. Subgroup A viruses cause gastrointestinal tract diseases, while viruses of B and C subgroups are associated with respiratory tract infections. Viruses of B (type 3), D and E subgroups cause conjunctivitis. Subgroup E viruses are also associated with respiratory tract infections. Viruses of F and G subgroups cause gastroenteritis.

Another family of I class is the Herpesviridae family comprising the Simplex Virus Genus that includes herpes simplex virus types 1 and 2 (HSV-1) and (HSV-2). After primary infection, these viruses cause latent infection that persists throughout life with periodic activation. In a child, infection can be both asymptomatic and severe, involving the central nervous system. HSV infection in babies before or during labor, which can cause a disease of the eye, skin, central nervous system or even lead to a disseminated infection, is especially dangerous. Infection of the central nervous system in children under the age of 3 months leads to herpes encephalitis that, in most cases, is caused by HSV-1. HSV-2 causes genital infection that in general is sexually transmitted. In 2012, 417 million people in the world aged 15 to 49 years have been calculated to be infected with the HSV-2 virus, which is 11.3%; 267 millions of them are women. In addition, 19.2 millions of the total number of infected people were infected in 2012, which is 0.5%. HSV-2 infection is characterized by periodical symptomatic or asymptomatic viral shedding and by the appearance of painful genital ulcers. In addition, HSV-2 has been shown to increase three times the chance of infection with human immunodeficiency virus and accelerates the disease progression.

Class IV includes representatives of the Enterovirus genus of the Picornaviridae family and the Coronaviridae family, and class V includes respiratory syncytial virus (RSV) and metapneumovirus of the Paramyxoviridae family.

The recited groups of viruses have developed an effective strategy of inhibiting the cellular antiviral program. Such an aggressive strategy of inhibiting the cellular antiviral defense system leads to high contagiousness and pathogenicity of these virus groups.

Infection caused by human coronavirus (CoV) (Coronaviridae family) traditionally has a low yearly percentage of lower and upper respiratory tract infections. More severe course of disease is observed in elderly people with weakened immunity, and in children. HCoV-OC43 (OC43) and HCoV-229E (229E) viruses are the first recorded human coronaviruses. In recent years, another two viruses, HCoV-NL63 (NL63) and HCoV-HKU1 (HKU1), have been registered. These four viruses usually cause acute upper respiratory tract infections and are rarely associated with an upper respiratory tract disorder. Severe diseases are rare and, as a rule, are associated with concomitant diseases and/or immunosuppressive conditions.

Today, among viruses of the Enterovirus genus, human rhinoviruses are the biggest problem. Rhinoviruses, which are replicated in the nasopharyngeal mucosal cells, cause in humans upper respiratory tract diseases. Rhinoviruses are causative agents of at least 80% of cold-related diseases. Apart from the enormous economic damage (20 million humans/hour annually in the U.S.), rhinovirus infections cause a large number of complications such as sinusitis and otitis media and are frequently detected in virologic examination of children with pneumonia. In asthmatic children, rhinovirus infection is also a cause of acerbations in 80% cases. In adults, rhinovirus may cause acerbations of asthma and chronic obstructive pulmonary disease, chronic bronchitis, and mucoviscidosis. Rhinoviruses were isolated from pneumonia patients with immunodeficiency conditions.

Since there are over 100 antigenic types of rhinovirus, it is impossible to develop an effective vaccine (Palmenberg, A. C; Spiro, D; Kuzmickas, R; Wang, S; Djikeng, A; Rathe, JA; Fraser-Liggett, CM; Liggett, SB (2009). "Sequencing and Analyses of All Known Human rhinovirus Genomes Reveals Structure and Evolution". Science 324 (5923): 55-9. doi:10.1126/science.1165557.PMID 19213880). In addition, there is no effective chemotherapeutic agent for the treatment of rhinovirus infection.

Enterovirus type 71 (EV71) was isolated for first time from patients with aseptic meningitis and a patient with encephalitis in California in 1970-1972 years. It should be noted that in severe cases the virus causes the development of neurological disorders such as meningitis, paralysis and encephalitis. The virus is spread under unsanitary conditions. After infection with virus EV71, the temperature increases, skin rash appears on hands and feet, on the palms and soles, the extremities become swollen, and ulcers appear in the mouth. In its severe form, Enterovirus can be fatal. Enterovirus 71 is reported to be the most "severe" virus among all human enteroviruses. This virus can cause large outbreaks with fatal outcomes. There are no vaccines against Enterovirus 71, and non-specific therapy has not yet been developed.

Coxsackie virus infection (HCXV) is a large group of diseases characterized by pronounced clinical polymorphism. Coxsackie virus infection can manifest in meningitis, paralysis, acute respiratory disorders, pneumonia, hemorrhagic conjunctivitis, myocarditis, hepatitis, diabetes and other syndromes. According to the modern classification of viruses, human enteroviruses belonging to the Enterovirus genus are divided into 5 species (14): 1) poliovirus; 2) human enterovirus A; 3) human enterovirus B; 4) human enterovirus C; and 5) human enterovirus D. Various serotypes of Coxsackie virus belong to the following enterovirus species: human enterovirus A (Coxsackie viruses A2-8, 10, 12, 14, and 16); human enterovirus B (Coxsackie viruses A9, B1-6); human enterovirus C (Coxsackie viruses A1, 11, 13, 15, 17-22, and 24) .

Coxsackie viruses, like other human enteroviruses, are ubiquitous in the world. In the temperate countries, their maximum circulation is in the summer-autumn season. The viruses are characterized by high invasiveness, which causes their rapid spread in the human population. Coxsackie viruses are often a cause of "sudden" outbreaks in organized children's groups and hospitals; intrafamilial spread of infection is registered as well. A high variability of the viral genome plays an important role in the epidemiology of Coxsackie virus and other enterovirus infections. The consequence of this is the ability of various serotypes to provoke different pathologies in certain circumstances. On the other hand, the same clinical syndrome may be caused by different serotypes and different enterovirus species. Genetic variability, selection and rapid spread of modified viruses result in major disease outbreaks, in the etiology of which these viruses have not previously been involved, or their circulation has not been seen for a long time.

The primary replication of Coxsackie virus occurs in the nasopharyngeal and gut-associated lymphoid tissue. The virus causes local lesions expressed in the symptoms of ARD, herpangina, pharyngitis, etc. In the throat the virus is detected until the seventh day, and it is excreted in the faeces for 3-4 weeks (in immunodeficiency for several years). Viremia, in which the virus penetrates into the target organs, follows its primary replication. For Coxsackie viruses, such target organs may be brain and spinal cord, meninges, upper respiratory tract, lungs, heart, liver, skin, etc. Coxsackie B virus can cause severe generalized pathological processes in newborns, resulting in necrosis in heart, brain and spinal cord, liver, and kidneys. The viruses cause the following clinic syndromes: aseptic meningitis (Coxsackie viruses A2, 3, 4, 6, 7, 9, 10, and B1-6); acute systemic disease in children with myocarditis and meningoencephalitis (Coxsackie viruses D1-5); paralysis (Coxsackie viruses A1, 2, 5, 7, 8, 9, 21, and B2-5); herpangina (Coxsackie viruses A2, 3, 4, 5, 6, 8, and 10); acute pharyngitis (Coxsackie viruses A10, 21); contagious rhinitis (Coxsackie viruses A21, 24); damage of the upper respiratory tract (Coxsackie virusesA9, 16, and B2-5) (16); pericarditis, myocarditis (Coxsackie viruses B1-5); hepatitis (Coxsackie viruses A4, 9, 20, and B5); diarrhea of newborns and infants (Coxsackie viruses A18, 20, 21, 24); acute hemorrhagic conjunctivitis (Coxsackie viruses A24); foot-and-mouth-like disease (Coxsackie viruses A5, 10, 16); exanthemata (Coxsackie viruses A4, 5, 6, 9, 16); pleurodynia (Coxsackie viruses B3, 5); rash (Coxsackie viruses B5); and fever (Coxsackie viruses B1-5). There are absent chemotherapeutic agents for the treatment of Coxsackie virus infections. Pathogenetic or symptomatic therapy is used, depending on the clinical form of the disease.

The Picornaviridae family includes the representatives of the Respirovirus genus (human parainfluenza virus types 1, 2, 3, 4, and 5), the Pneumovirus genus (respiratory-syncytial virus), and the Metapneumovirus genus (human metapneumovirus).

Paramyxoviruses are an important class of viruses that are associated with respiratory diseases. Respiratory-syncytial virus (RSV) is known to be a dominant pathogen of the lower respiratory tract throughout the world.

RSV is an important pathogen in newborns and infants and is a causative agent of at least 70% of severe viral bronchitis and/or pneumonias, the majority part of which is characterized by wheezing and dyspnea. This bronchiolitis is the most common cause of hospitalization in the winter season during the first year of child's life. RSV also causes bronchiolitis, pneumonia and chronic obstructive respiratory disease in humans of all-ages and makes a significant contribution to an excess mortality in the winter season.

RSV takes a leading position in the number of fatal cases among viral infections. Only the U.S. spends $2.4 billion on the treatment of viral diseases of the lower respiratory tract in children. 50-65% of children under the age of one year old are infected with this virus, and almost 100% of two-year-old children are infected. In addition to premature newborns and older persons, a high-risk group includes persons with diseases of the cardiovascular, respiratory and immune systems. Based on published and non-published data, RSV has been calculated to cause in the world 33.8 millions of cases of episodic acute infections of the lower respiratory tract (LRTI), wherein 3.4 millions of severe LRTI cases require hospitalization, and 66,000-99,000 of fatal cases among children under the age of 5 (Nair H, Nokes DJ, Gessner BD, Dherani M, Madhi SA, Singleton RJ, O'Brien KL, Roca A, Wright PF, Bruce N, Chandran A, Theodoratou E, Sutanto A, Sedyaningsih ER, Ngama M, Munywoki PK, Kartasasmita C, Simoes EA, Rudan I, Weber MW, Campbell H. Global burden of acute lower respiratory infections due to respiratory syncytial virus in young children: a systematic review and meta-analysis. Lancet; 375: 1545-55). Every year only in the U.S., 90,000 premature newborns, 125,000 hospitalized newborns, more than 3.5 million children under the age of 2, and 175,000 hospitalized adults need treatment (Storey S. Respiratory syncytial virus market. Nat Rev Drug Discov 2010; 9: 15-6). About a third of children hospitalized with acute bronchiolitis, in the first year of their life, have an episodic dyspnea and an increased sensitivity to common allergens (Schauer U, Hoffjan S, Bittscheidt J, Kochling A, Hemmis S, Bongartz S, Stephan V. RSV bronchiolitis and risk of wheeze and allergic sensitisation in the first year of life. Eur Respir J 2002; 20: 1277-83). These symptoms may return in following years (Sigurs N, Gustafsson PM, Bjarnason R, Lundberg F, Schmidt S, Sigurbergsson F, Kjellman B. Severe respiratory syncytial virus bronchiolitis in infancy and asthma and allergy at age 13. Am J Respir Crit Care Med 2005; 171: 137-41). Bronchiolitis may be caused by renovirus, coronovirus, enfluenza and parainfluenza viruses, and adenovirus. However, among the all recited viruses, RSV is the most frequent cause of hospitalization due to bronchiolitis. An adaptive immunity formed as a result of a past RSV infection both in children (with an immature immune system) and in adults are short-term and does not provide a complete antiviral protection. This fact leads to reinfections recorded throughout life. In first months of life, the blood of newborns comprises maternal anti-RSV antibodies.

Human metapneumovirus (HMPV) is the closest to respiratory-syncytial virus. For the first time, this virus was isolated in 2001 from children with bronchiolitis in Netherlands. HMPV also comprises genomic (-)ssRNA and belongs to the Pneumovirus genus. HMPV circulates throughout the body and causes almost universal infection in children. Like influenza and respiratory-syncytial virus, the activity of HMPV is highest in the winter period in moderate climate. Most of the available data on the clinical manifestations of HMPV infection suggests that the virus causes upper respiratory infections, bronchiolitis and pneumonia. Reinfection with HMPV occurs throughout adulthood. The disease is mild and, in adults, is often asymptomatic. A high-risk group includes elderly people, adults with lung diseases and with a defective immune system. HMPV outbreaks were registered in hospitals, and among frail elderly people, the mortality rate was 50%. In addition, exacerbations registered for chronic obstructive pulmonary disease is 6 to 12%. In recipients of hematopoietic stem cell transplants, HMPV was associated with severe idiopathic pneumonia.

Among the total number of acute infections of the respiratory tract, parainfluenza viruses are about 20% in adults and 30-40% in young children, second in frequency only to respiratory syncytial virus. Today there are 4 known types of parainfluenza viruses (1, 2, 3, 4a, and 4b) isolated from a human being. They are not characterized by variability of antigenic structure, which is inherent in influenza viruses. In most patients, parainfluenza proceeds as a short-term (no more than 3-6 days) disease without pronounced general intoxication. However, hypoxia, infection of the lower respiratory tract, and neurological manifestations are frequent in children and require hospitalization. In addition, the disease may take the form of croup, bronchiolitis, and pneumonia. Parainfluenza viruses of types 1 and 2 most often are associated with croup, while parainfluenza viruses of types 3 and 4 are considered to be most pathogenic, they cause bronchitis, bronchiolitis, and pneumonia more often than others. (Frost HM, Robinson CC, Dominguez SR Epidemiology and clinical presentation of parainfluenza type 4 in children: a 3-year comparative study to parainfluenza types 1-3. J Infect Dis. 2014 Mar 1; 209(5):695-702. doi: 10.1093/infdis/jit552. Epub 2013 Oct 16). Children under one-year old are especially sensitive. In this connection, it should be noted that parainfluenza infection is responsible for significant mortality in young children and immunosuppressed adults since, being complicated with bacterial infection, parainfluenza is a cause of mortality from the lower respiratory tract infections in 25-30% of these groups. Reinfection with parainfluenza is possible throughout life.

The most common cause of catarrhal inflammation of the upper respiratory tract is bacterial or viral infection (for example, nasopharyngitis, pharyngitis, laryngitis, rhinitis); thus, the inflammation of the nasopharyngeal mucous membrane is most often caused by an infection. This disease also includes acute and infectious rhinitis and rhinorrhea (acute rhinitis).

Nasopharyngitis is the most common manifestation of an acute respiratory infection associated with limitation of activity and needs medical advice. 82% of all acute nasopharyngitis are caused by rhinoviruses.

Over the past decade, viruses determining the severity of acute respiratory diseases with airway obstruction have been identified, especially in children in the early years. Special attention is paid to the role of respiratory syncytial virus, metapneumovirus, coronavirus, bocavirus, rhinovirus, and parainfluenza virus in the development of obstructive airway syndrome. Their role in the development of acute obstructive syndrome of respiratory tract in children is undeniable; at the same time, there is evidence of their role in the development of asthma in genetically predisposed individuals.

Respiratory syncytial virus, metapneumovirus, rhinovirus, parainfluenza, coronavirus, adenovirus, and herpes virus can cause primary pneumonia, bronchitis, and bronchiolitis. Viral respiratory tract diseases are often accompanied by bacterial infection. Respiratory bacterial pathogens are frequently present in the nasopharynx in healthy people. Airway damage resulting from viral infection may lead to an increased bacterial adhesion to the infected respiratory tract, and to secondary bacterial pneumonia, bronchitis, bronchiolitis, or tonsillitis, which are severe complications.

In most cases, laryngotracheitis is of infectious nature - it is caused by viruses (adenovirus, influenza viruses, parainfluenza viruses) or bacteria (staphylococcus, streptococcus, pneumococcus, Mycoplasma, etc.). Laryngotracheitis may occur as an independent disease or as a complication of an inflammatory process in the other parts of the respiratory tract (rhinitis, tonsillitis, sinusitis, etc.).

Infectious factors are important in the disease development. When viruses affect immature tissue structures, chronic inflammation in bronchi is possible already in early childhood. Acute respiratory virus infections facilitate secondary bacterial inflammation. Microbial reproduction leads to the progression of inflammation as a result of self-destruction of the bronchial structure and activation of enzymes of inflammatory cells. The consequence of these processes is impaired mucociliary clearance that leads to panbronchitis and peribronchitis, mediates the formation of bronchitis deformans.

It should be noted that the only chemotherapeutic agent exerting some beneficial effects in infections caused by (+) and (-) RNA viruses is ribavirin. However, ribavirin is relatively toxic drug, often causing anemia. Its main feature is a long-term deposition in erythrocytes. As a result, traces of ribavirin are detected even 6 months after the end of therapy. Reference is also made to the teratogenicity of ribavirin. There are absent effective drugs for the treatment of adenovirus infections. HSV is treated with acyclovir (licensed drug) and other derivatives of nucleoside analogues, but there is an urgent need for novel, more effective antiviral agents.

Frequently, respiratory infections are caused by mixed infections, i.e., by infectious processes that develop in the body under simultaneous combined effect of two or more causative agents, such as virus associations, which suggests the need to develop drugs being effective simultaneously against all these infections.

Etiologic agents of mixed infections can be microorganisms of the same family or larger taxons and kingdoms in such combinations as virus - virus, virus - bacteria, etc.

In these days, mixed respiratory viral infections caused, inter alia, by rhinoviruses, Coxsackie virus, respiratory syncytial virus, human metapneumovirus, parainfluenza virus, coronavirus, human adenovirus, herpes simplex virus 1 or type 2, become frequent.

### SUMMARY OF INVENTION

The present invention relates to a compound of formula (a) or a pharmaceutically acceptable salt thereof for use in the prevention and treatment of diseases caused by RNA- and/or DNA-containing viruses, wherein the formula (a) is:

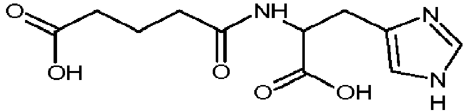

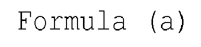

In particular, the inventors have surprisingly found that the compound of formula (a) can be used as non-toxic antiviral agent against infections caused by viruses belonging to the Enterovirus genus, the Metapneumovirus genus, the Pneumovirus genus, or to the Coronaviridae family (not limited to the recited ones). Furthermore, the following compound is mentioned in the present disclosure:

| Number in the application | Formula |
|---|---|
| 275 | 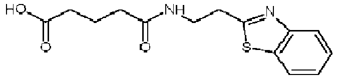 |

In view of the foregoing, the present invention relates to an agent for use in the treatment and/or prevention of a disease caused by an RNA-containing virus belonging to the Enterovirus, Metapneumovirus, Pneumovirus, Respirovirus, or Alfa-coronavirus genus, and/or by a DNA-containing virus belonging to the Adenoviridae and/or Herpesviridae family, wherein the agent is the compound of formula (a) or a pharmaceutically acceptable salt thereof.

The present disclosure further describes methods for preparing the compound of formula (a) or a pharmaceutically acceptable salt thereof.

The invention relates to the compound of formula (a) or a pharmaceutically acceptable salt thereof for use in preventing and treating a disease caused by an RNA-containing virus belonging to the Enterovirus, Metapneumovirus, Pneumovirus, Respirovirus, or Alfa-coronavirus genus, and/or by a DNA-containing virus belonging to the Adenoviridae and/or Herpesviridae family; to the compound of formula (a) or a pharmaceutically acceptable salt thereof for use in preventing or treating asthma exacerbation, chronic obstructive pulmonary disease, mucoviscidosis, conjunctivitis, gastroenteritis, hepatitis, myocarditis; to the compound of formula (a) or a pharmaceutically acceptable salt thereof for use in preventing or treating complications of an infectious disease caused by an RNA-containing virus belonging to the Enterovirus, Metapneumovirus, Pneumovirus, Respirovirus, or Alfa-coronavirus genus, and/or by a DNA-containing virus belonging to the Adenoviridae and/or Herpesviridae family; to the compound of formula (a) or a pharmaceutically acceptable salt thereof for use in preventing and treating rhinorrhea, acute and infectious rhinitis, pharyngitis, nasopharyngitis, tonsillitis, laryngitis, laryngotracheitis, laryngotracheobronchitis, bronchitis, bronchiolitis, pneumonia, or airway obstructive syndrome, wherein said uses comprise administering to a patient an effective amount of the compound of general formula (a) or a pharmaceutically acceptable salt thereof.

Further, the invention relates to a pharmaceutical composition for use in the treatment of a disease caused by an RNA-containing virus belonging to the Enterovirus, Metapneumovirus, Pneumovirus, Respirovirus, or Alfa-coronavirus genus, and/or by a DNA-containing virus belonging to the Adenoviridae and/or Herpesviridae family; to a pharmaceutical composition for use in the prevention or treatment of asthma exacerbation, chronic obstructive pulmonary disease, mucoviscidosis, conjunctivitis, gastroenteritis, hepatitis, myocarditis; to a pharmaceutical composition for use in the prevention or treatment of complications of an infectious disease caused by an RNA-containing virus belonging to the Enterovirus, Metapneumovirus, Pneumovirus, Respirovirus, or Alfa-coronavirus genus, and/or by a DNA-containing virus belonging to the Adenoviridae and/or Herpesviridae family; to a pharmaceutical composition for use in the prevention or treatment of rhinorrhea, acute and infectious rhinitis, pharyngitis, nasopharyngitis, tonsillitis, laryngitis, laryngotracheitis, laryngotracheobronchitis, bronchitis, bronchiolitis, pneumonia, or airway obstructive syndrome wherein said pharmaceutical compositions comprise an effective amount of the compound of formula (a) or a pharmaceutically acceptable salt thereof.

The invention also relates to a kit for use in the treatment of diseases caused by an RNA-containing virus belonging to the Enterovirus, Metapneumovirus, Pneumovirus, Respirovirus, or Alfa-coronavirus genus, and/or by a DNA-containing virus belonging to the Adenoviridae and/or Herpesviridae family; to a kit for use in the prevention or treatment of asthma exacerbation, chronic obstructive pulmonary disease, mucoviscidosis, conjunctivitis, gastroenteritis, hepatitis, or myocarditis; to a kit for use in the prevention or treatment of complications of an infectious disease caused by an RNA-containing virus belonging to the Enterovirus, Metapneumovirus, Pneumovirus, Respirovirus, or Alfa-coronavirus genus, and/or by a DNA-containing virus belonging to the Adenoviridae and/or Herpesviridae family; to a kit for use in the prevention and treatment of rhinorrhea, acute and infectious rhinitis, pharyngitis, nasopharyngitis, tonsillitis, laryngitis, laryngotracheitis, laryngotracheobronchitis, bronchitis, bronchiolitis, pneumonia, or airway obstructive syndrome, wherein said kits comprise the composition according to the invention and instructions for use thereof.

### EMBODIMENTS OF THE INVENTION

The present invention relates to a compound of formula (a) as shown below: 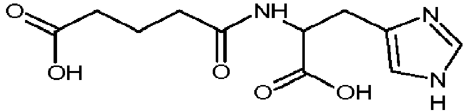 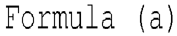 or a pharmaceutically acceptable salt thereof, for use in the prevention or treatment of diseases or complications of an infectious disease as recited in claims 1 to 9.

Formula (a) and a pharmaceutically acceptable salt thereof fall within the scope of the following general formula I: 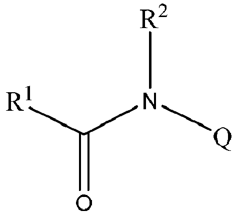 or a pharmaceutically acceptable salt thereof, wherein
R¹ is 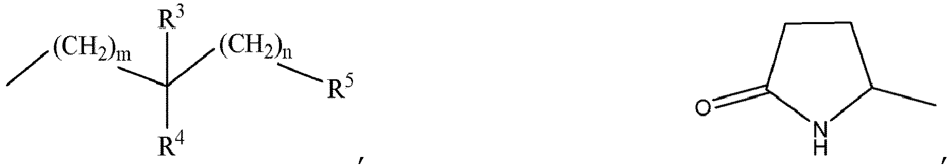 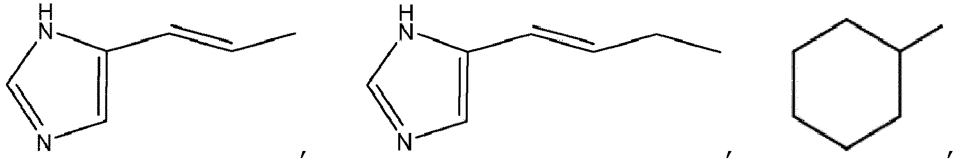 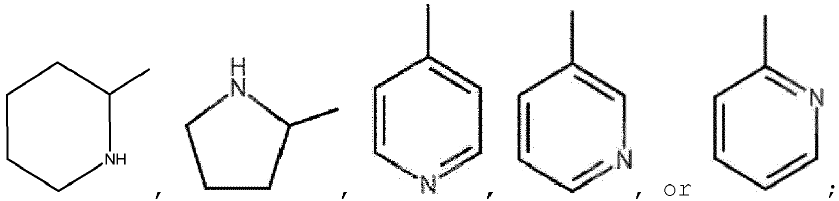
m is an integer of 0, 1, or 2;
n is an integer of 0, 1, or 2;
R² is H or C₁-C₆alkyl;
each R³ and R⁴ independently is H, O, C₁-C₆alkyl, -NH₂,-NHC(=O)CH₃, OH, and -NHC(O)CH₂COOH;
R⁵ is -COOH, -C(O)NH₂, 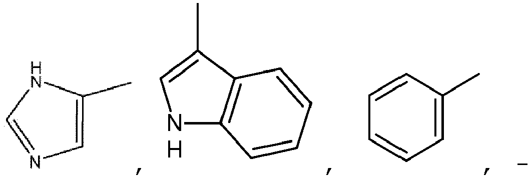 NH₂, HN=C(NH₂)NH-, NH₂S(O)₂-, (NH₂)₂CHNH-, or CH₃C(O)NH-;
wherein R⁵ can be optionally substituted with a substituent selected from the group consisting of benzyl, benzyl-OC(O)-, C₁-C₆alkyl, OH, and -NH₂;
Q is 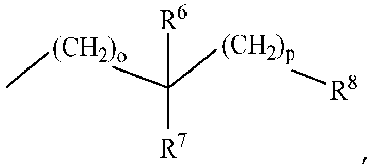
Q and R², together with the nitrogen atom to which they are attached, can form a 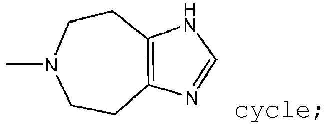
Q and R¹, together with -C(O)N- to which they are attached, can form a 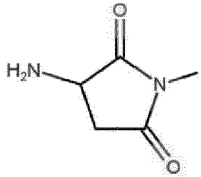 cycle optionally substituted at the amino group with -C(O)CH₃;
o is an integer of 0 or 2;
p is an integer of 0 to 3;
each R⁶ and R⁷ independently is H, C₁-C₆alkyl, -C(O)NH₂,-COOH, -CH₂OH, or C₁-C₆alkyl-NH₂; wherein R⁶ and R⁷ can be optionally substituted with one or two C₁-C₆alkyls, -CH(CH(OH)CH₃)(C(O)OC₂H₅),-CH(CH(OH)CH₃)(COOH), -CH(CH(CH₃)₂)(C(O)OCH₃),-CH(CH(CH₃)₂)(C(O)NH₂), -CH(CH₃)C(O)OCH₃, -CH(CH₃)C(O)NH₂,-CH(CH₂CH(CH₃)₂)(C(O)OCH₃), -CH(CH₂CH(CH₃)₂)(C(O)ONH₂),-CH(CH₂OH)(COOH), -CH(CH(OH)CH₃) (C(O)OCH₃),-CH(CH₂(OH))(C(O)OCH₃), -CH(C(O)NH₂) (CH₂OH), -CH₂CH(OH)CH₃,-(CH₂)₂OH, -(CH₂)₃OH, -CH₂C(O)NH₂, -CH₂C(O)OCH₃, -CH₂COOH,-C(O)OCH₃, or -CH(C(O)NH₂) (CH(OH)CH₃);
R⁸ is H, -COOH, NH₂, 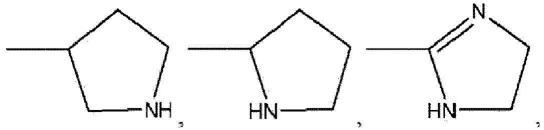 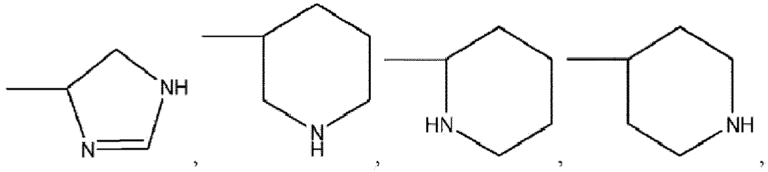 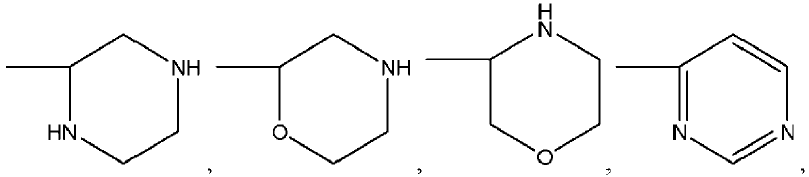 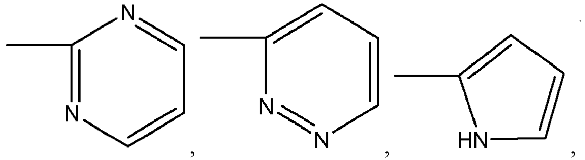 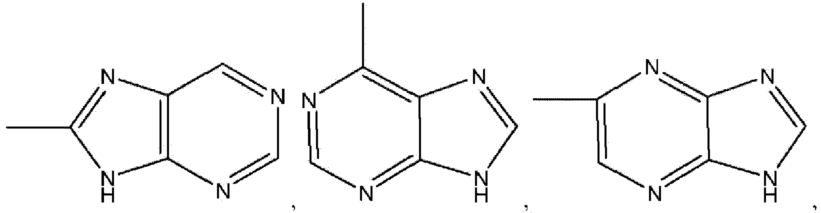 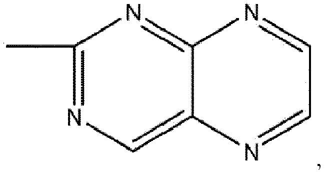 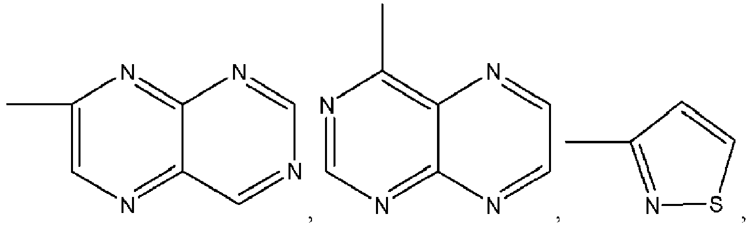 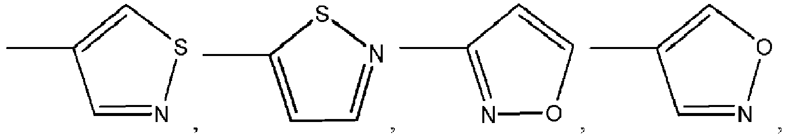 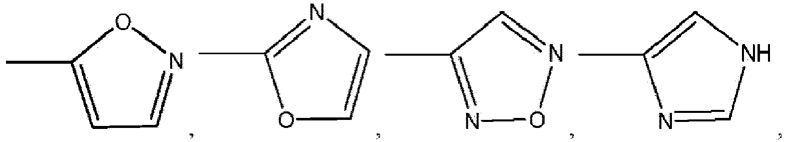 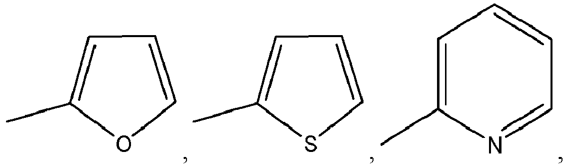 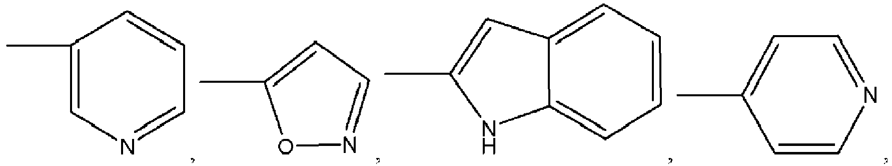 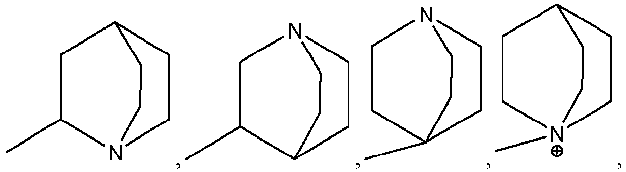 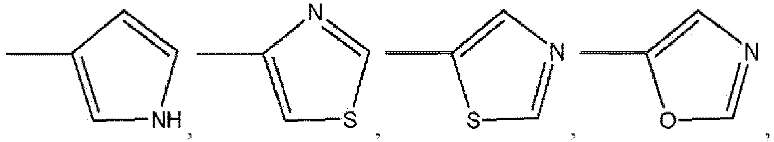 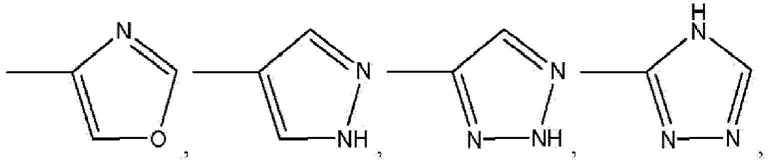 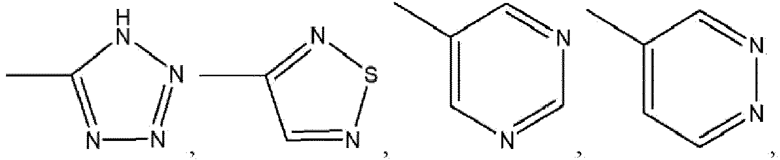 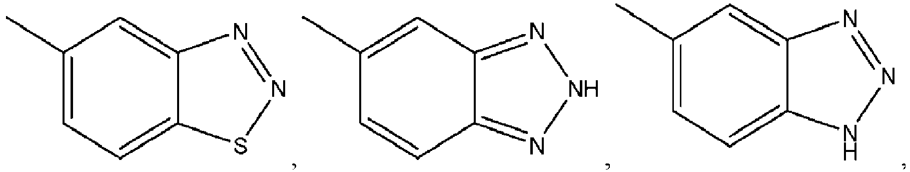 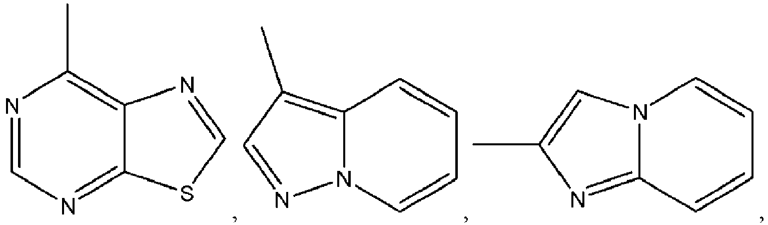 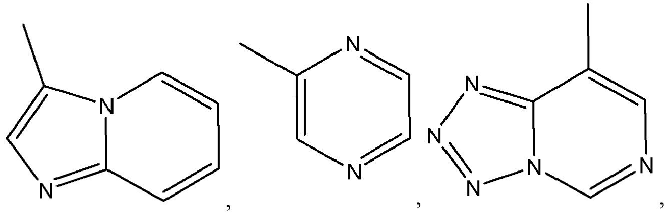 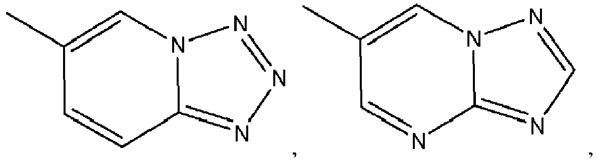 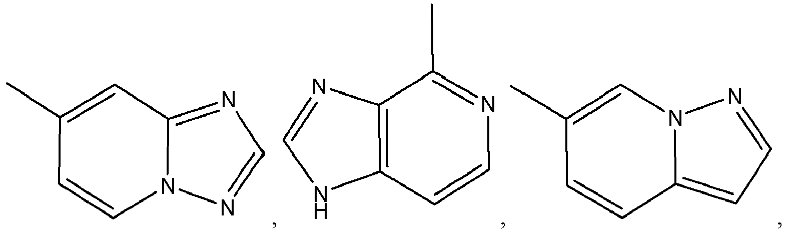 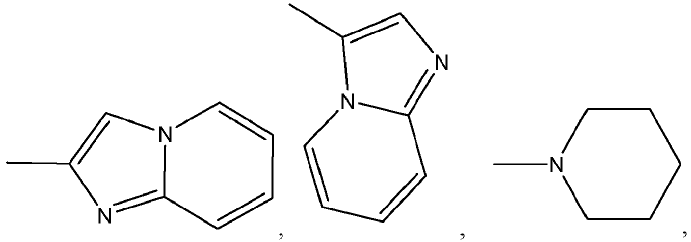 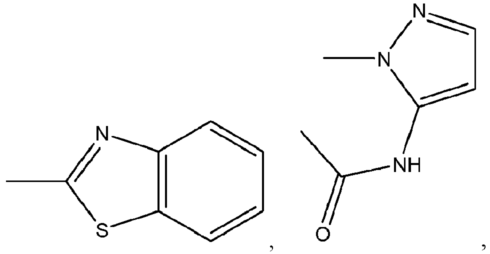 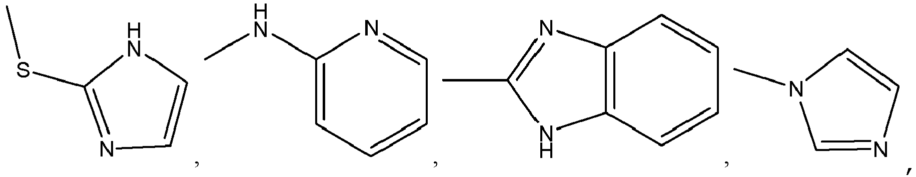 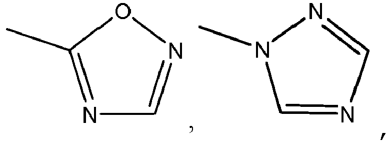 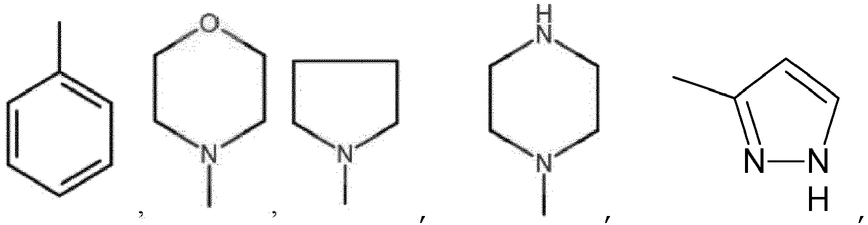 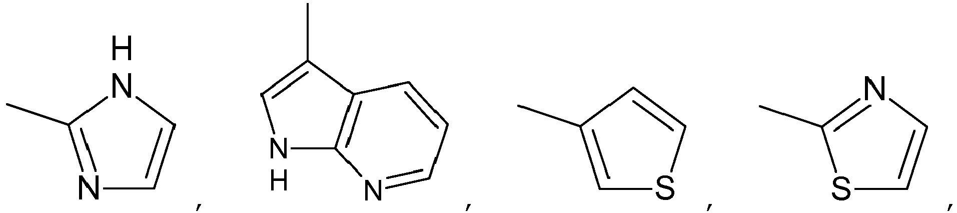 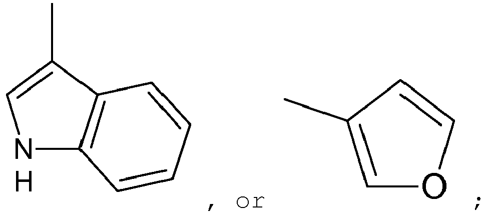
wherein R⁸ can be optionally substituted with one or more substituents selected from C₁-C₆alkyl, C₁-C₆alkoxy, halo, -COOH, -OH, pyridyl, -O-benzyl, and phenyl.

The compound of formula (a) or a pharmaceutically acceptable salt thereof, for use according to the invention is administered in a solid dosage form.

The present disclosure also relates to methods for preparing a compound of general formula I or a pharmaceutically acceptable salt thereof.

In particular, the present disclosure relates to a method for preparing a compound of general formula I, which is a dicarboxylic acid monoamide, or a pharmaceutically acceptable salt thereof, the method comprising reacting an appropriate anhydride with an amine or a peptide in a suitable organic solvent optionally in the presence of an organic base.

The present disclosure relates to a method for preparing a compound of general formula I, which is a C₁-C₆alkylamide, or a pharmaceutically acceptable salt thereof, the method comprising reacting an appropriate amine comprising a C₁-C₆alkyl substituent at the amino group, with glutaric anhydride in an organic solvent.

The present disclosure relates to methods for preparing a compound of general formula I, which is a dicarboxylic acid amide comprising a C₁-C₆alkyl-substituted carboxyl group in a glutaryl moiety, or a pharmaceutically acceptable salt thereof, the methods comprising:
(1)
   (a) reacting an appropriate anhydride with an amine optionally in a suitable organic solvent and under boiling;
   (b) suspending the resulting amide in a C₁-C₆ alcohol, and adding dropwise trimethylchlorosilane at room temperature;
(2)
   (a) synthesizing a glutaric acid mono C₁-C₆ ester from glutaric anhydride and an appropriate C₁-C₆ alcohol by an activated N-oxysuccinimide ester method in an anhydrous organic solvent; and
   (b) reacting the resulting glutaric acid C₁-C₆ ester with an appropriate amine in the presence of a condensing agent, preferably 1,1'-carbonyldiimidazole, in an organic solvent.

The present disclosure relates to a method for preparing a compound of general formula I, which is a dicarboxylic acid amide comprising mono- or dimethyl substituents in a glutaryl moiety, or a pharmaceutically acceptable salt thereof, the method comprising:
(a) obtaining an appropriate glutaric acid mono- or dimethyl substituted monomethyl ester by opening a mono- or dimethyl substituted glutaric anhydride upon stirring thereof in methanol at room temperature for 24 hours;
(b) reacting the glutaric acid mono- or dimethyl substituted monomethyl ester with an appropriate amine in an organic solvent, preferably in N,N-dimethylformamide, in the presence of a condensing agent, preferably 1,1'-carbonyldiimidazole.

The present disclosure relates to a method for preparing a compound of general formula I, which is a dicarboxylic acid amide comprising a hydroxyl group, as a substituent, in the α-position of a glutaryl moiety, or a pharmaceutically acceptable salt thereof, the method comprising:
(a) preparing 5-oxotetrahydrofuran-2-carbonyl chloride from 5-oxotetrahydrofuran-2-carboxylic acid by its reaction with oxalyl chloride in an organic solvent under cooling;
(b) reacting 5-oxotetrahydrofuran-2-carbonyl chloride with an appropriate amine in an organic solvent in the presence of potash, followed by hydrolysis of the lactone in the presence of alkali to obtain a target amide.

The present disclosure relates to a method for preparing a compound of general formula I, which is a glutaryl derivative of a dipeptide, or a pharmaceutically acceptable salt thereof, the method comprising:
(a) synthesizing a dipeptide from (di-Boc)-protected histidine and an appropriate amino acid by an activated p-nitrophenyl ester method in N,N-dimethylformamide;
(b) removing Boc-protection by the treatment of the protected dipeptide with trifluoroacetic acid; and
(c) adding glutaric anhydride to the trifluoroacetic derivative of the dipeptide in N,N-dimethylformamide in the presence of 2 equivalents of N-methylmorpholine.

The present disclosure relates to a method for preparing a compound of general formula I, which is a derivative of γ-aminobutyric acid and an appropriate amine, or a pharmaceutically acceptable salt thereof, the method comprising:
(a) preparing imidazolide of N-Boc-γ-aminobutyric acid by the reaction of N-Boc-γ-aminobutyric acid with 1,1'-carbonyldiimidazole in an anhydrous organic solvent; and
(b) reacting the imidazolide of N-Boc-γ-aminobutyric acid with an appropriate amine under heating in an anhydrous organic solvent.

The present disclosure relates to methods for preparing a compound of general formula I, which is a derivative of pyroglutamic acid, N-acetylglutamic acid at the α-carboxyl group, or glutamic acid at the γ-carboxyl group, 3-aminosulfonylpropionic acid and an appropriate amine, or a pharmaceutically acceptable salt thereof,
(1) by an activated N-oxysuccinimide ester method, comprising reacting N-oxysuccinimide ester of an appropriate acid with an appropriate amine in an anhydrous organic solvent at room temperature;
(2) by a method consisting in the use of an appropriate condensing agent, preferably N,N,N',N'-tetramethyl-O-(benzotriazol-1-yl)uronium tetrafluoroborate in the presence of an organic base in an organic solvent.
(3) by a method consisting in the long-term aging, preferably for a week, of an appropriate amine and pyroglutamic acid in an organic alcohol.

The present disclosure relates to methods for preparing a compound of general formula I, which is an amide formed with 3-(4-imidazolyl)acrylic acid and 3-(4-imidazolyl)propionic acid and an appropriate amino acid: 2-aminopentanoic acid, 4-aminobutyric acid, and 6-aminohexanoic acid, or a pharmaceutically acceptable salt thereof
(1) by the chloroanhydride method, comprising:
   (a) preparing chloroanhydride of an appropriate acid by using preferably thionyl chloride,
   (b) reacting the resulting chloroanhydride without additional purification with an appropriate amino acid in an anhydrous organic solvent at room temperature;
(2) by a method consisting in the use of a condensing agent, preferably 1,1'-carbonyldiimidazole. The reaction is carried out in an organic solvent in the presence of an organic base under heating, preferably, to 80°C.

The present disclosure relates to a method for preparing a compound of general formula I, which is a derivative comprising the -C-O-C(=O)- bond, or a pharmaceutically acceptable salt thereof, the method comprising preparing an appropriate ester by the Mitsunobu reaction from an appropriate alcohol or acid.

The nitrogen atom in a heterocycle is protected, if necessary for the synthesis of the compound according to the present invention, by using, for example, a carbamate-type protecting group, such as tert-butoxycarbonyl (Boc), and a benzoyl protecting group.

The invention also relates to the compound of formula (a) or a pharmaceutically acceptable salt thereof for use in preventing and treating a disease caused by an RNA-containing virus belonging to the Enterovirus, Metapneumovirus, Pneumovirus, Respirovirus, or Alfa-coronavirus genus, and/or by a DNA-containing virus belonging to the Adenoviridae and/or Herpesviridae family, comprising administering to a patient an effective amount of a compound of formula (a) or a pharmaceutically acceptable salt thereof.

The virus belonging to the Enetrovirus genus can be selected from the group consisting of rhinoviruses, Coxsackie viruses, and enterovirus type 71. The virus belonging to the Pneumovirus genus is respiratory syncytial virus, and the virus belonging to the Metapneumovirus genus is human metapneumovirus. The virus belonging to the Respirovirus genus is parainfluenza virus. The virus belonging to the Alfa-coronavirus genus is coronovirus. The Adenoviridae family includes the Mastadenovirus genus that includes human adenovirus. The Herpesviridae family includes the Simplex Virus Genus to which herpes simplex virus types 1 and 2 (HSV-1 and HSV-2) belong.

The invention further relates to the compound of formula (a) or a pharmaceutically acceptable salt thereof for use in preventing and treating a disease caused by an RNA-containing virus belonging to the Enterovirus, Metapneumovirus, Pneumovirus, Respirovirus, or Alfa-coronavirus genus, and/or by a DNA-containing virus belonging to the Adenoviridae and/or Herpesviridae family; to the compound of formula (a) or a pharmaceutically acceptable salt thereof for use in preventing or treating asthma exacerbation, chronic obstructive pulmonary disease, mucoviscidosis, conjunctivitis, gastroenteritis, hepatitis, or myocarditis; to the compound of formula (a) or a pharmaceutically acceptable salt thereof for use in preventing or treating complications of an infectious disease caused by an RNA-containing virus belonging to the genera of Enterovirus, Metapneumovirus, Pneumovirus, Respirovirus, or Alfa-coronavirus genus, and/or by a DNA-containing virus belonging to the Adenoviridae and/or Herpesviridae family; to the compound of formula (a) or a pharmaceutically acceptable salt thereof for use in preventing and treating rhinorrhea, acute and infectious rhinitis, pharyngitis, nasopharyngitis, tonsillitis, laryngitis, laryngotracheitis, laryngotracheobronchitis, bronchitis, bronchiolitis, pneumonia, or airway obstructive syndrome, wherein said uses comprise administering to a patient an effective amount of the compound of formula (a) or a pharmaceutically acceptable salt thereof.

The dose of the compound of formula (a) or a pharmaceutically acceptable salt thereof can be about 0.1 to 30, preferably 0.1 to 10 mg/kg of patient's body weight. A single dose of the compound of formula (a) can be about 2 to 300 mg. The administration of the compound of formula I lasts 3 to 14 days.

In addition, the invention relates to a pharmaceutical composition for use in the treatment of a disease caused by an RNA-containing virus belonging to the Enterovirus, Metapneumovirus, Pneumovirus, Respirovirus, or Alfa-coronavirus genus, and/or by a DNA-containing virus belonging to the Adenoviridae and/or Herpesviridae family, comprising an effective amount of the compound of formula (a) or a pharmaceutically acceptable salt thereof and pharmaceutically acceptable carriers and excipients. The effective amount of the compound of formula (a) or a pharmaceutically acceptable salt thereof is preferably from 0.1 to 30 mg/kg of body weight. A dose of the compound of formula (a) can be 2 to 300 mg in once-daily administration.

Further, the invention relates to a pharmaceutical composition for use in the prevention or treatment of asthma exacerbation, chronic obstructive pulmonary disease, mucoviscidosis, conjunctivitis, gastroenteritis, hepatitis, or myocarditis; to a pharmaceutical composition for use in the prevention or treatment of complications of an infectious disease caused by an RNA-containing virus belonging to the Enterovirus, Metapneumovirus, Pneumovirus, Respirovirus, or Alfa-coronavirus genus, and/or by a DNA-containing virus belonging to the Adenoviridae and/or Herpesviridae family; to a pharmaceutical composition for use in the prevention or treatment of rhinorrhea, acute and infectious rhinitis, pharyngitis, nasopharyngitis, tonsillitis, laryngitis, laryngotracheitis, laryngotracheobronchitis, bronchitis, bronchiolitis, pneumonia, or airway obstructive syndrome, wherein said compositions comprise an effective amount of the compound of formula (a) or a pharmaceutically acceptable salt thereof.

The invention also relates to a kit for use in the treatment of a disease caused by an RNA-containing virus belonging to the Enterovirus, Metapneumovirus, Pneumovirus, Respirovirus, or Alfa-coronavirus genus, and/or by a DNA-containing virus belonging to the Adenoviridae and/or Herpesviridae family, comprising the composition according to the invention and instructions for use thereof.

The invention also relates to a kit for use in the prevention or treatment of asthma exacerbation, chronic obstructive pulmonary disease, mucoviscidosis, conjunctivitis, gastroenteritis, hepatitis, or myocarditis; to a kit for use in the prevention or treatment of complications of an infectious disease caused by an RNA-containing virus belonging to the Enterovirus, Metapneumovirus, Pneumovirus, Respirovirus, or Alfa-coronavirus genus, and/or by a DNA-containing virus belonging to the Adenoviridae and/or Herpesviridae family; to a kit for use in the prevention and treatment of rhinorrhea, acute and infectious rhinitis, pharyngitis, nasopharyngitis, tonsillitis, laryngitis, laryngotracheitis, laryngotracheobronchitis, bronchitis, bronchiolitis, pneumonia, or airway obstructive syndrome, wherein said kits comprise the composition according to the invention and instructions for use thereof.

The pharmaceutically acceptable salt of the compound of formula (a) according to the invention may be a salt thereof with alkali or alkaline earth metals, preferably a sodium, potassium, or lithium salt.

In addition, the pharmaceutically acceptable salt of the compound according to the present invention can be an organic acid addition salt (for example, formiate, acetate, maleate, tartrate, methanesulfonate, benzene sulfonate, toluene sulphonate, etc.), inorganic acid addition salt (for example, hydrochloride, hydrobromide, sulphate, phosphate, etc.), and a salt with an amino acid (for example, an asparaginic acid salt, a glutamic acid salt, etc.), preferably chlorohydrate and acetate.

The compound of formula (a) or a salt thereof is administered in an effective amount to provide a desired therapeutic result.

The compound of formula (a) or a salt thereof may be administered to a patient in a dose of from 0.1 to 30 mg/kg of human body weight, preferably in a dose of from 0.3 to 1.5 mg/kg, one or more times a day.

However, it should be noted that a particular dose for a particular patient depends on many factors, such as patient's age, body weight, gender, general health condition, and diet; the schedule and route of administration of the agent, the rate of excretion thereof from the body; and the severity of a disease in an individual under treatment.

The pharmaceutical compositions according to the invention comprise the compound of formula (a) or a pharmaceutically acceptable salt thereof in an effective amount that provides a desired result, and may be prepared as a unit dosage form (for example, in a solid, semi-solid, or liquid form) that comprises the compound of formula (a) or a salt thereof as an active agent in a mixture with a carrier or an excipient suitable for intramuscular, intravenous, oral, sublingual, inhalation, intranasal, intrarectal, or transdermal administration. The active ingredient may be in a composition with a conventional nontoxic pharmaceutically acceptable carrier suitable for the manufacture of solutions, tablets, pills, capsules, coated pills, suppositories, emulsions, suspensions, ointments, gels, patches, and other dosage forms.

Diverse compounds are suitable as an excipient, for example, such as saccharides, for example, glucose, lactose, of sucrose; mannitol or sorbitol; cellulose derivatives; and/or calcium phosphates, for example, tricalcium phosphate or calcium hydrophosphate. Compounds such as starch paste (for example, corn, wheat, rice, or potato starch), gelatin, tragacanth, methylcellulose, hydroxypropylmethylcellulose, sodium carboxymethylcellulose, and/or polyvinylpyrrolidone are useful as a binder. If necessary, disintegrating agents, such as the aforementioned starches and carboxymethylstarch, crosslinked polyvinylpyrrolidone, agar, or alginic acid or a salt thereof, such as sodium alginate, may be added.

Additives that may be optionally used are flowability control agents and lubricants, such as silicon dioxide, talc, stearic acid and salts thereof, for example, magnesium stearate or calcium stearate, and/or propylene glycol.

Such additives as stabilizers, thickening agents, colorants, and fragrances can be also added.

The used ointment bases include hydrocarbon ointment bases, such as white Vaseline and yellow Vaseline (Vaselinum album and Vaselinum flavum, respectively), Vaseline oil (Oleum Vaselini), and white ointment and liquid ointment (Unguentum album and Unguentum flavum, respectively), wherein solid paraffin or wax can be used as an additive providing a firmer texture; absorptive ointment bases, such as hydrophilic Vaseline (Vaselinum hydrophylicum), lanoline (Lanolinum), and cold cream (Unguentum leniens); water-removable ointment bases, such as hydrophilic ointment (Unguentum hydrophylum); water-soluble ointment bases, such as polyethylene glycol ointment (Unguentum Glycolis Polyaethyleni); bentonite bases; and others.

The base for gels may be selected from methylcellulose, sodium caboxymethylcellulose, oxypropylcellulose, polyethylene glycol or polyethylene oxide, and carbopol.

The base for suppositories may be a water-insoluble base such as cocoa butter; a water-soluble or water-miscible base, such as gelatin-glycerol or polyethylene oxide; or a combined base, such as a soap-glycerol base.

In a unit dosage form, the amount of an active agent, used in combination with a carrier, may vary depending on a recipient under the treatment and on a particular method of administration of the therapeutic agent.

For example, when the compound of formula (a) or a salt thereof is used in the form of a solution for injection, the active agent is in an amount of 0.1 to 5%. A diluent may be selected from a 0.9% sodium chloride solution, distilled water, a Novocain solution for injection, Ringer's solution, and a glucose solution, which can comprise specific solubilizing adjuvants. When the compound of formula (a) or a salt thereof is administered in the form of a tablet or a suppository, its amount is 10 to 300 mg per unit dosage form.

The dosage forms of the present invention are manufactured by traditional methods, such as blending, granulation, forming pills, dissolution, and lyophilization.

### DEFINITIONS

The term "alkyl", as used herein, means a saturated linear or branched hydrocarbon. In some embodiments, the alkyl group comprises 1 to 6 carbon atoms. In other embodiments, the alkyl group comprises 1 to 5 carbon atoms. In yet other embodiments, the alkyl group comprises 1 to 4 carbon atoms, and in yet other embodiments, the alkyl group comprises 1 to 3 carbon atoms.

The term "alkoxy", as used herein, means an alkyl group, as defined above, that is attached to a molecule via an oxygen atom ("alkoxy", for example, -O-alkyl).

### EXPERIMENTAL PART

### Methods of synthesis

Identity of the obtained compounds was confirmed by the thin-layer chromatography (TLC) method on "Kieselgel 60 F254" plates (Merck, Germany). Chromatograms were stained with a chloro-tetramethylbenzene reagent and Pauly's reagent.

A UPLC/MS Shimadzu 2020 LC/MS system of analysis of multicomponent mixtures comprised: a CBM-20A analytical HPLC chromatograph, LC-30AD pumps, a SIL-30AC autosampler, SPD-M20A detectors, ELSD-LTII (evaporative light scattering detector) and an LCMS-20 mass-spectrometer.

A Waters ACQUITY UPLC BEH C18 column, 1.7µm, 2.1x50mm, was used with a solvent system for gradient-elution: solvent A - water with 0.1% HCOOH, solvent B - acetonitrile with 0.1% HCOOH (condition A).

A YMC-UltraHT Hydrosphere C18 column, 2.0µm, 50x2.0mm, was used with a solvent system for gradient-elution: solvent A - water with 0.1% HCOOH, solvent B - acetonitrile with 0.1% HCOOH (condition B).

A Synergi Fusion-RP column, 150x2mm, 4µm, 80Å, was used with a solvent system for gradient-elution: solvent A - water with 0.1% HCOOH, solvent B - acetonitrile with 0.1% HCOOH (condition C).

A Shim-pack XR-ODS II column, 75x3mm, was used with a solvent system for gradient-elution: solvent A - water with 0.1% HCOOH, solvent B - acetonitrile with 0.1% HCOOH (condition D).

A Synergi 2u Hydro-RP Mercury column, 20x2.0mm, was used with a solvent system gradient-elution: solvent A - water with 0.05% TFAA, solvent B - acetonitrile with 0.05% TFAA (condition E).

A UPLC/MS Shimadzu 2020 LC/MS system of analysis of multicomponent mixtures, comprised: a Surveyor MSQ chromatograph (Thermo Fisher Scientific), LC pumps (Thermo Fisher Scientific), a PAL system autosampler (CTC analytics), Surveyor PDA Plus detectors (Thermo Fisher), and a Surveyor MSQ mass-spectrometer (Thermo Fisher Scientific).

A SunFire C18 column, 3.5µm, 2.1x30mm, (Waters) was used with a solvent system for gradient-elution: solvent A - 0.1% aqueous solution of formic acid, solvent B - 95% acetonitrile, 5% water, 0.1% formic acid (condition F).

Analytical reversed-phase HPLC was carried out by using a HPLC Shimadzu system for analysis of organic multicomponent mixtures, comprising an analytical HPLC CBM-20A chromatograph, LC-20AD pumps, a SIL-20A autosampler, and a SPD-20A UV-detector.

A Symmetry C18 column, 150x4.6 mm, 5µm, was used with a gradient elution system: solvent A - an aqueous solution of 0,0025M sodium 1-hexylsulfonate, pH=3; solvent B - acetonitrile (condition 1).

Luna C18 (2) 100 A column, 250×4.6 mm (No. 599779-23), was used with a gradient elution system: phosphate buffer solution (pH 3.0) - methanol (condition 2).

A X-Bridge C 18 column, 150x4.6mm (3.5µm), was used with a gradient elution system: solvent A - an aqueous solution of 0,0025M sodium 1-hexylsulfonate, pH=3; solvent B - acetonitrile (condition 3).

A Symmetry C18 column, 150x4.6 mm, 5µm, was used with a gradient elution system: phosphate buffer solution (pH 3.0) - methanol (condition 4).

A Merk.LiChroCART column, 250×4mm, 5 µm. LiChrospher 100RP-8E 5 µm.C8. Serial number 1.50837.0001, was used with a gradient elution system: ammonium acetate buffer (pH 7.5) - acetonitrile (condition 5).

Analytical reversed-phase HPLC was carried out by using a system for analysis of organic multicomponent mixtures, comprising: a chromatograph (Agilent 1100), pumps (Hewlett-Packard series 1100, Bin Pump G1312A), and a UV-detector (DAD G1315B Agilent 1100).

An ELSICO ReproSil-PurC18-AO column, 5µm, 250x4.6mm, was used with a solvent system for gradient-elution: solvent A - an aqueous ammonium phosphate buffer, pH=8.6; solvent B - acetonitrile (condition 6).

¹H NMR spectra were registered in a Bruker DPX-400 spectrometer (German).

Dicarboxylic acid monoamides were prepared by the reaction of an appropriate anhydride with an amine or a dipeptide in an organic solvent at different temperature modes in the presence or without an organic base. In some cases, the NH group of a heterocycle in used amine derivatives was protected. Boc-protection was preferred. Preferred organic solvents for the condensation reaction include tetrahydrofuran, chloroform, methylene chloride, N,N-dimethylformamide, dichloromethane, acetonitrile, and a mixture of dioxane with N,N-dimethylformamide in a ratio of 3:1. The reaction was preferably carried out under cooling to 0°C or to 3-5°C, at room temperature, or under heating to 45°C or to 60°C, as well as at the boiling point of a solvent.

Dicarboxylic acid C₁-C₆alkylamides were prepared by the reaction of an appropriate amine comprising a C₁-C₆alkyl substituent at the amine group with glutaric anhydride in an organic solvent, preferably in isopropanol, under cooling.

Dicarboxylic acid amides comprising in a glutaryl moiety a C₁-C₆alkyl-substituted carboxyl group were prepared by the reaction of an appropriate anhydride with an amine optionally in an appropriate solvent under boiling. Then, the resulting amide was suspended in a C₁-C₆ alcohol, and trimethylchlorosilane was added dropwise thereto at room temperature.

The synthesis of a glutaric acid mono C₁-C₆ ester was carried out by using glutaric anhydride and an appropriate C₁-C₆ alcohol by an activated N-oxysuccinimide ester method in an anhydrous organic solvent. After that, the resulting glutaric acid C₁-C₆ ester was entered into reaction with an appropriate amine in the presence of a condensing agent, preferably 1,1'-carbonyldiimidazole, in an organic solvent.

Dicarboxylic acid amides comprising in a glutaryl moiety mono- or dimethyl substituents were prepared by opening a mono- or dimethyl substituted glutaric anhydride by stirring thereof in methanol at room temperature for 24 hours. Then the glutaric acid mono- or dimethylsubstituted monomethyl ester was entered into reaction with an appropriate amine in an organic solvent, preferably in N,N-dimethylformamide, in the presence of a condensing agent, preferably 1,1'-carbonyldiimidazole.

Dicarboxylic acid amides comprising a hydroxy group, as a substituent, in a glutaryl moiety in α-position were prepared by using 5-oxotetrahydrofuran-2-carbonyl chloride prepared from 5-oxotetrahydrofuran-2-carboxylic acid by the reaction with oxalyl chloride in an organic solvent under cooling, and then by the reaction of 5-oxotetrahydrofuran-2-carbonyl chloride with an appropriate amine in an organic solvent in the presence of potash, followed by hydrolysis of the lactone in the presence of alkali to obtain a target amide.

In the process of preparing glutaryl derivatives of dipeptides, a dipeptide was synthesized from di-Boc-protected histidine and an appropriate amino acid by an activated p-nitrophenyl ester method in N,N-dimethylformamide. Boc-protection was removed by treating the protected dipeptide with trifluoroacetic acid. A dipeptide glutaryl derivative was obtained by adding glutaric anhydride to trifluoroacetic derivative of the dipeptide in N,N-dimethylformamide in the presence of 2 equivalents of N-methylmorpholine.

Derivatives of γ-aminobutyric acid and an appropriate amine were synthesized using a condensing agent, preferably 1,1'-carbonyldiimidazole. The initial compound was a protected derivative of γ-aminobutyric acid, preferably N-Boc-γ-aminobutyric acid. The reaction of N-Boc-γ-aminobutyric acid with 1,1'-carbonyldiimidazole gave an activated derivative, imidazolide of N-Boc-γ-aminobutyric acid, which was entered into reaction with an appropriate amine. Both reactions ran in organic solvents, preferably anhydrous acetonitrile. The condensation reaction was carried out under heating, preferably at 45°C.

Derivatives of pyroglutamic acid, N-acetyl-glutamic acid at the α-carboxyl group, glutamic acid at the γ-carboxyl group, 3-aminosulgonylpropionic acid and an appropriate amine were prepared:
by an activated N-oxysuccinimide ester method, comprising the reaction of an N-oxysuccinimide ester of an appropriate acid with an appropriate amine in an anhydrous organic solvent at room temperature;
by a method consisting in the use of a condensing agent, preferably N,N,N',N'-tetramethyl-O-(benzotriazol-1-yl)uronium tetrafluoroborate, in the presence of an organic base in an organic solvent.
by a method consisting in the long-term aging, preferably for a week, of an appropriate amine and pyroglutamic acid in an organic alcohol, preferably in methanol or isopropanol.

The synthesis of amides of 3-(4-imidazolyl)acrilic acid and 3-(4-imidazolyl)propionic acid or a pharmaceutically acceptable salt thereof, preferably, with 2-aminopentanoic acid, 4-aminobutyric acid, and 6-aminohexanoic acid was carried out:
(1) by the chloranhydride method. Chloranhydride of an appropriate acid was prepared by using preferably thionyl chloride, the resulting chloranhydride, without further purification, was entered into reaction with an appropriate amine acid in an anhydrous organic solvent at room temperature;
(2) by a method consisting in the use of a condensing agent, preferably 1,1'-carbonyldiimidazole. The reaction was carried out in an organic solvent in the presence of an organic base under heating, preferably, to 80°C.

Derivatives comprising the -C-O-C(=O)- bond were prepared from an appropriate ester prepared by the Mitsunobu reaction from an appropriate alcohol or acid.

The rest of the compounds were synthesized by standard methods of Organic Chemistry.

### 1. Synthesis of dicarboxylic acid monoamide derivatives as illustrated by the synthesis of compound 196

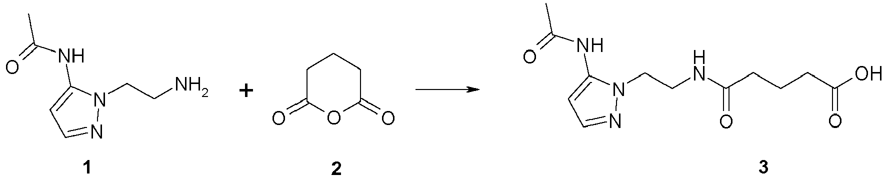

A solution of glutaric anhydride (2) (2.850 g, 25 mmol) in dichloromethane (25 mL) was added dropwise to a solution of N-[1-(2-aminoethyl)-1H-pyrazol-5-yl]acetamide dihydrochloride (1) (3.588 g, 15 mmol) and triethylamine (3.440 g, 4.8 mL, 34 mmol) in dichloromethane (50 mL) under stirring at room temperature. The resulting mixture was stirred at room temperature for 6 hours until the initial amine had completely disappeared (control by TLC, LCMS). The precipitated residue of a triethylamine salt was filtered off. The filtrate was concentrated under reduced pressure. The resulting residue was treated with acetone. The formed precipitate was filtered off, washed with acetone and diethyl ether, and dried in air and under reduced pressure. Compound 3 was obtained in the form of a white solid (1.101 g, 26%). Rf (3) 0.52 (DCM/isopropyl alcohol, 5:1+2 drops of acetic acid). LC/MS, an individual peak at a retention time of 1.06 min, [M+H]⁺=265 (condition A). HPLC under condition 1, individual peak at a retention time of 1 min. ¹H NMR (400.13 MHz, DMSO-d6, δ, m.d., J/Hz): 1.81 (quint, 2H, CH₂CH₂CH₂, J=6.5 Hz), 1.97 (s, 3H, Me), 2.55 (t, 4H, CH₂CH₂CH₂, J=6.5 Hz), 3.96 (t, 2H, CH₂NCO, J=6.3 Hz), 4.05 (t, 2H, CH₂Pyr, J=6.3 Hz), 6.38 (s, 1H, CH), 7.52 (s, 1H, CH=N), 10.35 (s, 1H, NH).

### 2. Synthesis of glutaryl derivatives of dipeptides as illustrated by the synthesis of compound 94

4 mL of 25% aqueous ammonia solution were added to a solution of 10 g (28.4 mmol) of Boc-leucine p-nitrophenyl ester in 20 mL of dimethylformamide; the reaction mixture was aged for 3 hours at room temperature and evaporated to dryness, and the residue was triturated with ether, filtered off, and washed with ether to obtain 2.5 g (10.87 mmol) of Boc-leucine amide (Rf - 0.6: chloroform:methanol:32% acetic acid (15:4:1)). The residue was dissolved in 25 mL of trifluoroacetic acid, aged for 1 hour, and evaporated; the residue was triturated with ether, filtered off, and dissolved in 50 mL of dimethylformamide, then NMM was added to pH 8.5, after that 5.14 g (10.8 mmol) of di-Boc-L-histidine p-nitrophenyl ester were added to the solution, the reaction mixture was allowed to stand over night at room temperature, dimethylformamide was evaporated, and the residue was dissolved in 10 mL of a mixture of ethyl acetate-hexane (8+2 mL) and passed through a column (3×17 cm) filled with suspension of silica gel in the same mixture. The product was eluted with ethylacetate, and the fractions comprising the target compound were combined and evaporated to dryness. The yield of the product was 3.95 g (9.6 mmol) with Rf-0.8 (chloroform:methanol:32% acetic acid (15:4:1)). The dipeptide amide was dissolved in 25 mL of trifluoroacetic acid, aged for 1 hour at room temperature, and evaporated; the residue was triturated with ether, filtered off, and dissolved in 25 mL of dimethylformamide, then NMM was added to pH 8.5, after that 1.14 g (10 mmol) of glutaric anhydride was added to the solution by three portions with intervals of 15-20 min, the reaction mixture was aged 2 hours at room temperature, and evaporated, 100 mL of ethyl acetate were added to the residue and allowed to stand over night, the precipitate was filtered off, dissolved in 100 mL of water, and extracted with 50 mL of ethyl acetate, and the aqueous layer was evaporated to half its volume, treated with activated carbon, evaporated, dissolved in 100 mL of a 2% acetic acid, and lyophilized. The yield of the target product was 3.5 g (91%) with Rf-0.75 (chloroform:methanol:32% acetic acid (5:3:1)). LC/MS, an individual peak at a retention time of 0.2 min, [M+H]⁺=382 (condition A). HPLC under condition 1, individual peak at a retention time of 11.8 min. ¹H NMR (400.13 MHz, DMSO-d6, δ, m.d., J/Hz) : 0.82, 0.87 (d, 6H, CH₂CH(CH₃)₂, J=6.1 Hz); 1.51 (m, 3H, CH₂CH (CH₃)₂) ; 1.67 (quin, 2H, CH₂CH₂CH₂, J=7.5 Hz); 2.15 (m, 4H, CH₂CH₂CH₂); 2.86, 2.98 (m, 2H, CCH₂CH); 4.19 (m, 1H, NCH); 4.52 (m, 1H, CCH₂CH); 7.02, 7.51 (br s, 2H, NH₂); 7.07 (s, 1H, CCH); 7.90 (d, 1H, NH, J=8.2 Hz); 8.08 (d, 1H, NH, J=7.9 Hz); 8.23 (s, 1H, NCHN)

### 3. Synthesis of γ-aminobutyric acid amides as illustrated by the synthesis of compound 103

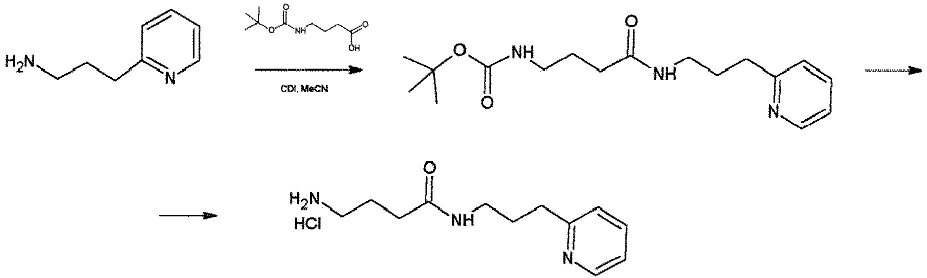

A solution of N-Boc-γ-aminobutyric acid imidazolide obtained from 2.23 mL (0.011 mol) of N-Boc-γ-aminobutyric acid and 1.95 g (0.012 mol) of carbonyl imidazolide, in 10 mL of anhydrous acetonitrile was added to a solution of 1.36 g (0.01 mol) of 2-(3-aminopropyl)pyridine in 25 mL of anhydrous acetonitrile. The reaction mixture was stirred for 4 hours at 45°C, the solvent was removed under vacuum, and the residue was dissolved in 300 mL ether, washed with a saturated aqueous solution of sodium hydrogen carbonate (3×100 mL). The organic layer was dried over sodium sulfate, the solvent was removed under vacuum, and the residue was dried under vacuum of an oil pump to a constant weight. The residue was dissolved in 80 mL of anhydrous ether, and 35 mL of a 5% solution of hydrogen chloride in anhydrous methanol were added thereto. The reaction mixture was stirred at room temperature until the initial compound had disappeared (according to TLC data) (for about 4 hours), solvents were removed under vacuum, the residue was triturated with anhydrous ether, the ether was decanted, and the procedure was repeated. The residue under ether was allowed to stand at 0°C for 8 hours. The precipitate was filtered off, washed with anhydrous ether (3×10 mL), and dried under vacuum. The yield was 1.62 g (63%), Rf (chloroform - methanol, 4/1) was 0.48. LC/MS, an individual peak at a retention time of 0.5 min, [M+H]⁺=222 (condition B). HPLC under condition 2, individual peak at a retention time of 4.00 min. ¹H NMR (400.13 MHz, DMSO-d6, δ, m.d., J/Hz): 1.78 (quin, 2H, CH₂CH₂CH₂NH₂, J=7.5 Hz); 1.87 (quin, 2H, CH₂CH₂CH₂NH, J=7.3 Hz); 2.20 (t, 2H, CH₂CONH, J=7.3 Hz); 2.77 (m, 2H, CH₂NH₂); 3.01 (t, 2H, CH₂-Pyr, J=7.5 Hz); 3.09 (q, 2H, CH₂NH, J=7.5 Hz); 7.80 (m, 1H, 5-Pyr); 7.89 (d, 1H, 3-Pyr, J=7.8 Hz); 8.15 (m, 1H, 4-Pyr); 8.39 (br t, 1H, NH); 8.74 (d, 1H, 6-Pyr, J=4.1 Hz).

### 4. Synthesis of pyroglutamic acid amides as illustrated by the synthesis of compound 178

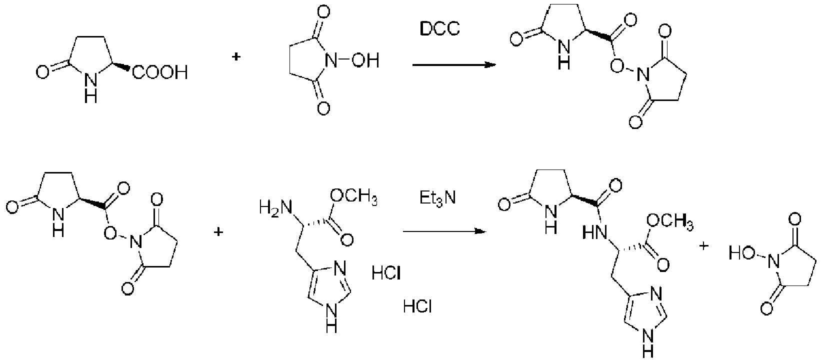

3.4 g (29.8 mmol) HONSu and a solution of 6.4 g (29.8 mmol) of DCC in 10 mL of DMF were added to a solution of 3.5 g (27.1 mmol) of pyroglutamic acid in 25 mL of DMF, and cooled to 0°C. The reaction mixture was stirred for 1 hour at 0°C and for 16 hours at room temperature. The residue was separated by filtration and washed with ethyl acetate (3×10 mL). The solvent of combined filtrates was removed under vacuum until stable foam was formed. The yield was 5.8 g (95%). Rf = 0.6 (chloroform - methanol (1:1)).

2.35 g (9.7 mmol) of histidine dihydrochloride and 2.87 mL (19.4 mmol) of triethylamine were added to a solution of 2.2 g (9.7 mmol) of pyroglutamic acid N-oxysuccinimide ester in 20 mL of DMF. The reaction mixture was stirred for 30 min at room temperature. The solvent was removed under vacuum, and the residue was dissolved in 20 mL of ethyl acetate and washed with 1% citric acid (3×10 mL) and water to a neutral reaction, and a saturated solution of sodium chloride. The organic layer was dried over anhydrous sodium sulfate. The solvent was removed under vacuum. The yield was 2.36 g (80%), Rf = 0.3 (chloroform - methanol (4:1)). LC/MS, individual peak at a retention time of 0.23 min, [M+H]⁺=281 (condition C). HPLC under condition 1, individual peak at a retention time of 7.0 min. ¹H NMR (400.13 MHz, DMSO-d6, δ, m.d., J/Hz): 1.83-2.23 (m, 4H, CH₂CH₂CH), 2.93 (m, 2H, CH₂CH) , 3.61 (s, 3H, OCH₃); 4.02 (m, 1H, CH₂CH₂CH); 4.50 (m, 1H, CH₂CH); 6.84 (s, 1H, CCH); 7.60 (s, 1H, NCHN); 7.78 (s, 1H, NH), 8.05 (d, 1H, NH, J=7.8 Hz).

### 5. Synthesis of amides of 3-(4-imidazolyl)acrylic acid and 3-(4-imidazolyl)propionic acid as illustrated by the synthesis of compound 85

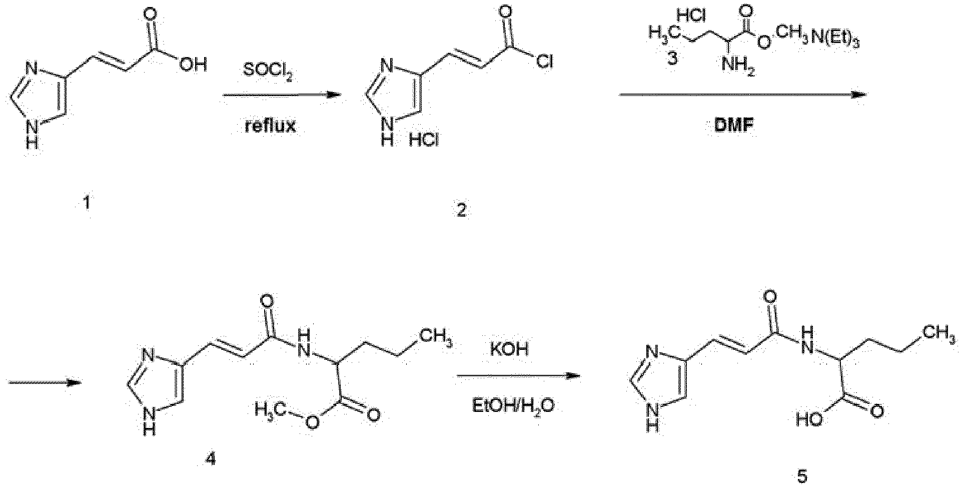

Acid 1 (1 g, 0.007 mmol) was added to 5 mL of cold thionyl chloride by small portions under vigorous stirring. When the total amount of acid 1 was added, the reaction mixture was stirred under heating for 3-4 hours and then cooled to room temperature. Thionyl chloride was removed under reduced pressure. Resulting product 2 was carefully washed on the Schott filter with absolute toluene (3×20 mL). The yield of technical product 2 was 1.4 g (99%). The product was used at the next step without further purification.

The initial chloroanhydride of acid 2 (1.4 g, 0.009 mol) was suspended in dry DMF, and amine hydrochloride 3 (1.34 g, 0.0098 mol) and triethylamine (4 mL, 0.036 mol) were added under stirring. The reaction mixture was stirred for 10 hours at room temperature. Upon completion of the reaction, triethylamine hydrochloride was filtered off, and the solvent was removed under vacuum. The residue was purified by column chromatography on silica gel with a solvent system: methylene chloride - methanol (15:1). The yield of pure intermediate 4 was 0.65 g (35%).

The initial ether 4 (0.65 g, 0.0026 mol) was dissolved in 10 mL of 50% ethanol, and KOH (0.18 g, 0.0032 mol) was added under stirring. The stirring was continued for 5-6 hours at room temperature. Upon completion of the reaction (control by TLC in the system of methylene chloride - methanol (10:1)), the solution was filtered through a thick layer of celite, and the solvent was removed under vacuum. The obtained potassium salt was washed on a filter with acetone and re-disolved in absolute ethanol, and target product 5 was precipitated by adding concentrated HCl (1 eqv.). The yield of product 5 was 0.55 g (90%). LC/MS, an individual peak at a retention time of 0.2 min, [M+H]⁺=238 (condition A). HPLC under condition 1, individual peak at a retention time of 11.8 min.

### 6. Synthesis of dicarboxylic acid amides comprising mono- or dimethyl derivatives in a glutaryl moiety, as illustrated by the synthesis of compound 45

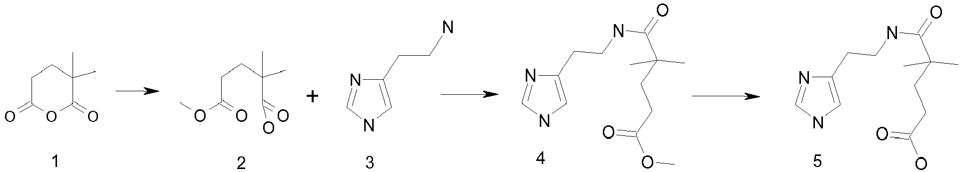

Compound 1 (2.6 g, 0.018 mol) was dissolved in 50 mL of methanol and stirred for 24 hours at room temperature. The solvent was removed under vacuum, and the resulting technical product 2 (3.2 g, 100%) was used at the next step without further purification.

1,1'-carbonyldiimidazole (8.3 g, 0.052 mol) was added to a solution of compound 2 (6.4 g, 0.037 mol) in 50 mL of dimethylformamide at room temperature. The reaction mixture was stirred at room temperature for 2 hours, and then a solution of histamine (4.1 g, 0.037 mol) in 20 mL of dimethylformamide was added thereto, and the mixture was stirred for 10 hours at room temperature. The solvent was removed under vacuum. The residue was purified by column chromatography on silica gel Purasil™ 60Å, 230-400 µm mesh (38-63 µm) (Whatman) (column: diameter = 50 mm, height = 400 mm, eluent: chloroform - methanol (4:1)). The yield of product 3 was 2.6 g (26%) in the form of light-yellow oil.

A solution of potassium hydroxide (0.65 g, 0.052 mol) in 25 mL of water was added to a solution of compound 3 (2.6 g, 0.010 mol) in 25 mL of ethanol at room temperature, and then the reaction mixture was stirred at room temperature for 10 hours. Further, the mixture was acidified with hydrochloric acid (1.2 mL, 0.052 mol), the solvent was removed under vacuum, and the residue was purified by column chromatography on silica gel Purasil™ 60Å, 230-400 µm mesh (38-63 µm) (Whatman) (column: diameter = 30 mm, height = 300 mm, eluent: chloroform - methanol (1:1)). The yield of product 4 was 1.1 g (44%). The white crystalline compound was freely soluble in water, Rf = 0.23 in the system of chloroform - methanol (1:1). LC/MS, an individual peak at a retention time of 0.55 min, [M+H]⁺=254 (condition E). HPLC under condition 3, individual peak at a retention time of 11.1 min. ¹H NMR (400.13 MHz, DMSO-d6, δ, m.d., J/Hz): 1.05 (s. 6H. C*H*₃); 1.67 (m, 2H, C*H*₂CH₂COOH), 2.06 (m, 2H, C*H*₂COOH), 2.62 (t, 2H, CH₂C*H*₂C, J= 7.4 Hz); 3.26 (q, 2H, CH₂C*H*₂NH, J= 7.4 Hz); 6.77(s, 1H, CCH); 7.51 (s, 1H, NCH); 7.60 (br s, 1H, NH)

### 7. Synthesis of dicarboxylic acid amides - comprising a hydroxyl group as a substituent at the α-position of a glutaryl moiety, as illustrated by the synthesis of compound 67

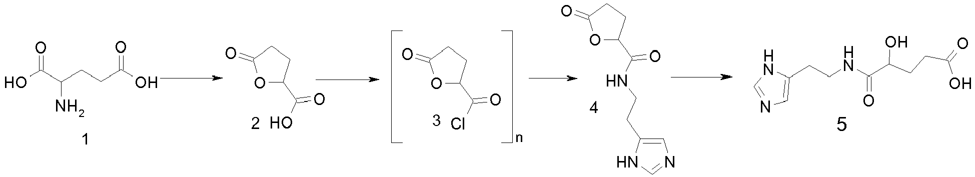

A solution of sodium nitrate (10.0 g, 144.0 mmol) in 140 mL of water and a solution of concentrated sulfur acid (7.2 g, 73.0 mmol) in 140 mL of water were added dropwise simultaneously to suspension of compound 1 (18.0 g, 122.0 mmol) at the temperature of the reaction mixture of not higher than 30°C, and the mixture was stirred for 10 hours at room temperature. The solvent was removed under vacuum, and the residue was extracted with hot acetate (3x100 mL). The solvent was removed under vacuum, and the residue was extracted with hot methylene chloride (2x100 mL). The solvent was removed under vacuum, and the yield of product 2 in the form of colorless syrup was 8 g (51%).

Some drops of dimethylformamide were added to a solution of compound 2 (10.0 g, 77.0 mmol) in 150 mL of methylene chloride. Then, the reaction mass was cooled to 5°C, and a solution of oxalyl chloride (9.78 g, 77.0 mmol, 6.6 mL) in 30 mL of methylene chloride was added dropwise thereto at 5-10°C. The reaction mass was stirred for 3 hours at room temperature, and the solvent was removed under vacuum. The residue was dissolved in 70 mL of tetrahydrofuran and added dropwise to suspension of histamine (5.0 g, 45.0 mmol) and potash (12.7 g, 92.0 mmol) in 100 mL of dimethylformamide at 0°C. The reaction mass was stirred for 10 hours at room temperature and filtered off, and the filtrate was removed under vacuum. The residue obtained in the form of oil was washed with ether (1x50 mL), hot tetrahydrofuran (2x50 mL), and then with acetonitrile (1x50 mL). The solvent was decanted, the residue was removed under vacuum, and the yield of product 4 in the form of dark red oil was 8 g (46%).

A solution of sodium hydroxide (0.52 g, 13.0 mmol) in 1 mL of water was added to a solution of compound 4 (3.0 g, 13.4 mmol) in the mixture of acetonitrile - water (10:1, 40 mL), and stirred for 10 min at room temperature. The solvent was removed under vacuum, and the residue was dissolved in 10 mL of water and purified by ion-exchange chromatography (Dowex 50WX8-400, filter: d=60 mm, h=35 mm; eluent - water (250 mL), then 5% aqueous ammonia (250 mL)). The control was carried out by thin-layer chromatography (chloroform-methanol-5% aqueous solution of ammonia (1:1:0.1), Rf=0.7). The obtained fractions were combined, the solvent was removed under vacuum, the residue was boiled in the mixture of acetonitrile-methanol (4:1, 70 mL) and filtered off, and the residue was dried in a dryer at 70°C to constant weight. The yield of product 5 was 1.6 (49%). The white crystalline compound was freely soluble in water, Rf = 0.15 in the system of chloroform - methanol (1:1). LC/MS, an individual peak at a retention time of 0.5 min, [M+H]⁺=242 (condition D). HPLC under condition 3, individual peak at a retention time of 7.4 min. ¹H NMR (400.13 MHz, DMSO-d6, δ, m.d., J/Hz): 1.65, 1.87 (m, 1H+1H, CH₂C*H*₂CH), 2.23 (m, 2H, C*H*₂CH₂CH), 2.64 (t, 2H, CH₂C*H*₂C, J= 7.2 Hz); 3.31 (q, 2H, CH₂C*H*₂NH, J= 6.7 Hz); 3.85 (dd, 1H, CH₂CH₂C*H*, J = 4.2, 7.7 Hz), 6.79 (s, 1H, CCH); 7.52 (s, 1H, NCHN), 7.82 (t, 1H, NH, J = 5.7 Hz).

### 8. Synthesis of 3-(4-imidazolyl)acrylic acid and 3-(4-imidazolyl)propionic acid amides as illustrated by the synthesis of compound 68

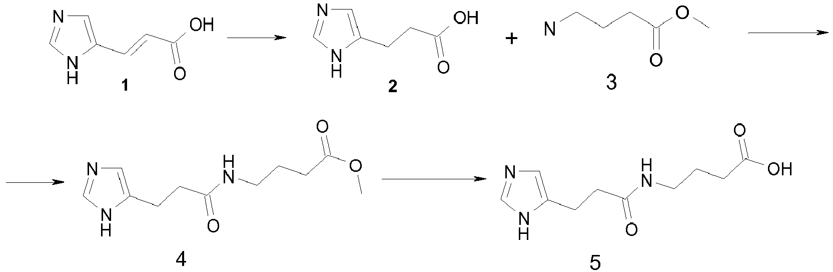

A catalyst of 10% Pd/C (5.0 g) was added to a solution of compound 1 (21.3 g, 0.154 mol) in a 30% aqueous solution of methanol (1200 mL), and the reaction mixture was hydrated by supplying hydrogen at 1 atm. and at 50°C for 48 hours. After that, the mixture was cooled to room temperature, the catalyst was filtered off through a paper filter on a funnel, and the solvent was removed under vacuum. The residue was crystallized from diethyl ether (150 mL). The obtained residue was filtered off and dried in air to constant weight. The yield of product 2 was 16.5 g (76%).

1,1'-carbonyldiimidazole (19.1 g, 0.118 mol) was added under stirring to a solution of compound 2 (16.5 g, 0.118 mol) in 200 mL of dimethylformamide. The reaction mixture was heated to 80°C and stirred for 2 hours. The mixture was cooled, and triethylamine (13.1 g, 0.129 mol) and compound 3 (19.9 g, 0.129 mol) were added thereto by small portions under vigorous stirring. The reaction mixture was stirred for 12 hours at room temperature and then filtered. The filtrate was removed under vacuum. The resulting product in the form of oil was purified by column chromatography (column: diameter = 90 mm, sorbent layer height = 75 mm, eluent: ethylacetate - methanol (15:1)). Product 4 was obtain in the form of yellowish oil; the yield was 18 g (63%).

Dry KOH (8.44 g, 0.15 mol) was mixed by small portions to a solution of product 4 (18 g, 0.075 mol) in 200 mL of water. The reaction mixture was stirred for 9 hours at room temperature. Upon completion of the reaction (control of the initial regent by TLC in the system of chloroform - methanol (5:1), Rf=0.7), the solvent was removed under vacuum. The obtained potassium salt of the acid was washed on a filter with acetone, re-dissolved in water, and acidified by adding concentrated HCl (15.2 mL, 2 eqv.) to pH 5-6. Water was removed under vacuum, and the residue was suspended in methanol (50 mL) and filtered. The filtrate was removed under vacuum, the residue was purified by column chromatography on silica gel Purasil™ 60Å, 230-400 µm mesh (38-63 µm) (Whatman) (column: d = 60 mm, sorbent layer height = 80 mm, eluent: cloroform - methanol - 25% aqueous ammonia solution (1:1:0.05)). The resulting product 5 in the form of yellowish oil freely soluble in water was dried to a constant weight. The yield of product 5 was 3 g (18%). The yellowish crystalline compound was freely soluble in water, Rf = 0.2 in the system of chloroform - methanol (1:1). LC/MS, an individual peak at a retention time of 0.28 min, [M+H]⁺=226 (condition E). HPLC under condition 3, individual peak at a retention time of 9.6 min. ¹H NMR (400.13 MHz, DMSO-d6, δ, m.d., J/Hz): 1.59 (quin 2H, CH₂C*H₂*CH₂, J= 7.2 Hz); 2.18 (t, 2H, C*H*₂COOH, J= 7.2 Hz); 2.34 (t, 2H, C*H*₂CONH, J= 7.8 Hz); 2.70 (t, 2H, C*H*₂C, J= 7.8 Hz); 3.03 (q, 2H, C*H*₂NH, J= 6.7 Hz); 6.70 (s, 1H, CCH); 7.48 (s, 1H, NCHN); 7.86 (br t, 1H, NH).

### 9. Synthesis of amides, which are derivatives of 3-aminosulfonylpropionic acid, as illustrated by the synthesis of compound 145

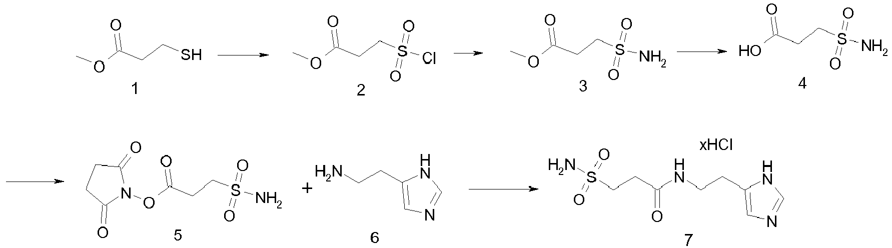

Sulfuryl chloride (64.8 mL, 0.8 mol) was added dropwise under vigorous stirring to a mixture of finely ground potassium nitrate (84.14 g, 0.883 mol) and compound 1 (36.9 mL, 0.333 mol) at 0-10°C (the temperature of the reaction mixture must not be higher than 0°C). The reaction mixture was stirred for 1 hour at 0°C. The cooling was removed, and the mixture was stirred at room temperature for additional 16-18 hours. A saturated aqueous solution of NaHCO₃ was added to pH 7-8, the exatraction was carried out with methyl acetate (2x200 mL), the organic phase was dried over sodium sulfate, and the solvent was removed under vacuum. The technical product in the form of a yellow residue was used at the next step without further purification. The yield of technical product 2 was 46.5 g (75%).

Diethyl ether (500 mL) was saturated with ammonia, while cooling to 0°C, and was added to a solution of compound 2 (46.5 g, 0.249 mol) in 500 mL of diethyl ether in a single portion under vigorous stirring. The reaction mass was stirred for 1 hour at room temperature. The precipitate was filtered. The filtrate was removed under vacuum, 30 mL of cold ether was added to the residue, and the precipitate was filtered off, washed with 20 mL of cold ether, and dried in air to constant weight. The yield of the resulting white or slightly yellowish crystalline product was 24.7 g (60%).

An aqueous solution of potassium hydroxide (6.71 g, 0.120 mol) in 50 mL of water was added to a solution of compound 3 (10 g, 0.066 mol) in 50 mL of water. The reaction mass was refluxed for 1 hour, cooled to room temperature, and 10% HCl was added thereto to pH 2-3 and removed under vacuum to dryness. Acetone (150 mL) was added to the resulting residue and stirred for 30 min, and the residue was filtered off and washed with acetone (100 mL). The filtrate was separated and removed under vacuum, and obtained colorless crystalline product 4 was dried in air to a constant weight.

A solution of N,N'-dicyclohexylcarbodiimide (6.2 g, 0.030 mol) in 50 mL dioxane was added dropwise to a solution of compound 4 (4.2 g, 0.027 mol) and hydroxysuccinimide (3.47 g, 0.030 mol) in a dioxane-aceton mixture (9:1, 400 mL) at room temperature. The reaction mass was stirred for 12 hours. The precipitate was filtered off, washed with 50 mL of dioxane, and the organic layer was removed under vacuum. Ethyl acetate (50 mL) was added to the resulting residue, the precipitate was filtered off, washed with 30 mL of ethyl acetate and dried in air to a constant weight. The yield of the white crystalline product 5 was 3.3 g (48%).

Compound 5 (3.3 g, 0.013 mol) was added to a solution of compound 6 (1.33 g, 0.012 mol) in 30 mL of dimethylformamide. The reaction mass was stirred for 16 hours at room temperature. A solvent excess was removed under vacuum, the resulting residue was purified by column chromatography on silica gel Purasil™ 60Å, 230-400 µm mesh (38-63 µm) (Whatman) (sorbent layer height = 40 mm, diameter = 20 mm; eluent: methanol - chloroform (1:5)). The obtained light yellow oil was dissolved in 20 mL of methanol, 4M HCl (10 mL) was added in ethyl acetate thereto, and allowed to stand for 10 hours at room temperature. The precipotate was filtered off, washed with a small amount of methanol and dried in air. The yield of the white crystalline product that was freely soluble in water was 1.88 g (82%), Rf = 0.45 in the system of chloroform - methanol (1:1) . LC/MS, an individual peak at a retention time of 0.5 min, [M+H]⁺=247 (condition D). HPLC under condition 1, individual peak at a retention time of 7.9 min. ¹H NMR (400.13 MHz, DMSO-d6, δ, m.d., J/Hz): 2.53 (t, 2H, C*H*₂C, J=7.8 Hz), 2.79 (t, 2H, C*H*₂C=O, J=6.7 Hz), 3.16 (t, 2H, C*H₂*S, J=6.7 Hz), 3.34 (q, 2H, C*H₂*NH, J=6.4 Hz), 6.84(s, 2H, NH₂); 7.43 (s, 1H, CCH), 8.26 (t, 1H, NH, J=5.6 Hz), 9.00 (s, 1H, NCHN); 14.51 (br s, 1H, NH).

### 10. Synthesis of dicarboxylic acid amides comprising a C₁-C₆alkyl-substituted carboxyl group in a glutaryl moiety, as illustrated by the synthesis of compound 63

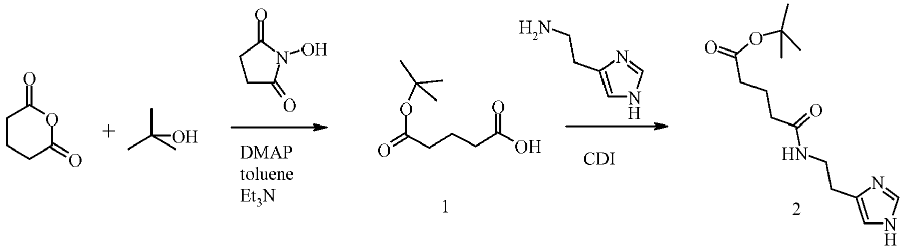

A mixture of glutaric anhydride (10 g, 87.6 mmol), N-hydroxysuccinimide (3 g, 2.1 mmol), 4-dimethylaminopyridine (1.07 g, 8.8 mmol), anhydrous tert-butanol (24 mL, 262 mmol), and triethylamine (3.6 mL, 25.8 mmol) in dry toluene was mixed at room temperature for 30 min, then boiled for 8 hours, and allowed to stand over night at room temperature. The mixture was diluted with ethyl acetate (250 mL), washed with a 10% solution of citric acid (3x100 mL), then with a saturated salt solution (2x50 mL), and dried over anhydrous sodium sulfate, and the solvent was removed under vacuum. The product was isolated by flash chromatography on silica gel with an elution mixture of ethyl acetate - hexane (1:1). The yield of ether (1) in the form of colorless oil was 4.5 g (27%), [M+H]⁺=187.49.

1,1'-carbonyldiimidazole (2.26 g, 14 mmol) was added to a solution of monoether (1) (2.2 g, 11.7 mmol) in 50 mL of anhydrous tetrahydrofuran, and the mixture was boiled for 1 hour. Then, the mixture was cooled to room temperature, and histamine gihydrochloride (2.15 g, 11.7 mmol) and triethylamine (3.28 mL, 23.4 mmol) were added thereto. The reaction mass was stirred for 8 hours at room temperature, poured into 100 mL of a 10% potash solution, extracted with dicloromethane (3x75 mL), dried over anhydrous sodium sulfate, and the solvent was removed under vacuum. The target product was isolated by flash chromatography on silica gel, with an elution mixture of dichloromethane - methanol (10:1). After recrystallization, the yield of the target product in the form of white crystals was 1.1 g (33%). LC/MS, an individual peak at a retention time of 0.93, min [M+H]⁺=282 (condition G). HPLC under condition 6, individual peak at a retention time of 13.4 min. ¹H NMR (400.13 MHz, DMSO-d6, δ, m.d., J/Hz): 1.38 (s, 9H, CH₃CH), 1.68 (quin, 2H, CH₂CH₂CH₂, J=7.5 Hz); 2.06 (t, 2H, CH₂CONH, J=7.4 Hz); 2.15 (t, 2H, CH₂COO, J=7.4 Hz); 2.60 (t, 2H, CH₂C, J=7.4 Hz); 3.24 (m, 2H, CH₂N); 6.73 (br s, 1H, CCH); 7.46 (d, 1H, NCHN, J=1 Hz); 7.70 (br s, 1H, NH); 11.67 (br s, 1H, NH).

### 11. Synthesis of dicarboxylic acid amides comprising a C₁-C₆alkyl-substituted carboxyl group in a glutaryl moiety, as illustrated by the synthesis of compound 64

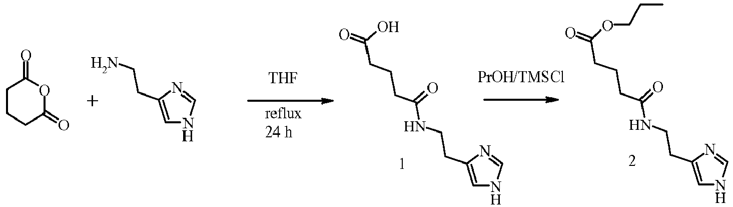

A mixture of glutaric anhydride (4.8 g, 42 mmol), histamine dihydrochloride (6 g, 32.6 mmol), and triethylamine (13.7 mL, 97.8 mmol) was boiled in 150 mL of anhydrous terahydrofuran for 24 hours, cooled to room temperature, and the residue was filtered off, washed with tetrahydrofuran, and dried at 70°C for 10 hours. 10 g of acid (1) was obtained in the form of triethylamine salt that was used at the next step without further purification. [M+H]+=225.99.

2.54 mL (20 mmol) of trimethylchlorosilane were added dropwise to suspension of acid (1) (3.26 g, 10 mmol) in 50 mL of anhudrous n-propanol. The reaction mass was stirred at room temperature for 4 hours, diluted with 100 mL of ethyl acetate, washed with a 10% solution of potash (2x100 mL), then with a saturated salt solution (2x50 mL), dried over anhydrous sodium sulfate, and the solvent was removed under vacuum. The product was isolated by flash chromatography on silica gel, with an elution mixture of dichloromethane - methanol (10:1). After recrystallization from ethyl acetate, the yield of the target product in the form of white crystals was 2 g (74%). LC/MS, an individual peak at a retention time of 0.4 min, [M+H]⁺=268 (condition G). HPLC under condition 6, individual peak at a retention time of 11.8 min. ¹H NMR (400.13 MHz, DMSO-d6, δ, m.d., J/Hz): 0.87 (t, 3H, CH₃CH₂, J=7.4 Hz), 1.56 (t, 2H, CH₂CH₂CH₂, J=7.1 Hz), 1.73 (quin, 2H, CH₂CH₂CH₂) , 2.07 (quin, 2H, CH₂CO, J=7.5 Hz); 2.06 (t, 2H, CH₂COO, J=7.5 Hz); 2.60 (t, 2H, CH₂C, J=7.4 Hz); 3.25 (m, 2H, CH₂N); 3.95 (t, 2H, CH₂O, J=6.7 Hz); 6.73 (br s, 1H, CCH); 7.46 (d, 1H, NCHN, J=1 Hz); 7.72 (br s, 1H, NH); 11.7 (br s, 1H, NH).

### 12. Synthesis of pyroglutamic acid amides as illustrated by the synthesis of compound 185

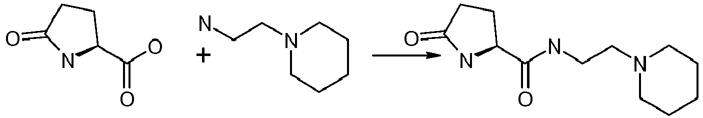

Suspension of the acid (1.55 g, 12 mmol), N,N,N',N'-tetramethyl-O-(benzotriazol-1-yl)uronium tetrafluoroborate (3.85 g, 12 mmol), and triethylamine (1.66 mL, 12 mmol) in 50 mL of dichloromethane was stirred for 10 min, an amine (1.54 g, 12 mmol) was added, and the mixture was stirred for additional 1 hour. The solvent was removed under vacuum to dryness, and the isolation was carried out by column chromatography on silica gel, with an elution mixture of dichloromethane - methanol - aqueous ammonia (5:1:0.01). Additional purification was carried out by paraffin HPLC on sorbent C18 with a gradient elution system of 0.1% formic acid in water - 0.1 formic acid in acetonitrile, and the drying was carried out under vacuum. The yield of the target product in the form of yellow crystals was 1.43 g (50%). LC/MS, an individual peak at a retention time of 0.2 min, [M+H]⁺=240 (condition G). HPLC under condition 3, individual peak at a retention time of 8.2 min. ¹H NMR (400.13 MHz, DMSO-d6, δ, m.d., J/Hz): 1.43 (m, 2H, CH₂CH₂CH₂CH₂CH₂); 1.59 (m, 4H, CH₂CH₂CH₂CH₂CH₂); 1.87 (dddd, 1H, CH₂CH₂CH, J=12. 0; 9.0; 5.6; 4.5 Hz); 2.11 (m, 2H, CH₂CH₂CO); 2.24 (dddd, 1H, CH₂CH₂CH, J=12.0; 9.8; 8.4; 6.8 Hz); 2.62 (br s, 6H, NCH₂); 3.28 (m, 2H, NHCH₂CH₂N); 3.96 (ddd, 1H, NHCHCH₂, J=8.6; 4.6; 1.0 Hz); 7.68 (br s, 1H, NH); 7.94 (br s, 1H, NH).

### 13. Synthesis of compounds comprising the -C-O-C(=O)- bond as illustrated by the synthesis of compound 72

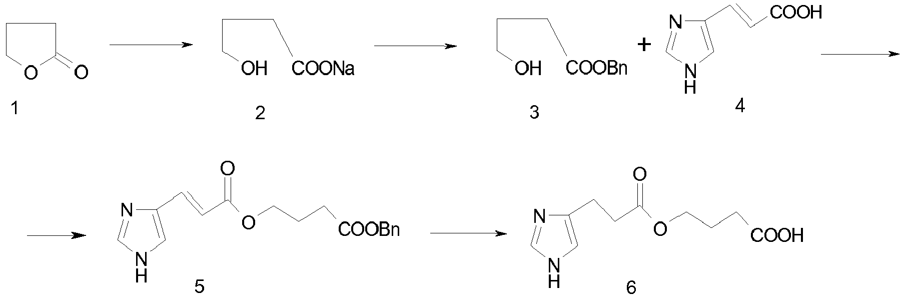

Compound 1 (25 mL, 0.32 mol) was added to a solution of NaOH (13 g, 0.32 mol) and heated to 45°C. The reaction mass was stirred for 12 hours at 60°C, and then methanol was removed under vacuum. The resulting residue was washed with diethyl ether (2x250 mL) and dried in a dryer at 40°C for 12 hours, and the yield of product 2 was 38 g (95%).

Benzyl bromide (29 mL, 0.25 mol) was added to suspension of compound 2 (38 g, 0.30 mol) in 100 mL of dimethylformamide. The reaction mass was aged under stirring for 2 hours at 60°C. The solvent was removed under vacuum, and the resulting residue was diluted with 10% aqueous NaHCO₃ (200 mL) and extracted with CCl₄ (3x250 mL). Organic fractions were combined. The solvent was removed under vacuum, and the residue was dried in air to a constant weight. The yield of product 3 was 47 g (80%).

Compound 4 (6.3 g, 0.046 mol) was added to a solution of compound 3 (9.0 g, 0.046 mol) and triphenylphosphine (15.0 g, 0.056 mol) in 100 mL of dimethylformamide. The resulting suspension was colled to -5°C, and diisopropyl azodicarboxylate (11 mL, 0.056 mol) was slowly added at a temperature of not higher than +5°C. The reaction mixture was stirred for 12 hours at room temperature. The solvent was removed under vacuum, and the residue was purified by column chromatography on silica gel Purasil™ 60Å, 230-400 µm mesh (38-63 µm) (Whatman) (column: d = 50 mm, sorbent layer height = 75 mm, system of hexane - ethyl acetate - methanol (300:150:1)). The yield of product 5 was 6.7 g (45%).

10% Pd/C (0.5 g) was added to a solution of compound 5 (4.9 g, 0.016 mol) in tetrahydrofuran (50 mL). The reaction mixture was hydrated with hydrogen at 80 atm. for 12 hours. Upon completion of the reaction, the mixture was passed through a celite layer (10 mm). The solvent was removed under vacuum. The residue was purified by column chromatography on silica gel Purasil™ 60Å, 230-400 µm mesh (38-63 µm) (Whatman) (column: diameter = 20 mm, sorbent layer height = 40 mm, system of chloroform - methanol (4:1)). The yield of product 6 was 2.7 g (75%). The product was in the form of colorless crystals, Rf = 0.2 in a system of chloroform-methanol (4:1) . LC/MS, an individual peak at a retention time of 1.9 min, [M+H]⁺=227 (condition D). HPLC under condition 1, individual peak at a retention time of 13.6 min. ¹H NMR (400.13 MHz, DMSO-d6, δ, m.d., J/Hz): 1.78 (quin, 2H, CH₂C*H₂*CH₂, J= 6.9 Hz); 2.25 (t, 2H, C*H*₂COOH, J=7.1 Hz); 2.58 (t, 2H, C*H*₂CO, J=7.5 Hz); 2.74 (t, 2H, C*H*₂C, J=7.5 Hz); 4.01 (t, 2H, C*H*₂O, J=6.5 Hz); 6.74 (s, 1H, CCH); 7.50 (s, 1H, NCHN).

### 14. Synthesis of pyroglutamic acid amides as illustrated by the synthesis of compound 188

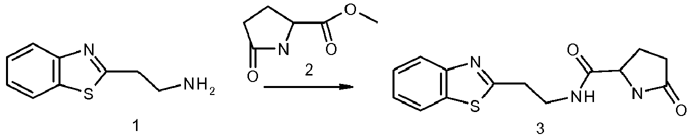

A solution of freshly distilled (or freshly obtained) amine 1 (3 g, 0.0168 mol) and pyroglutamic acid 2 (2.4 g, 0.0168 mol) in 10 mL of methanol was aged for a week, diluted with 10 mL of ether, and the precipitate was filtered off. The product was washed with dry ether supplemented with 10% methanol. The yield was 4 g (82.2%). LC/MS, an individual peak at a retention time of 3.6 min, [M+H]⁺=290 (condition D). HPLC under condition 1, individual peak at a retention time of 10.6 min. ¹H NMR (400.13 MHz, DMSO-d6, δ, m.d., J/Hz): 1.85-2.23 (m, 4H, CH₂CH₂CH), 3.26 (m, 2H, CCH₂CH₂N); 3.55 (m, 2H, CCH₂CH₂N); 3.95 (m, 1H, CH₂CH₂CH); 7.41 (t, 2H, benzothiazole, J=7.6 Hz), 7.49 (t, 2H, benzothiazole, J=7.6 Hz), 7.77 (s, 1H, NH), 7.95 (d, 2H, benzothiazole, J=8.0 Hz), 8.06 (d, 2H, benzothiazole, J=8.1 Hz); 8.19 (t, 1H, NH, J=5.7 Hz).

### 15. Synthesis of C₁-C₆alkylamides, as illustrated by the synthesis of compound 141

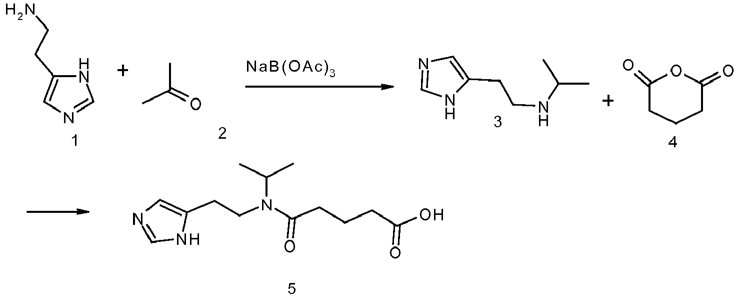

A well-stirred mixture of histamine (12 g, 0.108 mol), acetone (6.27 g, 0.108 mol), acetic acid (9.7 g, 0.162 mol), and triacetoxyborohydride (22.9 g, 0.108 mol) in 400 mL of methylene was aged for 3 days at 30-35°C. 100 mL of 20% NaOH was added thereto. The layers were separated, and extracted with methylene chloride supplemented with isopropanol (5*50). The solution was filtered through hygroscopic cotton wool. The solvent was removed under vacuum. The yield of compound 3 was 2.2 g (13.3%), which was further used without additional purification.

Glutaric anhydride (46 g, 0.053 mol) was added to a solution of compound 3 (2.2 g, 0.014 mol) in isopropanol (20 mL) under stirring and cooling. After addition, the reaction mixture was aged for 12 hours. The solvent was removed under vacuum. The residue was purified by chromatography on silica gel in a column with a height of 30 cm and a diameter of 5 cm. The eluent was chloroform-methanol (5:1). The yield was 1.5 g (39.1%). [M+H]⁺=268.17. ¹H NMR (400.13 MHz, DMSO-d6, δ, m.d., J/Hz) : 1.12 (s, 6H, C*H*₃); 1.75 (quin, 2H, CCH₂C*H*₂CH₂C, J=7.0 Hz); 2.17 (t, 2H, CCH₂C*H*₂CH₂C, J=7.0 Hz); 2.39 (t, 2H, CCH₂C*H*₂CH₂C, J=7.1 Hz); 2.90 (t, 2H, CC*H*₂CH₂NC, J=7.0 Hz); 3.37 (s, 1H, OH); 3.60 (s, 1H, CCH₂CH₂NC*H*); 3.69 (t, 2H, CCH₂C*H*₂NC, J=7.0 Hz); 6.90 (s, 1H, NCHC); 7.49 (s, 1H, NCHNH); 8.24 (s, 1H, *NH*)

The following compounds (without limitation to the recited ones), which are given in Table 2, were prepared according to the disclosed methods.

**Table 2**

| Number in the application | Formula | Constants |
|---|---|---|
| 1 | 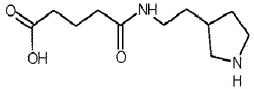 | LC/MS, an individual peak at a retention time of 0.3 min, [M+H]⁺=229 (condition A). HPLC under condition 1, individual peak at a retention time of 9.8 min. |
| 2 | 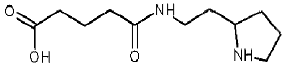 | LC/MS, an individual peak at a retention time of 0.3 min, [M+H]⁺=229 (condition A). HPLC under condition 1, individual peak at a retention time of 12.1 min. |
| 3 | 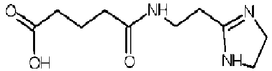 | LC/MS, an individual peak at a retention time of 2.1 min, [M+H]⁺=228 (condition D). HPLC under condition 1, individual peak at a retention time of 11.7 min. |
| 4 | 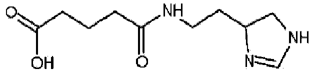 | [M+H]⁺=228.13 |
| | | ¹H NMR (400.13 MHz, DMSO-d6, δ, m.d., J/Hz): 1.62 (quin, 2H, CC*H*₂C*H*₂CH₂C, J=7.0 Hz); 1.65 (dt, 2H, NCHC*H*₂CH₂NH, J=11.2 Hz, J=6.5 Hz); 2.04 (t, 2H, CCH₂C*H*₂CH₂C, J=7.0 Hz); 2.37 (t, 2H, CCH₂C*H*₂CH₂C, J=7.1 Hz); 2.92 (d, 2H, NCHC*H*₂NH, J=7.5 Hz); 3.19 (t, 2H, NCHCH₂C*H*₂NH, J=6.5 Hz); 3.37 (s, 1H, O*H*); 4.01 (m, 1H, NC*H*CH₂CH₂NH); 5.70 (s, 1H, NCHCH₂N*H*); 7.28 (s, 1H, NC*H*); 7.65 (s, 1H, NCHCH₂CH₂N*H*) |
| 5 | 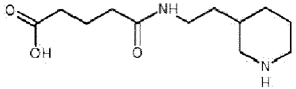 | LC/MS, an individual peak at a retention time of 0.7 min, [M+H]⁺=243 (condition A). HPLC under condition 1, individual peak at a retention time of 10.6 min. |
| 6 | 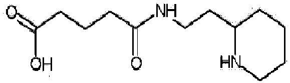 | LC/MS, an individual peak at a retention time of 0.7 min, [M+H]⁺=243 (condition A). HPLC under condition 1, individual peak at a retention time of 10.8 min. |
| 7 | 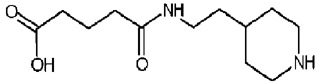 | LC/MS, an individual peak at a retention time of 0.7 min, [M+H]⁺=243 (condition A). HPLC under condition 1, individual peak at a retention time of 5.02 min. |
| 8 | 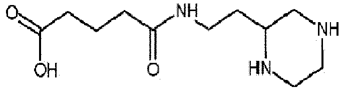 | [M+H]⁺=244.17. |
| | | ¹H NMR (400.13 MHz, DMSO-d6, δ, m.d., J/Hz): 1.52 (dt, 2H, CHC*H*₂CH₂NHC, J=9.2 Hz, J=6.8 Hz); 1.62 (quin, 2H, CCH₂C*H*₂CH₂C, J=7.0 Hz); 2.10 (s, 3H, N*H*CHCH₂CH₂NH) ; 2.12 (t, 2H, CC*H*₂CH₂CH₂C, J=7. 0 Hz); 2.37 (t, 2H, CCH₂CH₂C*H*₂C, J=7.1 Hz); 2.61 (d, 2H, NHC*H*₂CH, J=9.9 Hz); 2.67 (t, 2H, NHCH₂C*H*₂NH, J=7. 5 Hz); 2.86 (t, 2H, NHC*H*₂CH₂NH, J=7.5 Hz); 2.97 (m, 1H, C*H*); 3.04 (t, 2H, CHCH₂C*H*₂NHC, J=6.8 Hz); 3.37 (s, 1H, O*H*); 4.07 (s, 1H, NHCH₂CH₂N*H*) ; 7.65 (s, 1H, CHCH₂CH₂N*H*C) |
| 9 | 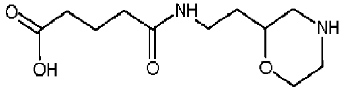 | LC/MS, an individual peak at a retention time of 2.2 min, [M+H]⁺=245 (condition D). HPLC under condition 2, individual peak at a retention time of 8.6 min. |
| 10 | 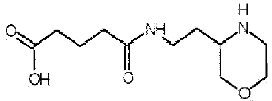 | LC/MS, an individual peak at a retention time of 0.2 min, [M+H]⁺=245 (condition D). HPLC under condition 3, individual peak at a retention time of 5.4 min. |
| | | ¹H NMR (400.13 MHz, DMSO-d6, δ, m.d., J/Hz): 1.30 (m, 2H, C*H*₂CH), 1.70 (p, 2H, COCH₂C*H₂*CH₂CO, J = 7.4 Hz), 2.07 (t, 2H, CH₂CONH, J = 7.4 Hz), 2.18 (t, 2H, CH₂COOH, J = 7.4 Hz), 2.59 (m, 1H, morph), 2.68 (m, 2H, morph), 2.97 (t, 1H, morph, J = 10.2 Hz), 3.07 (q, 2H, CH₂C*H*₂NH, J = 6.9 Hz), 3.29 (m, 1H, morph), 3.62 (m, 2H, morph), 7.79 (t, 1H, NH, J = 5.3 Hz). |
| 12 | 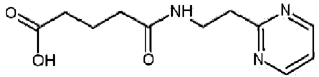 | [M+H]⁺=238.12 |
| | | ¹H NMR (400.13 MHz, DMSO-d6, δ, m.d., J/Hz): 1.64 (quin, 2H, CCH₂C*H*₂CH₂C, J=7.0 Hz); 2.16 (t, 2H, CCH₂C*H*₂CH₂C, J=7.0 Hz); 2.37 (t, 2H, CCH₂C*H*₂CH₂C, J=7.1 Hz); 3.37 (s, 3H, O*H*); 3.38 (t, 2H, CC*H*₂CH₂NHC, J=7.0 Hz); 3.48 (t, 2H, CCH₂C*H*₂NHC, J=7.0 Hz); 7.26 (s, 1H, NCHC*H*CH); 7.94 (s, 1H, N*H*); 8.69 (s, 2H, NC*H*CHCH) |
| 13 | 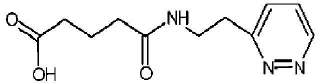 | [M+H]⁺=238.12 |
| | | ¹H NMR (400.13 MHz, DMSO-d6, δ, m.d., J/Hz): 1.64 (quin, 2H, CCH₂C*H*₂CH₂C, J=7.0 Hz); 2.16 (t, 2H, CC*H*₂C*H*₂CH₂C, J=7.0 Hz); 2.37 (t, 2H, CCH₂CH₂C*H*₂C, J=7.1 Hz); 3.05 (t, 2H, CC*H*₂CH₂NHC, J=7.2 Hz); 3.36 (t, 3H, CCH₂C*H*₂NHC, J=7.2 Hz); 3.37 (s, 1H, O*H*); 7.36 (d, 1H, C*H*CCH₂CH₂NH, J=8.0 Hz); 7.63 (dd, 1H, CHC*H*CHC, J=5.1 Hz, J=8.0 Hz); 7.94 (s, 1H, N*H*) ; 8.80 (d, 1H, C*H*CHCHC, J=5.1 Hz) |
| 14 | 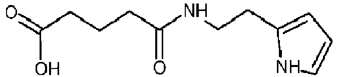 | [M+H]⁺=225.12 |
| | | ¹H NMR (400.13 MHz, DMSO-d6, δ, m.d., J/Hz): 1.64 (quin, 2H, CCH₂C*H*₂CH₂C, J=7.0 Hz); 2.16 (t, 2H, CC*H*₂CH₂CH₂C, J=7.0 Hz); 2.37 (t, 2H, CCH₂CH₂C*H*₂C, J=7.1 Hz); 2.82 (t, 2H, NHCC*H*₂CH₂NH, J=7.2 Hz); 3.15 (t, 2H, NHCCH₂C*H*₂NH, J=7.2 Hz); 3.37 (s, 1H, O*H*); 5.87 (dd, 2H, CHC*H*CH, C*H*CCH₂CH₂NH, J=4.0 Hz, J=4.0 Hz); 6.33 (s, 1H, C*H*CHCH); 7.94 (s, 1H, NHCCH₂CH₂N*H*); 11.18 (s, 1H, N*H*CCH₂CH₂NH) |
| 15 | 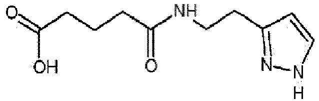 | LC/MS, an individual peak at a retention time of 2.6 min, [M+H]⁺=226 (condition D). HPLC under condition 1, individual peak at a retention time of 11.5 min. |
| 23 | 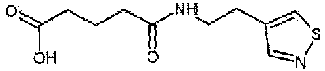 | [M+H]⁺=209.09 |
| | | ¹H NMR (400.13 MHz, DMSO-d6, δ, m.d., J/Hz): 1.64 (quin, 2H, CCH₂C*H*₂CH₂C, J=7.0 Hz); 2.16 (t, 2H, CC*H*₂CH₂CH₂C, J=7.0 Hz); 2.37 (t, 2H, CCH₂CH₂C*H*₂C, J=7.1 Hz); 2.57 (t, 2H, CC*H*₂CH₂NHC, J=7.0 Hz); 3.37 (t, 3H, CCH₂C*H*₂NHC, O*H*, J=7. 0 Hz); 7.07 (s, 1H, SC*H*C); 7.67 (s, 1H, NC*H*C); 7.92 (s, 1H, N*H*) |
| 29 | 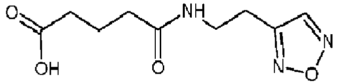 | [M+H]⁺=228.10 |
| | | ¹H NMR (400.13 MHz, DMSO-d6, δ, m.d., J/Hz): 1.64 (quin, 2H, CCH₂C*H*₂CH₂C, J=7.0 Hz); 2.16 (t, 2H, CC*H*₂CH₂CH₂C, J=7.0 Hz); 2.37 (t, 2H, CCH₂CH₂C*H*₂C, J=7.1 Hz); 2.87 (t, 2H, NCC*H*₂CH₂NH, J=7.0 Hz); 3.36 (t, 3H, NCCH₂C*H*₂NH, J=7. 0 Hz); 3.37 (s, 1H, O*H*) ; 7.65 (s, 1H, C*H*); 7.94 (s, 1H, N*H*) |
| 30 | 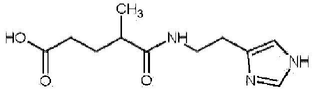 | [M+H]⁺=240.13 |
| | | ¹H NMR (400.13 MHz, DMSO-d6, δ, m.d., J/Hz): 1.03 (d, 3H, C*H*₃, J=6.7 Hz); 1.95 (dt, 2H, CCH₂C*H*₂CHCH₃, J=10.5 Hz, J=7.0 Hz); 2.44 (t, 2H, CC*H*₂CH₂CHCH₃, J=7.0 Hz); 2.73 (t, 2H, NCC*H*₂CH₂NH, J=6.8 Hz); 3.23 (t, 2H, NCCH₂C*H*₂NH, J=6.8 Hz); 3.35 (tq, 1H, CCH₂CH₂C*H*CH₃, J=10.5 Hz, J=6.7 Hz); 7.05 (s, 1H, NHC*H*C); 7.56 (s, 1H, NCHN*H*); 7.67 (s, 1H, NCCH₂CH₂N*H*); 7.70 (s, 1H, NC*H*NH); 10.57 (s, 1H, O*H*) |
| 32 | 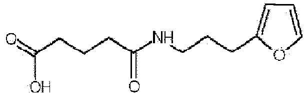 | LC/MS, an individual peak at a retention time of 1.8 min, [M+H]⁺=240 (condition B). HPLC under condition 2, individual peak at a retention time of 12.0 min. ¹H NMR (400.13 MHz, DMSO-d6, δ, m.d., J/Hz): 1.69 (m, 4H, CH₂CH₂CH₂,); 2.07 (t, 2H, CH₂CONH, J=7.5 Hz); 2.19 (t, 2H, CH₂COOH, J=7.5 Hz); 2.59 (t, 2H, CH₂C, J=7.6 Hz); 3.06 (q, 2H, CH₂NH, J=7.6 Hz); 6.09 (m, 1H, furan); 6.33 (m, 1H, furan); 7.50 (m, 1H, furan); 7.84 (br t, 1H, NH) |
| 34 | 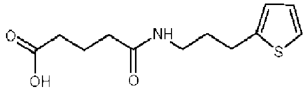 | LC/MS, an individual peak at a retention time of 2.0 min, [M+H]⁺=256 (condition B). HPLC under condition 3, individual peak at a retention time of 10.2 min. ¹H NMR (400.13 MHz, DMSO-d6, δ, m.d., J/Hz): 1.63 (quin, 2H, CH₂CH₂CH₂COOH, J=7.3 Hz); 1.72 (quin, 2H, CH₂CH₂CH₂NH, J=7.5 Hz); 1.85 (t, 2H, CH₂COOH, J=7.3 Hz); 2.03 (t, 2H, CH₂CONH, J=7.3 Hz); 2.79 (t, 2H, CH₂C, J=7.5 Hz); 3.06 (q, 2H, CH₂NH, J=7.5 Hz); 6.86 (m, 1H, thiophen); 6.93 (m, 1H, thiophen); 7.28 (d, 1H, thiophen); 8.11 (br t, 1H, NH) |
| 35 | 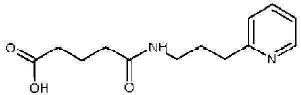 | LC/MS, an individual peak at a retention time of 0.4 min, [M+H]⁺=251 (condition B). HPLC under condition 2, individual peak at a retention time of 16.0 min. ¹H NMR (400.13 MHz, DMSO-d6, δ, m.d., J/Hz): 1.70 (quin, 2H, CH₂CH₂CH₂COOH, J=7.3 Hz); 1.78 (quin, 2H, CH₂CH₂CH₂NH, J=7.5 Hz); 2.09 (t, 2H, CH₂CONH, J=7.3 Hz); 2.20 (t, 2H, CH₂COOH, J=7.3 Hz); 2.71 (t, 2H, CH₂C, J=7.5 Hz); 3.03 (q, 2H, CH₂NH, J=7.5 Hz); 7.19 (m, 1H, 5-Pyr); 7.25 (d, 1H, 3-Pyr, J=7.8 Hz); 7.68 (m, 1H, 4-Pyr); 7.82 (br t, 1H, NH); 8.46 (d, 1H, 6-Pyr, J=4.1 Hz) |
| 36 | 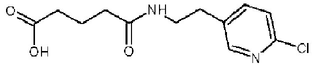 | LC/MS, an individual peak at a retention time of 1.5 min, [M+H]⁺=271 (condition B). HPLC under condition 2, individual peak at a retention time of 8.9 min. ¹H NMR (400.13 MHz, DMSO-d6, δ, m.d., J/Hz): 1.66 (quin, 2H, CH₂CH₂CH₂, J=7.5 Hz); 2.04 (t, 2H, CH₂CONH, J=7.5 Hz); 2.15 (t, 2H, CH₂COOH, J=7.5 Hz); 2.71 (t, 2H, CH₂C, J=7.0 Hz); 3.28 (q, 2H, CH₂NH, J=7.0 Hz); 7.42 (d, 1H, 5-Pyr, J=7.4 Hz); 7.68 (dd, 1H, 4-Pyr, J=7.4, 2.2 Hz); 7.86 (br t, 1H, NH); 8.23 (d, 1H, 2-Pyr, J=2.2 Hz); 12.02 (br s, 1H, -COOH) |
| 44 | 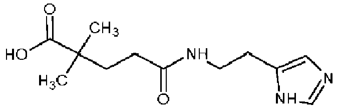 | LC/MS, an individual peak at a retention time of 1.2 min, [M+H]⁺=254 (condition E). HPLC under condition 3, individual peak at a retention time of 11.7 min. |
| | | ¹H NMR (400.13 MHz, DMSO-d6, δ, m.d., J/Hz): 1.07 (s. 6H. C*H*₃); 1.68 (m, 2H, C*H*₂CH₂CO) , 2.01 (m, 2H, C*H*₂CO), 2.60 (t, 2H, CH₂C*H*₂C, J= 7.3 Hz); 3.24 (q, 2H, CH₂C*H*₂NH, J= 7.3 Hz); 6.77 (s, 1H, CC*H)*; 7.53 (s, 1H, NC*H*N*)*; 7.85 (br s, 1H, N*H)* |
| 45 | 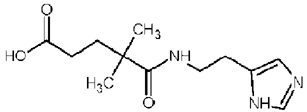 | LC/MS, an individual peak at a retention time of 0.55 min, [M+H]⁺=254 (condition E). HPLC under condition 3, individual peak at a retention time of 11.1 min. |
| | | ¹H NMR (400.13 MHz, DMSO-d6, δ, m.d., J/Hz): 1.05 (s. 6H. C*H*₃); 1.67 (m, 2H, C*H*₂CH₂COOH), 2.06 (m, 2H, C*H*₂COOH), 2.62 (t, 2H, CH₂C*H*₂C, J= 7.4 Hz); 3.26 (q, 2H, CH₂C*H*₂NH, J= 7.4 Hz); 6.77 (s, 1H, CC*H*); 7.51 (s, 1H, NC*H*N); 7.60 (br s, 1H, N*H*) |
| 52 | 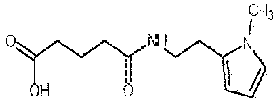 | LC/MS, an individual peak at a retention time of 0.9 min, [M+H]⁺=259 (condition A). HPLC under condition 1, individual peak at a retention time of 13.3 min. |
| 63 | 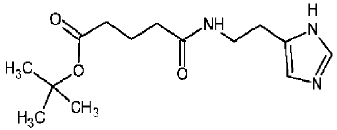 | LC/MS, an individual peak at a retention time of 0.93 min, [M+H]⁺=282 (condition G). HPLC under condition 6, individual peak at a retention time of 13.4 min. |
| | | ¹H NMR (400.13 MHz, DMSO-d6, δ, m.d., J/Hz): 1.38 (s, 9H, CH₃C), 1.68 (quin, 2H, CH₂CH₂CH₂, J=7.5 Hz); 2.06 (t, 2H, CH₂CONH, J=7.4 Hz); 2.15 (t, 2H, CH₂COO, J=7.4 Hz); 2.60 (t, 2H, CH₂C, J=7.4 Hz); 3.24 (m, 2H, CH₂N); 6.73 (br s, 1H, CCH); 7.46 (d, 1H, NCHN, J=1 Hz); 7.70 (br s, 1H, NH); 11.67 (br s, 1H, NH) |
| 64 | 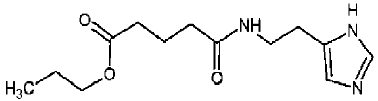 | LC/MS, an individual peak at a retention time of 0.4 min, [M+H]⁺=268 (condition G). HPLC under condition 6, individual peak at a retention time of 11.8 min. |
| | | ¹H NMR (400.13 MHz, DMSO-d6, δ, m.d., J/Hz): 0.87 (t, 3H, CH₃CH₂, J=7.4 Hz), 1.56 (t, 2H, CH₂CH₂CH₂, J=7.1 Hz), 1.73 (quin, 2H, CH₂CH₂CH₂), 2.07 (n, 2H, CH₂CO, J=7.5 Hz); 2.06 (t, 2H, CH₂COO, J=7.5 Hz); 2.60 (t, 2H, CH₂C, J=7.4 Hz); 3.25 (m, 2H, CH₂N); 3.95 (t, 2H, CH₂O, J=6.7 Hz); 6.73 (br s, 1H, CCH); 7.46 (d, 1H, NCHN, J=1 Hz); 7.72 (br s, 1H, NH); 11.7 (br s, 1H, NH) |
| 67 | 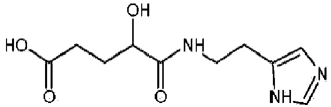 | LC/MS, an individual peak at a retention time of 0.5 min, [M+H]⁺=242 (condition D). HPLC under condition 3, individual peak at a retention time of 7.4 min. |
| | | ¹H NMR (400.13 MHz, DMSO-d6, δ, m.d., J/Hz): 1.65, 1.87 (m, 1H+1H, CH₂C*H*₂CH), 2.23 (m, 2H, C*H*₂CH₂CH), 2.64 (t, 2H, CH₂C*H*₂C, J= 7.2 Hz); 3.31 (q, 2H, CH₂C*H*₂NH, J= 6.7 Hz); 3.85 (dd, 1H, CH₂CH₂C*H*, J = 4.2, 7.7 Hz), 6.79(s, 1H, CC*H)*; 7.52 (s, 1H, NC*H*N), 7.82 (t, 1H, NH, J = 5.7 Hz) |
| 68 | 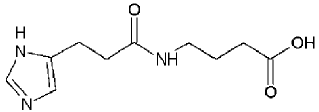 | LC/MS, an individual peak at a retention time of 0.28 min, [M+H]⁺=226 (condition E). HPLC under condition 3, individual peak at a retention time of 9.6 min. |
| | | ¹H NMR (400.13 MHz, DMSO-d6, δ, m.d., J/Hz): 1.59 (quin 2H. CH₂C*H₂*CH₂, J= 7.2 Hz); 2.18 (t, 2H, C*H*₂COOH, J= 7.2 Hz); 2.34 (t, 2H, C*H*₂CONH, J= 7.8 Hz); 2.70 (t, 2H, C*H*₂C, J= 7.8 Hz); 3.03 (q, 2H, C*H*₂NH, J= 6.7 Hz); 6.70 (s, 1H, CC*H)*; 7.48 (s, 1H, NC*H*N*)*; 7.86 (br t, 1H, N*H*) |
| 71 | 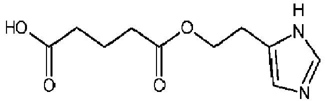 | [M+H]⁺=227.10 |
| | | ¹H NMR (400.13 MHz, DMSO-d6, δ, m.d., J/Hz): 2.39 (t, 2H, CCH₂CH₂C*H*₂C, J=7.5 Hz); 2.49 (t, 2H, CC*H*₂CH₂CH₂C, J=7.5 Hz); 2.56 (quin, 2H, CCH₂C*H*₂CH₂C, J=7.5 Hz); 3.14 (t, 2H, CC*H*₂CH₂OC, J=7.3 Hz); 4.29 (t, 2H, CCH₂C*H*₂OC, J=7.3 Hz); 7.03 (s, 1H, NC*H*C); 7.71 (s, 1H, NC*H*NH); 8.24 (s, 1H, N*H*); 11.00 (s, 1H, O*H*) |
| 72 | 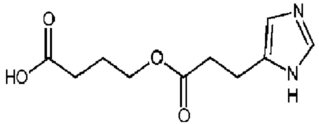 | LC/MS, an individual peak at a retention time of 1.9 min, [M+H]⁺=227 (condition D). HPLC under condition 1, individual peak at a retention time of 13.6 min. |
| | | ¹H NMR (400.13 MHz, DMSO-d6, δ, m.d., J/Hz): 1.78 (quin 2H. CH₂C*H₂*CH₂, J= 6.9 Hz); 2.25 (t, 2H, C*H*₂COOH, J= 7.1 Hz); 2.58 (t, 2H, C*H*₂CO, J= 7.5 Hz); 2.74 (t, 2H, C*H*₂C, J= 7.5 Hz); 4.01 (t, 2H, C*H*₂O, J=6.5 Hz); 6.74 (s, 1H, CC*H)*; 7.50 (s, 1H, NC*H*N) |
| 75 | 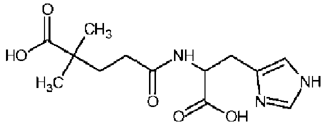 | [M+H]⁺=298.14 |
| | | ¹H NMR (400.13 MHz, DMSO-d6, δ, m.d., J/Hz): 1.18 (s, 6H, C*H*₃); 1.76 (t, 2H, CCH₂C*H*₂CCH₃, J=8.3 Hz) ; 2.24 (t, 2H, CC*H*₂CH₂CCH₃, J=8.3 Hz); 3.12 (d, 2H, CC*H*₂CHCOH, J=7.5 Hz); 4.44 (t, 1H, CCH₂C*H*COH, J=7. 5 Hz); 7.20 (s, 1H, NHC*H*C); 7.56 (s, 1H, NCHN*H*); 8.10 (s, 1H, NC*H*NH); 8.20 (s, 1H, N*H*CCH₂CH₂C); 12.17 (s, 1H, CH₃CCO*H*); 12.37 (s, 1H, CCH₂CHCO*H*) |
| 85 | 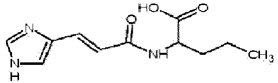 | LC/MS, an individual peak at a retention time of 0.3 min, [M+H]⁺=237 (condition A). HPLC under condition 1, individual peak at a retention time of 12.0 min. |
| 89 | 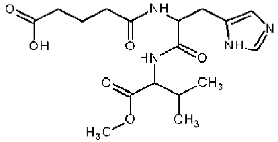 | LC/MS, an individual peak at a retention time of 0.8 min, [M+H]⁺=383 (condition A). HPLC under condition 1, individual peak at a retention time of 12.2 min. ¹H NMR (400.13 MHz, DMSO-d6, δ, m.d., J/Hz): 0.85 (m, 6H, CH(CH₃)₂); 1.66 (quin, 2H, CH₂CH₂CH₂, J=7.4 Hz); 2.03 (m, 1H, CHCH₃) ; 2.13 (m, 4H, CH₂CH₂CH₂) ; 2.73, 2.89 (m, 2H, CCH₂CH); 3.62 (s, 3H, OCH₃) ; 4.15 (m, 1H, NCH); 4.56 (m, 1H, CCH₂CH); 6.76 (s, 1H, CCH); 7.53 (s, 1H, NCHN); 8.04 (d, 1H, NH, J=8.0 Hz); 8.10 (d, 1H, NH, J=8.2 Hz); 11.9 (br s, 1H, -COOH) |
| 90 | 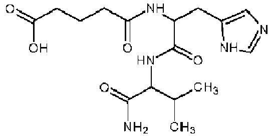 | LC/MS, an individual peak at a retention time of 0.3 min, [M+H]⁺=368 (condition A). HPLC under condition 1, individual peak at a retention time of 10.9 min. ¹H NMR (400.13 MHz, DMSO-d6, δ, m.d., J/Hz): 0.81 (m, 6H, CH(CH₃) ₂); 1.67 (quin, 2H, CH₂CH₂CH₂, J=7.5 Hz); 1.99 (m, 1H, CHCH₃); 2.14 (m, 4H, CH₂CH₂CH₂) ; 2.78, 2.92 (m, 2H, CCH₂CH); 4.09 (m, 1H, NCH); 4.54 (m, 1H, CCH₂CH); 6.85 (s, 1H, CCH); 7.06, 7.53 (br s, 2H, NH₂); 7.59 (d, 1H, NH, J=8.7 Hz); 7.72 (s, 1H, NCHN); 8.10 (d, 1H, NH, J=7.9 Hz); 12.19 (br s, 1H, -COOH) |
| 91 | 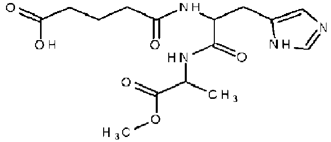 | LC/MS, an individual peak at a retention time of 0.3 min, [M+H]⁺=355 (condition A). HPLC under condition 1, individual peak at a retention time of 11.12 min. ¹H NMR (400.13 MHz, DMSO-d6, δ, m.d., J/Hz): 1.26 (d, 3H, CHCH₃, J=7.3 Hz); 1.65 (quin, 2H, CH₂CH₂CH₂, J=7.4 Hz); 2.11 (m, 4H, CH₂CH₂CH₂) ; 2.72, 2.91 (m, 2H, CCH₂CH); 3.60 (s, 3H, OCH₃); 4.26 (p, 1H, NCHCH₃, J=7.3 Hz); 4.50 (m, 1H, CCH₂CH); 6.78 (s, 1H, CCH); 7.57 (s, 1H, NCHN); 7.98 (d, 1H, NH, J=8.2 Hz); 8.35 (d, 1H, NH, J=7.2 Hz) |
| 92 | 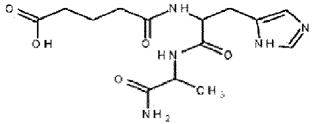 | LC/MS, an individual peak at a retention time of 0.3 min, [M+H]⁺=340 (condition A). HPLC under condition 1, individual peak at a retention time of 9.7 min. ¹H NMR (400.13 MHz, DMSO-d6, δ, m.d., J/Hz): 1.20 (d, 3H, CHCH₃, J=7.2 Hz); 1.67 (quin, 2H, CH₂CH₂CH₂, J=7.4 Hz); 2.15 (m, 4H, CH₂CH₂CH₂); 2.78, 2.91 (m, 2H, CCH₂CH); 4.26 (p, 1H, NCHCH₃, J=7.2 Hz); 4.43 (m, 1H, CCH₂CH); 6.78 (s, 1H, CCH); 7.00, 7.64 (br s, 2H, NH₂); 7.50 (s, 1H, NCHN); 7.97 (m, 2H, NH); 11.9 (br s, 1H, -COOH) |
| 94 | 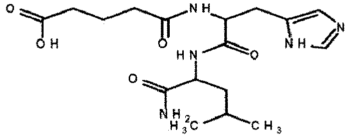 | LC/MS, an individual peak at a retention time of 0.3 min, [M+H]⁺=382 (condition A). HPLC under condition 1, individual peak at a retention time of 11.8 min. ¹H NMR (400.13 MHz, DMSO-d6, δ, m.d., J/Hz): 0.82, 0.87 (d, 6H, CH₂CH(CH₃)₂, J=6.1 Hz); 1.51 (m, 3H, CH₂CH(CH₃) ₂); 1.67 (quin, 2H, CH₂CH₂CH₂, J=7.5 Hz); 2.15 (m, 4H, CH₂CH₂CH₂) ; 2.86, 2.98 (m, 2H, CCH₂CH); 4.19 (m, 1H, NCH,); 4.52 (m, 1H, CCH₂CH); 7.02, 7.51 (br s, 2H, NH₂); 7.07 (s, 1H, CCH); 7.90 (d, 1H, NH, J=8.2 Hz); 8.08 (d, 1H, NH, J=7.9 Hz); 8.23 (s, 1H, NCHN) |
| 96 | 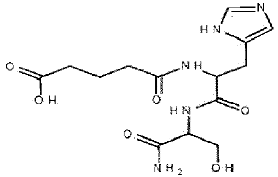 | LC/MS, an individual peak at a retention time of 0.3 min, [M+H]⁺=356 (condition A). HPLC under condition 1, individual peak at a retention time of 8.8 min. ¹H NMR (400.13 MHz, DMSO-d6, δ, m.d., J/Hz) : 1.67 (quin, 2H, CH₂CH₂CH₂, J=7.5 Hz); 2.15 (m, 4H, CH₂CH₂CH₂); 2.87 (m, 2H, CCH₂CH); 3.60 (m, 2H, CHCH₂OH); 4.14 (m, 1H, NCH,); 4.46 (m, 1H, CCH₂CH); 6.82 (s, 1H, CCH); 7.09, 7.61 (br s, 2H, NH₂); 7.52 (s, 1H, NCHN); 7.83 (d, 1H, NH, J=7.7 Hz); 7.97 (d, 1H, NH, J=7.6 Hz); 11.9 (br s, 1H,-COOH) |
| 100 | 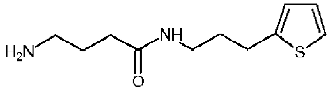 | LC/MS, an individual peak at a retention time of 1.1 min, [M+H]⁺=227 (condition B). HPLC under condition 3, individual peak at a retention time of 7.4 min. ¹H NMR (400.13 MHz, DMSO-d6, δ, m.d., J/Hz): 1.74 (m, 4H, CH₂CH₂CH₂); 2.19 (t, 2H, CH₂CONH, J=7.3 Hz); 2.79 (m, 4H, CH₂NH₂, CH₂C) ; 3.08 (q, 2H, CH₂NH, J=7.5 Hz); 6.85 (m, 1H, thiophen); 6.93 (m, 1H, thiophen); 7.30 (d, 1H, thiophen); 7.94 (br s, 2H, NH₂); 8.04 (br t, 1H, NH) |
| 103 | 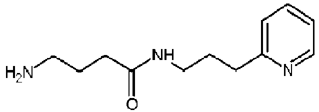 | LC/MS, an individual peak at a retention time of 0.2 min, [M+H]⁺=222 (condition B). HPLC under condition 2, individual peak at a retention time of 4.0 min. ¹H NMR (400.13 MHz, DMSO-d6, δ, m.d., J/Hz): 1.78 (quin, 2H, CH₂CH₂CH₂NH₂, J=7.5 Hz); 1.87 (quin, 2H, CH₂CH₂CH₂NH, J=7.3 Hz); 2.20 (t, 2H, CH₂CONH, J=7.3 Hz); 2.77 (m, 2H, CH₂NH₂); 3.01 (t, 2H, CH₂-Pyr, J=7.5 Hz); 3.09 (q, 2H, CH₂NH, J=7.5 Hz); 7.80 (m, 1H, 5-Pyr); 7.89 (d, 1H, 3-Pyr, J=7.8 Hz); 8.15 (m, 1H, 4-Pyr); 8.39 (br t, 1H, NH); 8.74 (d, 1H, 6-Pyr, J=4.1 Hz) |
| 104 | 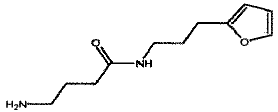 | LC/MS, an individual peak at a retention time of 0.8 min, [M+H]⁺=222 (condition A). HPLC under condition 1, individual peak at a retention time of 12.5 min. ¹H NMR (400.13 MHz, DMSO-d6, δ, m.d., J/Hz): 1.77 (quin, J=7.2 Hz, 2H, CH₂), 2.18 (t, J=7.2 Hz, 2H, CH₂), 2.46-2.54 (m, 2H, CH₂), 2.66-2.78 (m, 4H, -CH₂CH₂-), 3.28 (dd, J₁=13.1 Hz, J₂=6.1 Hz, CH₂), 6.15 (d, 1H, H-furan), 6.34 (t, 1H, H-furan), 7.51 (s, 1H, H-furan), 8.06-8.22 (m, 4H, NH₃⁺+NH ) |
| 115 | 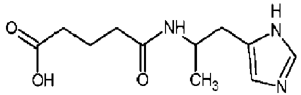 | [M+H]⁺=240.13 |
| | | ¹H NMR (400.13 MHz, DMSO-d6, δ, m.d., J/Hz): 1.11 (d, 3H, C*H*₃, J=6.5 Hz); 1.64 (quin, 2H, CCH₂C*H*₂CH₂C, J=7.0 Hz); 2.15 (t, 2H, CC*H*₂CH₂CH₂C, J=7.0 Hz); 2.37 (t, 2H, CCH₂CH₂C*H*₂C, J=7.1 Hz); 2.95 (d, 2H, NHCC*H*₂CHCH₃, J=10.2 Hz); 3.37 (s, 1H, O*H*); 3.92 (qt, 1H, NHCCH₂C*H*CH₃, J=6.5 Hz, J=10.2 Hz); 7.14 (s, 1H, NC*H*C); 7.65 (s, 1H, NC*H*NH); 7.82 (s, 1H, NHCCH₂CHN*H*); 8.24 (s, 1H, N*H*CCH₂CHCH₃) |
| 117 | 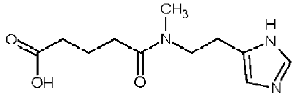 | [M+H]⁺=240.13 |
| | | ¹H NMR (400.13 MHz, DMSO-d6, δ, m.d., J/Hz): 1.75 (quin, 2H, CCH₂C*H*₂CH₂C, J=7.2 Hz); 2.14 (t, 2H, CC*H*₂CH₂CH₂C, J=7.2 Hz); 2.39 (t, 2H, CCH₂C*H*₂CH₂C, J=7.1 Hz); 2.75 (s, 3H, C*H*₃); 2.80 (t, 2H, CC*H*₂CH₂N, J=7.1 Hz); 3.37 (s, 1H, O*H*); 3.38 (t, 2H, CCH₂C*H*₂N, J=7.1 Hz); 6.94 (s, 1H, CC*H*N); 7.52 (s, 1H, NC*H*NH); 8.24 (s, 1H, N*H*) |
| 118 | 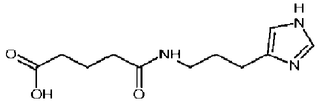 | LC/MS, an individual peak at a retention time of 0.45 min, [M+H]⁺=240 (condition E). HPLC under condition 3, individual peak at a retention time of 10.0 min. |
| | | ¹H NMR (400.13 MHz, DMSO-d6, δ, m.d., J/Hz): 1.72 (quin 2H. COCH₂C*H₂*CH₂CO, J= 7.4 Hz); 1.80 (quin 2H. CH₂C*H₂*CH₂N, J= 6.4 Hz); 2.13 (m, 4H, C*H*₂CONH, C*H*₂COOH); 2.67 (t, 2H, C*H*₂CH, J=7.5 Hz); 3.14 (t, 2H, C*H₂*NH, J=6.7 Hz), 7.14(s, 1H, C*H*N); 8.47 (s, 1H, NC*H*N) |
| 120 | 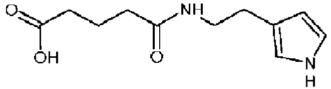 | [M+H]⁺=225.12 |
| | | ¹H NMR (400.13 MHz, DMSO-d6, δ, m.d., J/Hz): 1.64 (quin, 2H, CCH₂C*H*₂CH₂C, J=7.0 Hz); 2.16 (t, 2H, CC*H*₂CH₂CH₂C, J=7.0 Hz); 2.37 (t, 2H, CCH₂CH₂C*H*₂C, J=7.1 Hz); 2.44 (t, 2H, CC*H*₂CH₂NHC, J=6.9 Hz); 3.23 (t, 2H, CCH₂C*H*₂NHC, J=6.9 Hz); 3.37 (s, 1H, O*H*); 5.83 (s, 1H, C*H*CCH₂CH₂NH); 6.68 (s, 2H, NHC*H*CH); 6.70 (s, 1H, NHC*H*C); 7.92 (s, 1H, CCH₂CH₂N*H*C); 12.05 (s, 1H, N*H*CHC) |
| 121 | 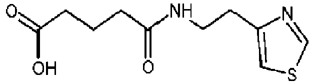 | LC/MS, an individual peak at a retention time of 0.4 min, [M+H]⁺=243 (condition B). HPLC under condition 2, individual peak at a retention time of 27.7 min. ¹H NMR (400.13 MHz, DMSO-d6, δ, m.d., J/Hz): 1.69 (quin, 2H, CH₂CH₂CH₂, J=7.6 Hz); 2.07 (t, 2H, CH₂CONH, J=7.6 Hz); 2.17 (t, 2H, CH₂COOH, J=7.5 Hz); 2.87 (t, 2H, CH₂C, J=7.2 Hz); 3.34 (q, 2H, CH₂NH, J=7.2 Hz); 7.36 (d, 1H, NCHS, J=2.0 Hz) ; 7.88 (br t, 1H, NH); 9.01 (d, 1H, CCHS, J=2.0 Hz) |
| 122 | 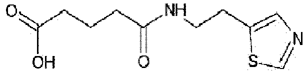 | [M+H]⁺=209.09 |
| | | ¹H NMR (400.13 MHz, DMSO-d6, δ, m.d., J/Hz): 1.64 (quin, 2H, CCH₂C*H*₂CH₂C, J=7.0 Hz); 2.16 (t, 2H, CC*H*₂CH₂CH₂C, J=7.0 Hz); 2.37 (t, 2H, CCH₂CH₂C*H*₂C, J=7.1 Hz); 2.80 (t, 2H, SCC*H*₂CH₂NH, J=7.0 Hz); 3.33 (t, 2H, SCCH₂C*H*₂NH, J=7.0 Hz); 3.37 (s, 1H, O*H*); 7.67 (s, 1H, NC*H*C); 7.94 (s, 1H, NH); 8.70 (s, 1H, SC*H*N) |
| 123 | 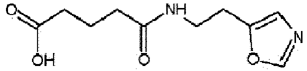 | [M+H]⁺=227.10 |
| | | ¹H NMR (400.13 MHz, DMSO-d6, δ, m.d., J/Hz): 1.64 (quin, 2H, CCH₂C*H*₂CH₂C, J=7.0 Hz); 2.19 (t, 2H, CC*H*₂CH₂CH₂C, J=7.0 Hz); 2.37 (t, 2H, CCH₂CH₂C*H*₂C, J=7.1 Hz); 3.16 (t, 2H, CC*H*₂CH₂NHC, J=7.0 Hz); 3.31 (t, 2H, CCH₂C*H*₂NHC, J=7.0 Hz); 3.37 (s, 1H, O*H*); 7.94 (s, 1H, N*H*); 8.24 (s, 1H, NC*H*C); 8.59 (s, 1H, C*H*OC) |
| 124 | 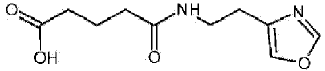 | LC/MS, an individual peak at a retention time of 1.2 min, [M+H]⁺=227 (condition E). HPLC under condition 3, individual peak at a retention time of 8.8 min. |
| | | ¹H NMR (400.13 MHz, DMSO-d6, δ, m.d., J/Hz): 1.70 (quin 2H. CH₂C*H₂*CH₂, J= 7.4 Hz); 2.07 (t, 2H, C*H*₂CONH, J= 7.4 Hz); 2.18 (t, 2H, C*H*₂COOH, J= 7.4 Hz); 2.59 (t, 2H, C*H₂*C, J=7.2 Hz), 3.26 (m, C*H₂*NH, 2H), 7.84 (s, 1H, CC*H*O), 7.87 (t, 1H, NH, J=5.8 Hz), 8.26 (s, 1H, NC*H*O), 12.00 (s, 1H, -COOH) |
| 126 | 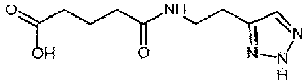 | LC/MS, an individual peak at a retention time of 0.45 min, [M+H]⁺=227 (condition D). HPLC under condition 1, individual peak at a retention time of 5.6 min. |
| | | ¹H NMR (400.13 MHz, DMSO-d6, δ, m.d., J/Hz): 1.70 (quin 2H. CH₂C*H₂*CH₂, J= 7.3 Hz); 2.07 (t, 2H, C*H*₂CONH, J= 7.3 Hz); 2.18 (t, 2H, C*H*₂COOH, J= 7.3 Hz); 2.77 (t, 2H, C*H₂*C, J=7.0 Hz), 3.29 (m, C*H₂*NH, 2H), 7.61 (s, 1H, CHN), 7.90 (s, 1H, NH) |
| 131 | 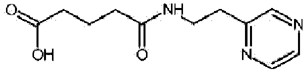 | LC/MS, an individual peak at a retention time of 0.4 min, [M+H]⁺=238 (condition B). HPLC under condition 2, individual peak at a retention time of 23.2 min. ¹H NMR (400.13 MHz, DMSO-d6, δ, m.d., J/Hz): 1.66 (quin, 2H, CH₂CH₂CH₂, J=7.5 Hz); 2.05 (t, 2H, CH₂CONH, J=7.5 Hz); 2.15 (t, 2H, CH₂COOH, J=7.5 Hz); 2.89 (t, 2H, CH₂C, J=7.5 Hz); 3.41 (q, 2H, CH₂NH, J=7.5 Hz); 7.88 (br t, 1H, NH); 8.47 (s, 1H, CH); 8.52 (s, 1H, CH); 8.56 (s, 1H, CH) |
| 139 | 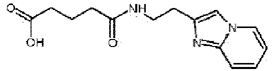 | LC/MS, an individual peak at a retention time of 0.4 min, [M+H]⁺=276 (condition B). HPLC under condition 2, individual peak at a retention time of 18.0 min. ¹H NMR (400.13 MHz, DMSO-d6, δ, m.d., J/Hz) : 1.70 (quin, 2H, CH₂CH₂CH₂, J=7.5 Hz); 2.08 (t, 2H, CH₂CONH, J=7.5 Hz); 2.19 (t, 2H, CH₂COOH, J=7.5 Hz); 2.80 (t, 2H, CH₂C, J=7.3 Hz); 3.38 (q, 2H, CH₂NH, J=7.3 Hz); 6.81 (t, 1H, 6-ImPyr, J=6.4 Hz); 7.16 (t, 1H, 7-ImPyr, J=8.0 Hz); 7.44 (d, 1H, 8-ImPyr, J=8.0 Hz); 7.70 (s, 1H, 3-ImPyr); 7.89 (br t, 1H, NH); 8.44 (d, 1H, 5-ImPyr, J=6.4 Hz) |
| 141 | 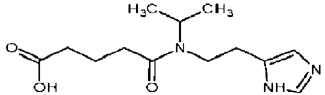 | [M+H]⁺=268.17 |
| | | ¹H NMR (400.13 MHz, DMSO-d6, δ, m.d., J/Hz): 1.12 (s, 6H, C*H*₃) ; 1.75 (quin, 2H, CCH₂C*H*₂CH₂C, J=7.0 Hz); 2.17 (t, 2H, CC*H*₂CH₂CH₂C, J=7.0 Hz); 2.39 (t, 2H, CCH₂CH₂C*H*₂C, J=7.1 Hz); 2.90 (t, 2H, CC*H*₂CH₂NC, J=7.0 Hz); 3.37 (s, 1H, O*H*); 3.60 (s, 1H, CCH₂CH₂NC*H*) ; 3.69 (t, 2H, CCH₂C*H*₂NC, J=7.0 Hz); 6.90 (s, 1H, NC*H*C); 7.49 (s, 1H, NCHNH); 8.24 (s, 1H, N*H*) |
| 145 | 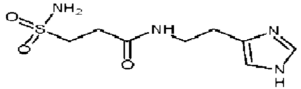 | LC/MS, an individual peak at a retention time of 0.5 min, [M+H]⁺=247 (condition D). HPLC under condition 1, individual peak at a retention time of 7.9 min. |
| | | ¹H NMR (400.13 MHz, DMSO-d6, δ, m.d., J/Hz): 2.53 (t, 2H, C*H₂*C, J=7.8 Hz), 2.79 (t, 2H, C*H₂*C=O, J=6.7 Hz), 3.16 (t, 2H, C*H₂*S, J=6.7 Hz), 3.34 (q, 2H, C*H₂*NH, J=6.4 Hz), 6.84(s, 2H, NH2); 7.43 (s, 1H, CC*H*), 8.26 (t, 1H, NH, J=5.6 Hz), 9.00 (s, 1H, NC*H*); 14.51 (br s, 1H, N*H*) |
| 148 | 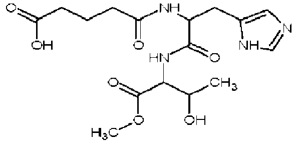 | LC/MS, an individual peak at a retention time of 0.3 min, [M+H]⁺=385 (condition A). HPLC under condition 1, individual peak at a retention time of 10.6 min. ¹H NMR (400.13 MHz, DMSO-d6, δ, m.d., J/Hz): 1.03 (d, 3H, CCH₃, J=6.5 Hz); 1.66 (quin, 2H, CH₂CH₂CH₂, J=7.4 Hz); 2.13 (m, 4H, CH₂CH₂CH₂); 2.75, 2.93 (m, 2H, CCH₂CH); 3.61 (s, 3H, OCH₃); 4.10 (m, 1H, OCHCH₃); 4.26 (m, 1H, NCH); 4.58 (m, 1H, CCH₂CH) ; 6.76 (s, 1H, CCH); 7.51 (s, 1H, NCHN); 7.80 (d, 1H, NH, J=8.5 Hz); 8.10 (d, 1H, NH, J=8.0 Hz) |
| 149 | 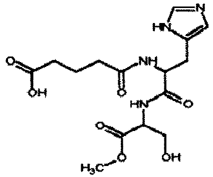 | LC/MS, an individual peak at a retention time of 0.3 min, [M+H]⁺=371 (condition B). HPLC under condition 1, individual peak at a retention time of 10.4 min. ¹H NMR (400.13 MHz, DMSO-d6, δ, m.d., J/Hz): 1.67 (m, 2H, CH₂CH₂CH₂,); 2.10 (m, 4H, CH₂CH₂CH₂); 2.74, 2.93 (m, 2H, CCH₂CH); 3.60, 3.72 (m, 2H, OCH₂CH); 3.61 (s, 3H, OCH₃); 4.28 (m, 1H, OCH₂CH); 4.54 (m, 1H, CCH₂CH); 6.74 (br s, 1H, CCH); 7.50 (s, 1H, NCHN); 8.03 (d, 1H, NH, J=7.8 Hz); 8.33 (d, 1H, NH, J=7.5 Hz) |
| 178 | 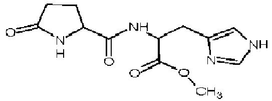 | LC/MS, an individual peak at a retention time of 0.2 min, [M+H]⁺=281 (condition C). HPLC under condition 1, individual peak at a retention time of 7.0 min. ¹H NMR (400.13 MHz, DMSO-d6, δ, m.d., J/Hz): 1.83-2.23 (m, 4H, CH₂CH₂CH), 2.93 (m, 2H, CH₂CH), 3.61 (s, 3H, OCH₃) ; 4.02 (m, 1H, CH₂CH₂CH) ; 4.50 (m, 1H, CH₂CH); 6.84 (s, 1H, CCH); 7.60 (s, 1H, NCHN); 7.78 (s, 1H, NH), 8.05 (d, 1H, NH, J=7.8 Hz) |
| 184 | 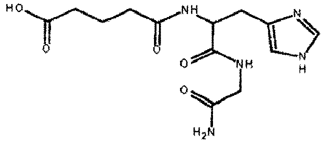 | LC/MS, an individual peak at a retention time of 0.3 min, [M+H]⁺=326 (condition A). HPLC under condition 1, individual peak at a retention time of 8.9 min. ¹H NMR (400.13 MHz, DMSO-d6, δ, m.d., J/Hz) : 1.67 (quin, 2H, CH₂CH₂CH₂, J=7.4 Hz); 2.15 (m, 4H, CH₂CH₂CH₂); 2.79, 2.92 (m, 2H, CCH₂CH); 3.60 (m, 2H, CCH₂NH); 4.38 (m, 1H, CCH₂CH); 6.80 (s, 1H, CCH); 7.08, 7.49 (br s, 2H, NH₂); 7.51 (s, 1H, NCHN); 7.99 (d, 1H, NH, J=7.4 Hz); 8.17 (t, 1H, NH, J=5.9 Hz); 11.9 (br s, 1H, -COOH) |
| 185 | 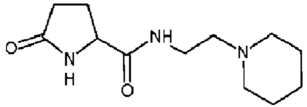 | LC/MS, an individual peak at a retention time of 0.2 min, [M+H]⁺=240 (condition G). HPLC under condition 3, individual peak at a retention time of 8.2 min. |
| | | ¹H NMR (400.13 MHz, DMSO-d6, δ, m.d., J/Hz): 1.43 (m, 2H, CH₂CH₂CH₂CH₂ CH₂) ; 1.59 (m, 4H, CH₂CH₂CH₂CH₂ CH₂); 1.87 (dddd, 1H, CH₂CH₂CH, J=12.0; 9.0; 5.6; 4.5 Hz); 2.11 (m, 2H, CH₂CH₂CO); 2.24 (dddd, 1H, CH₂CH₂CH, J=12.0; 9.8; 8.4; 6.8 Hz); 2.62 (br s, 6H, NCH₂) ; 3.28 (m, 2H, NHCH₂CH₂N) ; 3.96 (ddd, 1H, NHCHCH₂, J=8.6; 4.6; 1.0 Hz); 7.68 (br s, 1H, NH) ; 7.94 (br s, 1H, NH) |
| 188 | 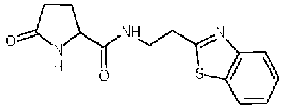 | LC/MS, an individual peak at a retention time of 3.6 min, [M+H]⁺=290 (condition D). HPLC under condition 1, individual peak at a retention time of 10.6 min. ¹H NMR (400.13 MHz, DMSO-d6, δ, m.d., J/Hz): 1.85-2.23 (m, 4H, CH₂CH₂CH), 3.26 (m, 2H, CCH₂CH₂N) ; 3.55 (m, 2H, CCH₂CH₂N) ; 3.95 (m, 1H, CH₂CH₂CH); 7.41 (t, 2H, benzothiazole, J=7.6 Hz), 7.49 (t, 2H, benzothiazole, J=7.6 Hz), 7.77 (s, 1H, NH), 7.95 (d, 2H, benzothiazole, J=8.0 Hz), 8.06 (d, 2H, benzothiazole, J=8.1 Hz); 8.19 (t, 1H, NH, J=5.7 Hz) |
| 193 | 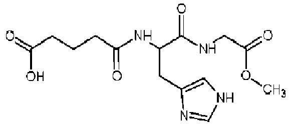 | LC/MS, an individual peak at a retention time of 0.3 min, [M+H]⁺=341 (condition A). HPLC under condition 1, individual peak at a retention time of 10.5 min. ¹H NMR (400.13 MHz, DMSO-d6, δ, m.d., J/Hz): 1.66 (quin, 2H, CH₂CH₂CH₂, J=7.4 Hz); 2.11 (m, 4H, CH₂CH₂CH₂); 2.73, 2.93 (m, 2H, CCH₂CH); 3.60 (s, 3H, OCH₃); 3.82 (m, 2H, CCH₂NH); 4.49 (m, 1H, CCH₂CH); 6.75 (s, 1H, CCH); 7.50 (s, 1H, NCHN); 8.01 (d, 1H, NH, J=8.2 Hz); 8.29 (t, 1H, NH, J=5.9 Hz) |
| 196 | 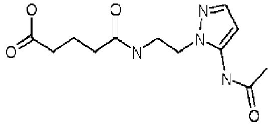 | LC/MS, an individual peak at a retention time of 1.06 min, [M+H]⁺=265 (condition A). HPLC under condition 1, individual peak at a retention time of 10.4 min. ¹H NMR (400.13 MHz, DMSO-d6, δ, m.d., J/Hz): 1.81 (quin, 2H, CH₂CH₂CH₂, J=6.5 Hz); 1.97 (s, 3H, Me), 2.55 (t, 4H, CH₂CH₂CH₂, J=6.5 Hz), 3.96 (t, 2H, CH₂NCO, J=6.3 Hz), 4.05 (t, 2H, CH₂Pyr, J=6.3 Hz), 6.38 (s, 1H, CH), 7.52 (s, 1H, CH=N), 10.35 (s, 1H, NH). |
| 197 | 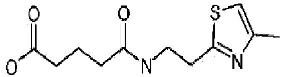 | LC/MS, an individual peak at a retention time of 1.15 min, [M+H]⁺=257 (condition A). HPLC under condition 1, individual peak at a retention time of 11.2 min. ¹H NMR (400.13 MHz, DMSO-d6, δ, m.d., J/Hz): 1.63 (quin, 2H, CH₂CH₂CH₂, J=7.1 Hz); 1.86 (t, 2H, CH₂COOH, J=7.0 Hz); 2.04 (t, 2H, CH₂CONH, J=7.2 Hz); 2.32 (s, 3H, Me), 3.03 (t, 2H, CH₂C, J=7.3 Hz); 3.33 (q, 2H, CH₂NH, J=6.9 Hz); 7.08 (s, 1H, CH), 8.21 (t, 1H, NH, J=5.7 Hz). |
| 198 | 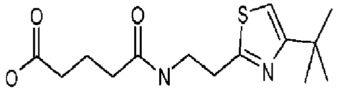 | LC/MS, an individual peak at a retention time of 1.3 min, [M+H]⁺=281 (condition A). HPLC under condition 1, individual peak at a retention time of 16.6 min. ¹H NMR (400.13 MHz, DMSO-d6, δ, m.d., J/Hz): 1.26 (s, 9H, t-Bu), 1.81 (quin, 2H, CH₂CH₂CH₂, J=6.5 Hz); 2.58 (t, 4H, CH₂CH₂CH₂, J=6.5 Hz), 3.06 (t, 2H, CH₂C, J=7.3 Hz), 3.95 (t, 2H, CH₂N, J=7.3 Hz), 7.09 (s, CH, 1H). |
| 341 | 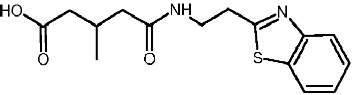 | LC/MS, an individual peak at a retention time of 1.8 min, [M+H]⁺=307 (condition E). HPLC under condition 3, individual peak at a retention time of 13.7 min. |

### Tests for biological activity

Below is detailed description of experimental examples supporting the efficiency of the compounds of formula (a) in the prevention and treatment of diseases in accordance with the present invention, wherein the disclosed examples are not intended to limit the scope of the invention.

### Example 1

### Antiviral activity of the amide compound of formula (a) and reference compounds against Coxsackie virus in vivo

In the study a trypsin-dependent strain HCXV A2 was used, previously adapted and causing death in mice from Coxsackie virus infection.

The experiment was carried out in white mice weighing 6 to 7 g. The animals were infected intramuscularly with a dose of 0.1 mL/mouse. The used infectious dose causing mortality in mice was 10LD₅₀.

The ability of compounds to provide a therapeutic effect was determined by the mortality rate of HCXV A2 virus-infected mice in the experimental group relative to the group of untreated animals.

The studied compounds and placebo were administered orally according to the treatment regimen. Normal saline solution was administered to mice as placebo. Intact animals served as a negative control and were hold in a separate room under the same conditions as the experimental animals.

For the experiment, groups were formed with 14-15 animals each. Compounds were administered at a dose of 30 mg/kg of body weight. The studied compounds were administered orally once daily for 7 days (first administration was made 24 hours after infection). The animals were monitored for 15 days during which the animals were weighed and the mortality rate was registered every day.

The compound of formula (a) and the reference compounds exhibited a protective effect against the experimental model of Coxsackie virus infection by reducing the mortality rate and increasing the average life expectancy of the animals. Data the compound of formula (a) and reference compounds are represented in the Table 3.

The antiviral activity of the studied compounds, disclosed in the example, suggests that these chemical compounds can be used as effective drugs in Coxsackie enterovirus infection.

**Table 3**

| Efficiency of the compound of formula (a) and reference compounds against Coxsackie A2 virus infection in a murine model. | | | | | | |
|---|---|---|---|---|---|---|
| Studied compounds | Dose of studied compounds and reference drug mg/kg | Total number of animals in a group | Total mortality, % | Average life expectancy (days) | | Protective index (%) |
| | | | | Relative | Compared with control | |
| Compound 193 | 30 | 15 | 40.1 | 24.9 | +14.2 | 45.3 |
| Compound 149 | 30 | 15 | 53.3 | 19.6 | +8.9 | 27.2 |
| Compound (a) | 30 | 14 | 42.9 | 22.2 | +10.0 | 33.3 |
| Compound 2 | 30 | 14 | 28.6 | 32.3 | +20.1 | 55.6 |
| Compound 34 | 30 | 14 | 50.0 | 20.5 | +8.3 | 22.2 |
| Compound 12 | 30 | 15 | 40.0 | 24.9 | +14.2 | 45 |
| Compound 13 | 30 | 15 | 46.7 | 19.0 | +8.3 | 36 |
| Compound 14 | 30 | 15 | 50.0 | 21.4 | +10.4 | 36 |
| Compound 23 | 30 | 15 | 50.0 | 23.8 | +13.1 | 36 |
| Compound 30 | 30 | 15 | 60.0 | 13.1 | +2.4 | 18 |
| Compound 35 | 30 | 15 | 53.3 | 16.6 | +5.9 | 27 |
| Compound 36 | 30 | 15 | 53.3 | 16.7 | +6.0 | 27 |
| Compound 89 | 30 | 15 | 53.3 | 17.7 | + 7.0 | 27 |
| Virus control | | 15 | 73.3 | 10.7 | | |
| Compound 90 | 30 | 14 | 35.7 | 25.9 | +15.1 | 50 |
| Compound 67 | 30 | 14 | 35.7 | 27.0 | +16.2 | 50 |
| Virus control | | 14 | 71.4 | 10.8 | | |
| Compound 75 | 30 | 14 | 35.7 | 27.3 | +14.3 | 50 |
| Compound 29 | 30 | 14 | 35.7 | 26.7 | +13.7 | 50 |
| Compound 275 | 30 | 14 | 42.9 | 22.4 | +9.5 | 40 |
| Compound 197 | 30 | 14 | 42.9 | 22.2 | +11.2 | 33.3 |
| Virus control | | 14 | 71.4 | 13.0 | | |
| Compound 32 | 30 | 14 | 42.9 | 22.2 | +10.0 | 33 |
| Compound 44 | 30 | 14 | 28.6 | 32.3 | +20.1 | 55 |
| Compound 71 | 30 | 14 | 35.7 | 28.6 | +16.4 | 44 |
| Virus control | | 14 | 64.3 | 12.2 | | |

### Example 2*

### Antiviral activity of reference compounds against mouse-adapted RS virus

### *Reference Example

Antiviral effectiveness of the chemical compounds against RSV in an experimental murine model *in vivo* was determined for human virus hRSV that was previously adapted to the growth in mouse lungs. The animals were infected with the virus at a dose of 0.5 logTCID₅₀ intranasally under brief ether anesthesia in a volume of 0.05 ml/mouse. The studied compounds were administered orally once daily for 5 days according to the treatment regimen at a dose of 30 mg/kg. The first administration was made 24 hours after infection. Normal saline solution was administered to mice as placebo. Intact animals served as a negative control and were hold in a separate room under the same conditions as the experimental animals. Each experimental group comprised 12 animals. Ribavirin was used as a reference drug at dose of 40 mg/kg.

The antiviral activity of the studied compounds was determined by the efficiency in the prevention of weight loss and by suppression of the reproduction of hRSV in the mouse lungs by measuring a viral titer in the experimental groups relative to the control on days 5 and 7 after infection.

The results of measuring the body weight in animals for the reference compound are represented in Table 4. In the virus control group, the mice showed statistically significant weight loss relative to the intact animals. The antiviral activity of the reference compounds of was evident in body weight gain, as compared with the control animals.

**Table 4**

| **Average body weight in the mice on days 5 and 7 after infection with hRSV** | | |
|---|---|---|
| Preparation | Body weight of the mice on days 5 and 7 after infection with hRSV (M±SD), n=6 | |
| | 5 cyTκ 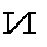 | 7 cyTκ 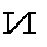 |
| Compound 149 | 15.44±0.31# | 17.32±0.59# |
| Compound 1 | 16.43±0.14# | 17.98±0.26# |
| Compound 117 | 16.07±0.12# | 16.48±0.28# |
| Compound 3 | 16.65±0.28# | 17.32±0.25# |
| Compound 120 | 16.12±0.27# | 17.22±0.20# |
| Compound 4 | 16.77±0.20 | 17.08±0.32# |
| Compound 5 | 16.02±0.16# | 17.78±0.26# |
| Compound 121 | 16.35±0.20# | 17.38±0.29# |
| Compound 122 | 16.93±0.32 | 16.37±0.21# |
| Compound 123 | 15.87±0.20# | 17.55±0.53 |
| Compound 124 | 16.43±0.26# | 16.37±0.43# |
| Compound 6 | 16.47±0.26# | 17.02±0.29# |
| Compound 7 | 17.17±0.26# | 18.53±0.55 |
| Compound 8 | 15.18±0.18 | 17.13±0.27# |
| Compound 9 | 15.75±0.33 | 16.18±0.29# |
| Compound 10 | 16.18±0.29# | 16.53±0.20# |
| Ribavirin | 16.20±0.24# | 17.23±0.22# |
| Virus control | 15.45±0.25 | 15.32±0.31 |
| Intact | 17.30±0.19# | 18.00±0.24# |

| | | |
|---|---|---|
| # - statistically significant differences vs. the control animals (t-criterion, p<0.05). | | |

In addition, the therapeutic action of the reference compounds was determined by their ability to suppress the reproduction of hRSV in the mouse lungs on days 5 and 7 after infection. The viral titer was determined by the titration of a 10% lung suspension in Hep-2 cell culture. The result was registered 2 days after the incubation at 37°C by TCID. The results of the determination of the infectious activity of hRSV in the mouse lung suspensions in Hep-2 cell culture after the administration of the studied compounds and the reference drug are given in Table 5. The administration of the reference compounds to the animals led to a reduction in the hRSV infectious activity.

The study of the antiviral activity of the reference compounds in the murine model of hRSV infection showed that the compounds prevented weight loss and reduced the reproduction of the virus in the lungs of animals.

**Table 5**

| **Suppression of the reproduction of hRSV in the lungs of mice** | | | | |
|---|---|---|---|---|
| Preparation | Day 5 | | Day 7 | |
| | lg | Δlg | lg | Δlg |
| Compound 149 | 2.6±0.1 | 1,9±0.1 | 2.3±0.4 | 1.4±0.4 |
| Compound 1 | 2.88±0.59 | 1.73±0.59 | 1.46±0.17 | 2.34±0.17 |
| Compound 117 | 3.00±0.41 | 1.60±0.41 | 1.46±0.24 | 2.22±0.34 |
| Compound 3 | 3.04±0.42 | 1.56±0.42 | 1.46±0.17 | 2.18±0.28 |
| Compound 120 | 3.04±0.47 | 1.56±0.47 | 1.50±0.25 | 2.05±0.25 |
| Compound 4 | 2.58±0.51 | 2.02±0.51 | 1.38±0.24 | 2.58±0.53 |
| Compound 5 | 2.17±0.37 | 2.43±0.37 | 0.88±0.31 | 2.93±0.31 |
| Compound 121 | 3.08±0.47 | 1.52±0.47 | 1.50±0.14 | 2.09±0.22 |
| Compound 122 | 3.04±0.44 | 1.56±0.44 | 1.75±0.41 | 1.88±0.47 |
| Compound 123 | 2.50±0.43 | 2.10±0.43 | 1.33±0.19 | 2.62±0.50 |
| Compound 124 | 2.46±0.22 | 2.14±0.22 | 0.83±0.37 | 2.97±0.37 |
| Ribavirin | 2.1±0.12 | 2.4±0.12 | 1.15±0.12 | 2.4±0.12 |
| Virus control | 4.60±0.30 | | 3.8±0.29 | |

### Example 3

### Antiviral activity of the compound of formula (a) and reference compounds against RS virus in a murine model of suppressed immune system.

Antiviral activity of the chemical compounds against human respiratory syncytial virus (strain A2, an infectious titer of 5×10⁶ TCID₅₀/mL) was assessed in a Balb/c murine model of viral pneumonia. The virus was inoculated to animals intranasally in a volume of 50 µL under brief ether anesthesia. The immune response in animals to RS virus was suppressed by intraperitoneal administration of cyclophosphan at a dose of 100 mg/kg 5 days before infection. The studied compounds were administered according to the treatment regimen once daily at a dose of 30 mg/kg for 5 days, starting 24 hours after infection. The activity of the compounds was determined by a reduction in edema of the lung infected with respiratory syncytial virus, compared to the control, on day 5 after infection.

The results represented in Table 6 for the compound of formula (a) and reference compounds show that infection of the animals with the virus led to the formation of severe lung edema (3.15-2.05 scores from possible 4). The studied compounds provided a normalizing action on the structure of the pulmonary tissue.

**Table 6**

| **Degree of lung edema in RS-virus pneumonia in Balb/c Mice on day 5 after infection (M±SD, n=5)** | | |
|---|---|---|
| Studied compounds and reference drug | Dose, mg/kg | Degree of lung edema on day 5 after infection, score |
| Virus control | - | 2.70±0.25 |
| Compound 148 | 30 | 2.00±0.31* |
| Compound 2 | 30 | 1.95±0.32* |
| Compound 34 | 30 | 1.75+0.4* |
| Ribavirin | 50 | 1.85±0.42* |
| Virus control | 0 | 2.05±0.23 |
| Compound 35 | 30 | 1.48±0.17* |
| Virus control | - | 3.15±0.22 |
| Compound 121 | 30 | 1.30±0.60* |
| Compound 139 | 30 | 1.75±0.77* |
| Compound 115 | 30 | 1.60±0.5* |
| Compound 118 | 30 | 1.85±0.45* |
| Ribavirin | 50 | 1.75±0.59* |
| Virus control | - | 1.70±0.17 |
| Compound 193 | 30 | 0.80±0.17* |
| Compound 184 | 30 | 1.00±0.19* |
| Compound 96 | 30 | 0.35±0.11* |
| Compound 141 | 30 | 0.55±0.21* |
| Virus control | - | 1.90±0.12 |
| Compound 89 | 30 | 0.75±0.17* |
| Compound 91 | 30 | 0.40±0.11* |
| Compound 197 | 30 | 1.15±0.23* |
| Ribavirin | 50 | 1.75±0.47* |
| Virus control | - | 3.15±0.22 |
| Compound 3 | 30 | 1.6±0.89* |
| Compound 1 | 30 | 1.3±0.27* |
| Compound 198 | 30 | 1.55±0.30* |
| Compound 275 | 30 | 1.80±0.20* |
| Ribavirin | 50 | 1.75±0.59* |
| Virus control | - | 2.70±0.25 |
| Compound 5 | 30 | 1.10±0.19* |
| Compound 6 | 30 | 0.90±0.22* |
| Compound 4 | 30 | 1.95±0.31 |
| Compound 9 | 30 | 1.00±0.17* |
| Ribavirin | 50 | 1.00±0.17* |
| Virus control | - | 2.05±0.23 |
| Compound 120 | 30 | 1.05±0.14* |
| Compound (a) | 30 | 0.90±0.21* |
| Compound 123 | 30 | 1.30±0.17* |
| Ribavirin | 50 | 1.24±0.18* |

| | | |
|---|---|---|
| * marked values different from the control values according to t-criterion (p<0.05). | | |

### Example 4

### Antiviral activity of the compound of formula (a) and reference compounds against rhinovirus

In this study, author's strain of hRV was used. The animals were infected with the virus intranasally under brief ether anesthesia in a volume of 0.05 ml/mouse.

To determine the efficiency of the compounds against hRV in the experimental model in vivo, the virus was previously titrated in mice, then the mice were infected, and the studied compounds were administered orally. The infectious titer was assessed on day 4 after infection by titration of a lung suspension in Hela cell culture. The infectious titer of the hRV virus in the lugs of the experimental group was determined in comparison with that in the control group by TCID.

The studied compounds and placebo (physiological solution) were orally administered to the mice once daily for 4 days, starting 12 hours after infection. The compounds were administered at a dose of 30 mg/kg of animal body weight. Intact animals served as a negative control and were kept under the same conditions as the experimental animals in a separate room.

The antiviral activity of the studied compounds was determined on day 4 after infection by a reduction of the virus infectious activity determined in Hela cell culture.

The development of the infectious process was associated with a reduction in body weight of the animals in the virus control group, wherein the body weight in the mice treated with the studied compounds was higher on days 3 and 4 than the weight of the control animals.

The study of the lung weight showed that during the experiment, the lung weight in the infected mice exceeded the lung weight in the intact mice, which was indicative of an infectious process. The weight of the animal lungs exposed to the effect of the studied compounds was significantly different (was lower) from that in the virus control group and was almost the same as the lung weight in the intact animals.

The results of the determination of hRV infectious activity in mouse lung suspensions in Hela cell culture after administration of the compound of formula (a) and reference compounds are represented in Table 7.

**Table 7**

| **Suppression of the reproduction of HRV in mouse lungs** | | |
|---|---|---|
| Preparation | Dose of preparation, mg/kg | Infectious titer of the virus in lungs lg TCID₅₀ (4 days after infection) |
| Compound 2 | 30 | 0.1 ±0.05 |
| Compound 6 | 30 | 0.4 ±0.15 |
| Compound 34 | 30 | 0.5 ±0.20 |
| Compound 115 | 30 | 0.05 ±0.05 |
| Compound 118 | 30 | 0±0 |
| Compound 141 | 30 | 0±0 |
| Compound 197 | 30 | 0.37 ±0.06 |
| Compound 198 | 30 | 0.35 ±0.04 |
| Compound (a) | 30 | 0±0 |
| Compound 275 | 30 | 0.45 ±0.1 |
| Control | - | 2.3 ±0.3 |

The treatment with the compound of formula (a) and the reference compounds led to a reduction in the infectious activity of hRV.

The study of the antiviral activity of the compound of formula (a) and the reference compounds in murine model of hRV-infection showed that the claimed compounds prevented weight loss and an increase in the lung weight to the values observed in the group of intact animals and reduced the reproduction of the virus in the animal lungs.

### Example 5

### Antiviral activity of the compound of formula (a) and reference compounds against parainfluenza virus

In the study the Sendai strain of parainfluenza virus was used. White outbred mice weighing 10-12 g were infected intranasally with the Sendai strain of parainfluenza virus adapted to mouse lungs under brief ether anesthesia in a volume of 0.05 ml/mouse. The infectious dose of the virus that caused 70-80% mortality in mice was 10LD50. Each group used in the experiment comprised 20 animals. Intact animals served as control and were hold in a separate room under the same conditions as the experimental animals. The antiviral activity of the compound of formula (a) and the reference compounds was studied by oral administration of the compounds to infected mice once daily at a dose of 30 mg/kg/mouse 24, 48, 72, 96, and 120 hours after infection of the animals with the virus. The mice of the control group were administered placebo (0.2 mL of physiological solution) under the same conditions. The animals were monitored for 14 days after infection by registration the mouse death in the groups.

Each animal was subjected to once-daily observation. The observation included the assessment of general behavior and body condition of animals. In days of administration of preparations, the observation was conducted before administration of a preparation in a certain time and about two hours after administration. The animals were handled according to the International Standards.

The activity of the compounds was evaluated by comparing mortality rates in the groups of animals administered a preparation and placebo.

The mortality rate of the groups of animals administered the compound of formula (a) and the reference compounds was reduced by 30-60%. Data for the tested compounds are represented in Table 8.

**Table 8**

| **Mortality in the experimental groups of animals** | | | |
|---|---|---|---|
| No. | Preparation | Dose of preparation (mg/kg) | Mortality, % |
| 1 | Compound 2 | 30 | 35.0 |
| 2 | Compound 6 | 30 | 25.0 |
| 3 | Compound 34 | 30 | 15.0 |
| 4 | Compound 115 | 30 | 30.0 |
| 5 | Compound 118 | 30 | 30.0 |
| 6 | Compound 141 | 30 | 40.0 |
| 7 | Compound 197 | 30 | 25.0 |
| 8 | Compound 198 | 30 | 30.0 |
| 9 | Compound (a) | 30 | 35.0 |
| 10 | Compound 275 | 30 | 40.0 |
| 11 | Virus control | - | 75.0 |
| 12 | Intact | - | 0.0 |

### Example 6

### Antiviral activity of the compound of formula (a) and reference compounds in the model of experimental adenovirus virus

In the study, human adenovirus type 5 was used. In order to play adenovirus infection were used Newborn Syrian hamsters, in which the virus caused disseminated viral infection with damage to liver, lung and heart, were used to reproduce the viral infection. The animals were studied 48 hours after birth. Each group comprised 5 hamsters. The virus was inoculated subcutaneously in a volume of 0.1 mL, at a dose of 10⁵ TCID₅₀. The treatment was carried out orally with the compound of formula (a) and the reference compounds at a dose of 30 mg/kg of body weight 12, 36, and 60 hours after infection. The animals of the placebo group were administered phosphate buffered saline. Intact animals served as control and were hold in a separate room under the same conditions as the experimental animals. 72 hours after infection, the animals of each group were euthanized, dissected, and the liver was isolated. The therapeutic effect was evaluated by the action on ultrastructural features of the morphogenesis of adenovirus infection in the liver by electron microscopy.

As a result, it was shown that the treatment with the compound of formula (a) and the reference compounds reduced the intensity of destructive processes and inflammatory reactions in the liver, normalizing its structure at the both levels of tissue and hepatocytes. The results of integral estimation of damages for the tested compounds are represented in the table 9.

**Table 9**

| **Evaluation of the intensity of destructive processes in the liver** | | |
|---|---|---|
| Preparation | Dose of preparation (mg/kg) | Evaluation of damage |
| Compound 2 | 30 | Mild damage |
| Compound 6 | 30 | Mild damage |
| Compound 34 | 30 | Mild damage |
| Compound 115 | 30 | Mild damage |
| Compound 118 | 30 | Mild damage |
| Compound 141 | 30 | Mild damage |
| Compound 197 | 30 | Mild damage |
| Compound 198 | 30 | Mild damage |
| Compound (a) | 30 | Mild damage |
| Compound 275 | 30 | Mild damage |
| Intact | - | No damages |
| Virus control | - | Intensive damages |

### Example 7

### Antiviral activity of the compound of formula (a) and reference compounds in the murine model of experimental herpetic meningoencephalitis

In the study, herpes simplex virus belonging to antigenic type 2 was used. White outbred mice weighing 7-8 g were infected i/c (intracerebrally) in a volume of 0.05 ml/mouse comprising a dose of 10LD50. The infectious dose of the virus that caused 100% mortality in mice was 10LD50. Each experimental group comprised 20 mice. Intact animals served as control and were hold in a separate room under the same conditions as the experimental animals. The antiviral activity of the compound of formula (a) and the reference compounds was studied by oral administration of the compounds to infected mice once daily at a dose of 30 mg/kg/mouse 24, 48, 72, 96, and 120 hours after infection of the animals with the virus. The mice of the control group were administered placebo (0.2 mL of physiological solution) under the same conditions. The animals were monitored for 14 days after infection by registration the mouse death in the groups.

Each animal was subjected to once-daily observation. The observation included the assessment of general behavior and body condition of animals. The animals were handled according to the International Standards.

The activity of the compounds was evaluated by comparing mortality rates in the groups of animals administered a preparation and placebo.

The mortality rate of the groups of animals administered the compound of formula (a) and the reference compounds was reduced by 25-50%. Data for the tested compounds are represented in Table 10.

**Table 10**

| **Mortality in the experimental groups of animals** | | | |
|---|---|---|---|
| No. | Preparation | Dose of preparation (mg/kg) | Mortality, % |
| 1 | Compound 2 | 30 | 55.0 |
| 2 | Compound 6 | 30 | 60.0 |
| 3 | Compound 34 | 30 | 70.0 |
| 4 | Compound 115 | 30 | 65.0 |
| 5 | Compound 118 | 30 | 70.0 |
| 6 | Compound 141 | 30 | 50.0 |
| 7 | Compound 197 | 30 | 55.0 |
| 8 | Compound 198 | 30 | 60.0 |
| 9 | Compound (a) | 30 | 65.0 |
| 10 | Compound 275 | 30 | 60.0 |
| 11 | Compound 5 | 30 | 35.0 |
| 12 | Compound 7 | 30 | 20.0 |
| 13 | Compound 15 | 30 | 30.0 |
| 14 | Compound 44 | 30 | 40.0 |
| 15 | Compound 52 | 30 | 25.0 |
| 16 | Compound 68 | 30 | 30.0 |
| 17 | Compound 92 | 30 | 25.0 |
| 18 | Compound 100 | 30 | 30.0 |
| 19 | Compound 104 | 30 | 45.0 |
| 20 | Compound 124 | 30 | 40.0 |
| 21 | Compound 126 | 30 | 40.0 |
| 22 | Compound 131 | 30 | 45.0 |
| 23 | Compound 149 | 30 | 25.0 |
| 24 | Compound 193 | 30 | 25.0 |
| 25 | Compound 341 | 30 | 30.0 |
| 26 | Virus control | - | 100.0 |
| 27 | Intact | - | 0.0 |

### Example 8

### Antiviral activity of the compound of formula (a) and reference compounds in the murine model of coronavirus infection

In the study, author's strain HCoV was used, identified as group 2 virus antigenically similar to prototype strain OC-43. The efficiency of the compounds was studied in C57BL/6 mice by comparing mortality rates in the treated and control mice for 14 days after infection. Each experimental group comprised 20 mice. The animals were infected intranasally in a volume of 0.03 ml/mouse under brief ether anesthesia.

The studied compounds were administered to the animals orally at a dose of 30 mg/kg of body weight. The animals of the control group were administered physiological solution. Preparations were administered once daily for 5 days. The treatment of animals was started 24 hours after infection.

The mortality rate of the groups of animals administered the compound of formula (a) and the reference compounds was reduced by 30-50%. Data for the tested compounds are represented in Table 11.

**Table 11**

| **Mortality in the experimental groups of animals** | | |
|---|---|---|
| Preparation | Dose of preparation (mg/kg) | Mortality, % |
| Compound 2 | 30 | 30.0 |
| Compound 6 | 30 | 35.0 |
| Compound 34 | 30 | 45.0 |
| Compound 115 | 30 | 30.0 |
| Compound 118 | 30 | 40.0 |
| Compound 141 | 30 | 35.0 |
| Compound 197 | 30 | 45.0 |
| Compound 198 | 30 | 40.0 |
| Compound (a) | 30 | 30.0 |
| Compound 275 | 30 | 40.0 |
| Virus control | - | 60.0 |
| Intact | - | 0.0 |

### Example 9

### Evaluation of the efficiency of the compound of formula (a) and reference compounds in a rat model of nasopharyngitis

Nasopharyngitis was induced by intranasal administration of formalin to each nasal passage of rats.

The administration of formalin to the nasal passages of rats leads to the dissemination of inflammation to adjacent tissues, resulting in a clinical pattern similar to the symptoms of nasopharyngitis in a human.

After an acclimatization period, the following groups were formed:
- intact animals administered intragastrically a physiological solution in an amount of 0.2 ml, without induction of nasopharyngitis;
- a control group consisted of animals administered intragastrically a physiological solution in an amount of 0.2 ml for 3 days after induction of nasopharyngitis; and
- animals administered the studied compounds at a dose of 18 mg/kg for 3 days after induction of nasopharyngitis.

Clinical observation of each animal was performed every day at least twice daily.

In the experiment of the induction of nasopharyngitis in Wistar rats by administration of formalin to the nasal passages, pathological changes were observed in the animals of the control group, which were characterized by the development of acute inflammation process in the upper respiratory tract. The caused pathology was characterized by hyperplasia, increased number of caliciform cells, pronounced infiltration of mononuclear cells and leucocytes, and mucus hyperproduction by submucosal glands.

After euthanasia, the inflammation pattern in the nasal passages and throat was studied in each group of animals. The nasal passages were washed with 5 mL of physiological solution and the score of cell elements were counted in 1 µL.

**Table 12**

| **Macroscopic characteristic of changes in the mucous membrane of nasal passages** | | | |
|---|---|---|---|
| **Group** | **n** | **Without changes** | **Discharge mucus from the nasal passages** |
| Intact | 20 | 20 | 0 |
| Control | 20 | 0 | 20 |
| Compound 2 | 10 | 3 | 7 |
| Compound 6 | 10 | 4 | 6 |
| Compound 34 | 10 | 3 | 7 |
| Compound 115 | 10 | 3 | 7 |
| Compound 118 | 10 | 5 | 5 |
| Compound 141 | 10 | 4 | 6 |
| Compound 197 | 10 | 6 | 4 |
| Compound 198 | 10 | 7 | 3 |
| Compound (a) | 10 | 4 | 6 |
| Compound 275 | 10 | 3 | 7 |
| Compound 92 | 10 | 6 | 4 |
| Compound 149 | 10 | 5 | 5 |
| Compound 193 | 10 | 6 | 4 |
| Compound 341 | 10 | 6 | 4 |
| Compound 5 | 10 | 5 | 5 |
| Compound 7 | 10 | 6 | 4 |
| Compound 15 | 10 | 7 | 3 |
| Compound 52 | 10 | 5 | 5 |
| Compound 68 | 10 | 3 | 7 |
| Compound 100 | 10 | 3 | 7 |
| Compound 131 | 10 | 5 | 5 |
| Compound 44 | 10 | 4 | 6 |
| Compound 104 | 10 | 6 | 4 |
| Compound 124 | 10 | 7 | 3 |
| Compound 126 | 10 | 4 | 6 |

| | | | |
|---|---|---|---|
| n - the number of animals | | | |

As can be seen from Table 12, the compound of formula (a) and the reference compounds exhibit anti-inflammatory activity and are therapeutically effective in the model of nasopharyngitis. The pharmacological action of the studied compounds was expressed in a reduction in the flow of inflammatory cells and mucus hyperproduction. Most of the studied compounds reduced the number of cell elements in nasal lavages by 40-58% relative to the control.

### Example 10

### Evaluation of the efficiency of the compound of formula (a) and reference compounds in a murine model of staphylococcal pneumonia

The efficiency of the compounds was evaluated in outbred mice (females) infected with *Staphylococcus aureus* (mouse-adapted strain). The administration of the compounds was started 5 days before infection, orally at doses of 15 and 30 mg/kg in a volume of 0.2 mL. On day 5, the mice were infected intranasally under brief ether anesthesia by administration of *Staphylococcus aureus* at a dose of 10⁹ CFU in a volume of 0.05 mL. One hour after infection, the administration of the compounds to the mice was continued at the above-indicated doses for additional 2 days. The reference drug was ampicillin administered intravenously at a single dose of 20 mg/kg. The control was mice infected with *Staphylococcus aureus* intranasally and treated with PBS. The mice were sacrificed two days after, the breast was dissected, and lung imprints were made in Petri dishes with Columbia agar. After incubation for 24 hours at 30°C, the presence (or absence) of *Staphylococcus aureus* bacterial growth was fixed in comparison with the control. The intensity of bacterial growth was evaluated in scores and expressed in %. The results are given in Table 13.

As can be seen from Table 13, the compound of formula (a) and the reference compounds exhibit antibacterial activity and are effective in the model of pneumonia.

**Table 13**

| **Efficiency of the amide compound of formula (a) and reference compounds in the murine model of staphylococcal pneumonia** | | |
|---|---|---|
| **Preparation** | **Bacterial growth, in %** | |
| | **15 mg/kg** | **30 mg/kg** |
| Compound 2 | **50** | **55** |
| Compound 6 | **45** | **55** |
| Compound 34 | **25** | **50** |
| Compound 115 | **5**5 | **25** |
| Compound 118 | **55** | **50** |
| Compound 141 | **55** | **40** |
| Compound 197 | **45** | **60** |
| Compound 198 | **45** | **55** |
| Compound (a) | **55** | **20** |
| Compound 275 | **35** | **40** |
| **Ampicillin** | **25** | |
| **Control** | **82.5** | |
| **Intact** | **0** | |

0 - no growth
25 - sporadic colonies (up to 10)
50 - sporadic colonies (up to 100)
75 - multiple colonies (more than 100)
100 - confluent growth

### Example 11

### Evaluation of the efficiency of the compound of formula (a) and reference compounds in a rat model of peribronchitis

Sephadex G-200 was administered to Wistar male rats by a single inhalation at a dose of 5 mg/kg. The studied compounds were administered to the animals intragastrically four times: 24 and 1 hour before and 24 and 45 hours after the Sephadex administration. Euthanasia was made 48 hours after the Sephadex inhalation, and a lung was taken for histological analysis. 4-µm-thick sections were stained with hematoxylin and eosin. The inflammatory changes in the lungs were evaluated by a 5-point scale, wherein:
1 means inflammatory infiltrate that occupies 0-20% of the area of a studied histological preparation;
2 means inflammatory infiltrate that occupies 21-40% of the area of a studied histological preparation;
3 means inflammatory infiltrate that occupies 41-60% of the area of a studied histological preparation;
4 means inflammatory infiltrate that occupies 61-80% of the area of a studied histological preparation; and
5 means inflammatory infiltrate that occupies 81-100% of the area of a studied histological preparation;

The number of rats in a group varies from 7 to 10 animals.

The histological analysis of the lungs showed that a single inhalation of Sephadex causes in rats a pronounced flow of inflammatory infiltration cells, preferably lymphocytes, to the bronchioles area (peribronchitis) (Table 14).

Intragastric administration of the compounds to the rats reduced the peribronchitis symptoms. All the studied compounds exhibited activity within the tested doses.

**Table 14**

| **Histological analysis of a lung in the rat model of peribronchitis** | | |
|---|---|---|
| Group | Dose of compound (mg/kg) | Peribronchitis (scores) |
| Intact | - | 1.57±0.2 |
| Control | - | 3.14+0.26 |
| XC2 | 1.8 | 2.14±0.34 |
| | 18 | 2.37±0.3 |
| XC6 | 1.8 | 2.14±0.14 |
| | 18 | 2.37+0.37 |
| XC34 | 1.8 | 2.21±0.36 |
| | 18 | 2.17±0.3 |
| XC115 | 1.8 | 2.27±0.37 |
| | 18 | 2.43±0.2 |
| XC118 | 1.8 | 2.4±0.31 |
| | 18 | 2.1±0.22 |
| XC141 | 1.8 | 2.25±0.31 |
| | 18 | 1.71±0.29 |
| XC197 | 1.8 | 2±0 |
| | 18 | 1.86±0.26 |
| Intact | - | 1.86±0.34 |
| Control | - | 3±0.31 |
| XC198 | 1.8 | 2.44±0.34 |
| | 18 | 2.44±0.29 |
| XC (a) | 1.8 | 2.44±0.29 |
| | 18 | 2.0±0.31 |
| XC275 | 1.8 | 2.08±0.15 |
| | 18 | 2±0.29 |

### Example 12

Dosage forms of the compound according to the invention

The compound according to the invention can be administered orally, intranasally, intramuscularly, or intravenously in a unit dosage form comprising non-toxic pharmaceutically acceptable carriers.

The compound can be administered to a patient in daily doses of from 0.1 to 10 mg/kg of body weight, preferably in doses of from 0.5 to 5 mg/kg, one or more times a day.

However, it should be noted that a particular dose for a particular patient depends on many factors, such as patient's age, body weight, gender, general health condition, dietary pattern, and the schedule and route of drug administration, the excretion rate of the drug from the body, and the disease severity in the patient under treatment.

Pharmaceutical compositions comprise the compound according to the invention in an amount effective for achieving a positive result and can be administered in standard dosage forms (for example, in solid, semi-solid, or liquid forms) that comprise the compound as an active agent in a mixture with a carrier or an excipient suitable for oral, intramuscular, or intravenous administration. The active ingredient can be in a composition with a conventional nontoxic pharmaceutically acceptable carrier suitable for the manufacture of solutions, tablets, pills, capsules, coated pills, and other dosage forms.

Diverse compounds may be used as excipients, such as saccharides, for example, glucose, lactose, of sucrose; mannitol or sorbitol; cellulose derivatives; and/or calcium phosphates, for example, tricalcium phosphate or calcium hydrogen phosphate. Compounds, which are suitable as a binder, include starch paste (for example, corn, wheat, rice, or potato starch), gelatin, tragacanth, methylcellulose, hydroxypropyl methylcellulose, sodium carboxymethylcellulose, and/or polyvinylpyrrolidone. Optionally used disintegrating agents include the above-mentioned starches and carboxymethyl starch, crosslinked polyvinylpyrrolidone, agar-agar, alginic acid, and a salt thereof, such as sodium alginate.

Optional additives include, for example, flowability control agents and lubricating agents, such as silica, talc, stearic acid and salts thereof, such as magnesium stearate or calcium stearate, and/or propylene glycol.

Stabilizers, thickening agents, colorants, and fragrances also can be used as additives.

In a standard dosage form, the amount of an active agent used in combination with a carrier can vary depending on a patient under the therapy and on the route of administration of the therapeutic agent.

For example, when the compound is used in the form of solutions for injection, the active agent in such solutions is in an amount of 0.01 to 5 wt.%. A diluent may be selected from a 0.9% sodium chloride solution, distilled water, a Novocain solution for injection, Ringer's solution, a glucose solution, comprising specific solubilizing adjuvants. When the compound is administered in tablet form, its amount is 5.0 to 500 mg per unit dosage form.

Dosage forms of the compound according to the present invention are prepared by standard methods such as, for example, processes of mixing, granulation, forming coating pills, dissolution and liophilization.

### Tablet form

Tablet form is prepared by using the following ingredients:

| Active agent | | |
|---|---|---|
| Compound according to the invention or a pharmaceutically acceptable salt thereof | 10 mg | 100 mg |
| Additives | | |
| Microcrystalline cellulose | 70.55 mg | 95.90 mg |
| Lactose monohydrate | 67.50 mg | 99.00 mg |
| Sodium glycolate starch | 0.75 mg | 1.50 mg |
| Talc | 0.60 mg | 1.20 mg |
| Magnesium stearate | 0.60 mg | 2.40 mg |
| Weight of the tablet core | 150.00 mg | 300.00 mg |
| Coating | 4.50 mg | 9.00 mg |
| Tablet weight | 154.50 mg | 309.00 mg |

The components are mixed and compressed to form tablets.

### Suppositories

### Example of the formulation of a suppository

| | |
|---|---|
| Compound according to the invention or a pharmaceutically acceptable salt thereof | 1-100 mg |
| Cacao oil | in an amount required for a suppository |

If necessary, rectal, vaginal, and urethral suppositories can be prepared with corresponding excipients.

### Solution for injection

Example of the formulation of a solution for injection:

| | |
|---|---|
| Compound according to the invention or a pharmaceutically acceptable salt thereof | 1-50 mg |
| Water for injection | 2 mL |

### Preparation of dosage forms

Dosage forms of the compound according to the present invention are prepared by standard methods such as, for example, processes of mixing, granulation, forming coating pills, dissolution and liophilization.

### Tablet form

Tablet form is prepared by using the following ingredients:

| | |
|---|---|
| Compound of formula (a) or a pharmaceutically acceptable salt thereof | 100 mg |
| Potato starch | 20-50 mg |
| Magnesium stearate | 3 mg |
| Aerosil | 1 mg |
| Lactose | up to 300 mg |

The ingredients are mixed and compressed to form tablets weighing 300 mg

### Gelatinous capsules

Compound of formula (a) or a pharmaceutically acceptable salt 100 mg thereof

Lactose (milk sugar), potato starch, in an amount to colloidal silica (aerosil), magnesium obtain a 220 mg stearate capsule

These ingredients are mixed and granulated, and the resulting granules are placed into solid gelatinous capsules in an amount of 220 mg.

### Suppositories

### Example of the formulation of a suppository

| | |
|---|---|
| Compound of formula (a) or a pharmaceutically acceptable salt thereof | 10-100 mg |
| Cacao oil | in an amount required for a suppository |

If necessary, rectal, vaginal, and urethral suppositories can be prepared with corresponding excipients.

### Solution for injection

### Example of the formulation of a solution for injection:

| | |
|---|---|
| Compound of formula (a) or a pharmaceutically acceptable salt thereof | 10-100 mg |
| Water for injection | 2 mL |

The solvent in the solution for injection can be a 0.9% sodium chloride solution, distilled water, or a novocaine solution. Pharmaceutical forms are ampules, flasks, syringe-tubes, and "inserts".

### Formulation 1 of solution for injection:

| | |
|---|---|
| Compound of formula (a) or a salt thereof | 10-100 mg |
| Water for injection | 5 mL |

The solvent in the solution for injection can be a 0.9% sodium chloride solution or an isotonic phosphate buffer. Pharmaceutical forms are ampules, flasks, syringe-tubes, and "inserts".

Formulations for injection can be prepared in various dosage units such as sterile solution, sterile powders, and tablets.

## Claims

1. A compound having the following structure: 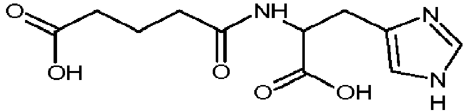 or a pharmaceutically acceptable salt thereof for use in the prevention or treatment of a disease caused by an RNA-containing virus belonging to the Enterovirus, Metapneumovirus, Pneumovirus, Respirovirus, or Alfa-coronavirus genus, and/or by a DNA-containing virus belonging to the Adenoviridae and/or Herpesviridae family.

2. The compound of claim 1 for use according to claim 1, wherein the virus belonging to the Enterovirus genus is selected from the group including rhinoviruses, Coxsackie viruses, and Enterovirus 71.

3. The compound of claim 1 for use according to claim 1, wherein the virus belonging to the Pneumovirus genus is respiratory syncytial virus, the virus belonging to the Metapneumovirus genus is human metapneumovirus, the virus belonging to the Respirovirus genus is parainfluenza virus, and the virus belonging to the Alfa-coronavirus genus is coronavirus.

4. The compound of claim 1 for use according to claim 1, wherein the Adenoviridae family includes the Mastadenovirus genus to which human adenovirus belongs.

5. The compound of claim 1 for use according to claim 1, wherein the disease is a disease caused by herpes simplex virus type 1 or 2.

6. A compound having the following structure: 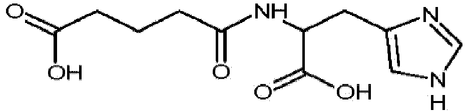 or a pharmaceutically acceptable salt thereof for use in the prevention or treatment of asthma exacerbation, chronic obstructive pulmonary disease, mucoviscidosis, conjunctivitis, gastroenteritis, hepatitis, and myocarditis.

7. A compound having the following structure: 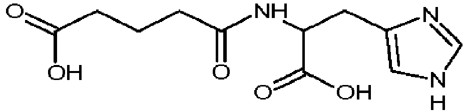 or a pharmaceutically acceptable salt thereof for use in the prevention or treatment of complications of an infectious disease caused by an RNA-containing virus belonging to the Enterovirus, Metapneumovirus, Pneumovirus, Respirovirus, or Alfa-coronavirus genus, and/or by a DNA-containing virus belonging to the Adenoviridae and/or Herpesviridae family.

8. The compound of claim 7, wherein the complication is pharyngitis, nasopharyngitis, tonsillitis, laryngitis, laryngotracheitis, laryngotracheobronchitis, bronchitis, bronchiolitis, pneumonia or airway obstructive syndrome.

9. A compound having the following structure: 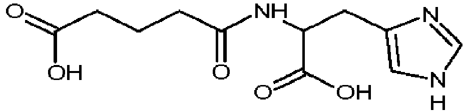 or a pharmaceutically acceptable salt thereof for use in the prevention or treatment of rhinorrhea, acute and infectious rhinitis, pharyngitis, nasopharyngitis, tonsillitis, laryngitis, laryngotracheitis, laryngotracheobronchitis, bronchitis, bronchiolitis, pneumonia or airway obstructive syndrome.

10. A pharmaceutical composition for use in the prevention or treatment of a disease or complications of an infectious disease, the disease or infectious disease being caused by an RNA-containing virus belonging to the Enterovirus, Metapneumovirus, Pneumovirus, Respirovirus, or Alfa-coronavirus genus, and/or by a DNA-containing virus belonging to the Adenoviridae and/or Herpesviridae family, wherein the pharmaceutical composition comprises an effective amount of a compound having the following structure: 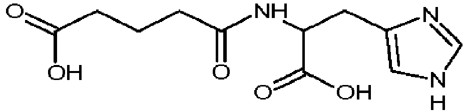 or a pharmaceutically acceptable salt thereof,
and pharmaceutically acceptable carriers and excipients.

11. A pharmaceutical composition for use in the prevention or treatment of asthma exacerbation, chronic obstructive pulmonary disease, mucoviscidosis, conjunctivitis, gastroenteritis, hepatitis or myocarditis, the composition comprising an effective amount of a compound having the following structure: 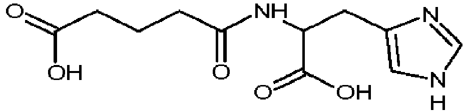 or a pharmaceutically acceptable salt thereof, and pharmaceutically acceptable carriers and excipients.

12. A pharmaceutical composition for use in the prevention or treatment of rhinorrhea, acute and infectious rhinitis, pharyngitis, nasopharyngitis, tonsillitis, laryngitis, laryngotracheitis, laryngotracheobronchitis, bronchitis, bronchiolitis, pneumonia or airway obstructive syndrome, the composition comprising an effective amount of a compound having the following structure: 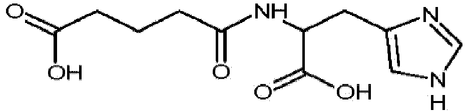 or a pharmaceutically acceptable salt thereof, and pharmaceutically acceptable carriers and excipients.

13. A kit for use in the prevention or treatment of a disease or complications of an infectious disease, the disease or infectious disease being caused by an RNA-containing virus belonging to the Enterovirus, Metapneumovirus, Pneumovirus, Respirovirus, or Alfa-coronavirus genus, and/or by a DNA-containing virus belonging to the Adenoviridae and/or Herpesviridae family, wherein the kit comprises a pharmaceutical composition comprising an effective amount of a compound having the following structure: 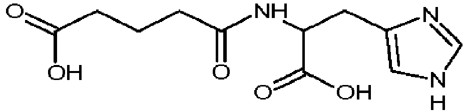 or a pharmaceutically acceptable salt thereof, and pharmaceutically acceptable carriers and excipients ; and instructions for use thereof.

14. A kit for use in the prevention or treatment of asthma exacerbation, chronic obstructive pulmonary disease, mucoviscidosis, conjunctivitis, gastroenteritis, hepatitis, myocarditis, comprising a pharmaceutical composition comprising an effective amount of a compound having the following structure: 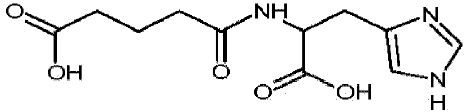 or a pharmaceutically acceptable salt thereof, and pharmaceutically acceptable carriers and excipients ; and instructions for use thereof.

15. A kit for use in the prevention or treatment of rhinorrhea, acute and infectious rhinitis, pharyngitis, nasopharyngitis, tonsillitis, laryngitis, laryngotracheitis, laryngotracheobronchitis, bronchitis, bronchiolitis, pneumonia or airway obstructive syndrome, comprising a pharmaceutical composition comprising an effective amount of a compound having the following structure: 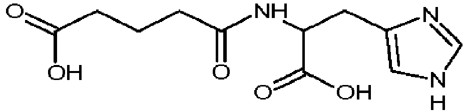 or a pharmaceutically acceptable salt thereof, and pharmaceutically acceptable carriers and excipients ; and instructions for use thereof.
